# EUROPEAN PATENT APPLICATION

(11) **EP 3 900 735 A1**
(43) Date of publication of application: **27.10.2021**
(21) Application number: 19898362.9
(22) Date of filing: 23.12.2019
(51) Int. Cl.: A61K 38/28, A61K 38/22, A61K 47/68, A61K 47/60, A61P 3/10, C07K 14/62, C07K 14/605, C07K 14/575

(54) **PHARMACEUTICAL COMPOSITION COMPRISING INSULIN AND TRIPLE AGONIST HAVING ACTIVITY WITH RESPECT TO ALL OF GLUCAGON AND GLP-1 AND GIP RECEPTOR**

(30) Priority: 21.12.2018 KR 20180167698
(71) Applicant: Hanmi Pharm. Co., Ltd., Hwaseong-si, Gyeonggi-do 18536 (KR)
(72) Inventor: LEE, Jong Suk, Hwaseong-si, Gyeonggi-do 18469 (KR); KIM, Jung Kuk, Hwaseong-si, Gyeonggi-do 18469 (KR); LEE, Sang Don, Hwaseong-si, Gyeonggi-do 18469 (KR); LEE, Jong-Soo, Hwaseong-si, Gyeonggi-do 18469 (KR); KIM, Sang Yun, Hwaseong-si, Gyeonggi-do 18469 (KR)
(74) Representative: Mewburn Ellis LLP
(86) International application number: PCT/KR2019/018316
(87) International publication number: WO 2020/130749

(57) **Abstract**

The present invention relates to a composition comprising insulin and a triple agonist having activity with respect to all of a glucagon receptor, GLP-1 receptor, and GIP receptor, and to a composite preparation comprising the composition.

## Description

### [Technical Field]

The present invention relates to a composition containing insulin and a triple agonist having activity to all of glucagon, GLP-1, and GIP receptors and a complex preparation containing the same.

### [Background Art]

Insulin, which is a blood glucose control hormone secreted by the pancreas of the human body, serves to transport excess glucose in the blood to cells to thereby supply a source of energy to the cells and to maintain blood glucose levels within a normal range. In diabetic patients, however, insulin does not function properly due to the lack of insulin, insulin resistance, and loss of beta-cell function. As a result, diabetic patients cannot utilize glucose in the blood as an energy source, and show symptoms of hyperglycemia with high blood glucose levels, resulting in excretion of glucose in the urine, which causes various complications. Therefore, diabetic patients with abnormal insulin production (Type I) or insulin resistance (Type II) are in need of insulin treatment, and blood glucose levels can be regulated to a normal range through insulin administration.

In current years, 25% or more of type I diabetes cases and 80% or more of type II diabetes cases have high blood glucose levels and are obese. The administration of insulin alone is known to cause a side effect of weight gain in spite of an excellent blood glucose control effect. Therefore, there is a need to develop medicines capable of effectively reducing the weight gain side effect of insulin while exhibiting the blood glucose lowering effect of insulin.

The present applicant has developed various insulin analogs retaining insulin activity (WO 2014/133324 A1 and WO 2017/039267), and developed triple agonists having activity to all of glucagon, GLP-1, and GIP receptors (WO 2017/116204 and WO 2017/116205). However, these have not been used in combination as a combined preparation.

### [Disclosure]

### [Technical Problem]

It was verified in the present application that the use of insulin and a triple agonist in combination showed notably excellent blood glucose control ability and effectively reduced weight gain caused by insulin, compared with the administration of insulin or the triple agonist alone.

### [Technical Solution]

An object of the present invention is to provide a composition including: (i) insulin; and (ii) an isolated peptide having activity to glucagon, glucagon-like peptide-1 (GLP-1), and glucose-dependent insulinotropic polypeptide (GIP) receptors.

Specifically, one object of the present invention is to provide a pharmaceutical composition for prevention or treatment of an insulin-associated disease, the composition including: (i) insulin; and (ii) an isolated peptide having activity to glucagon, GLP-1, and GIP receptors.

Another object of the present invention is to provide a pharmaceutical composition for reducing weight gain caused by insulin administration: (i) insulin; and (ii) an isolated peptide having activity to glucagon, GLP-1, and GIP receptors.

Another object of the present invention is to provide a complex preparation for weight loss in an insulin-administered patient, the complex preparation including: (i) insulin; and (ii) an isolated peptide having activity to glucagon, GLP-1, and GIP receptors.

Another object of the present invention is to provide a kit for prevention or treatment of an insulin-associated disease, the kit including: (i) insulin; and (ii) an isolated peptide having activity to glucagon, GLP-1, and GIP receptors.

Still another object of the present invention is to provide use of the composition in the preparation of a medicinal material.

Still another object of the present invention is to provide a method for prevention or treatment of an insulin-associated disease, the method including administering to a subject (i) insulin; and (ii) an isolated peptide having activity to glucagon, GLP-1, and GIP receptors.

Still another object of the present invention is to provide a method for reducing weight gain caused by insulin administration, the method including administering to a subject an isolated peptide having activity to glucagon, GLP-1, and GIP receptors.

Specifically, still another object of the present invention is to provide a method for reducing weight gain caused by insulin administration, the method including administering to a subject (i) insulin; and (ii) an isolated peptide having activity to glucagon, GLP-1, and GIP receptors.

### [Advantageous Effects]

The composition or complex preparation of the present invention, which contains insulin or a long-acting conjugate thereof and a triple agonist or a long-acting conjugate thereof, provides a novel combined therapy capable of not only exhibiting a prevention or treatment effect on an insulin-associated disease, such as diabetes, but also mitigating side effects caused by insulin administration, such as weight gain.

### [Brief Description of Drawings]

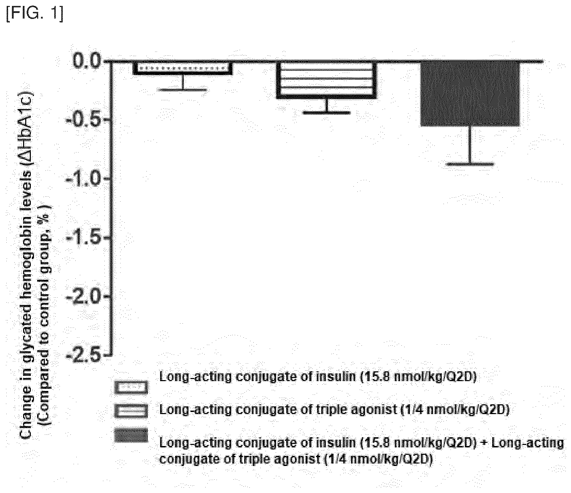

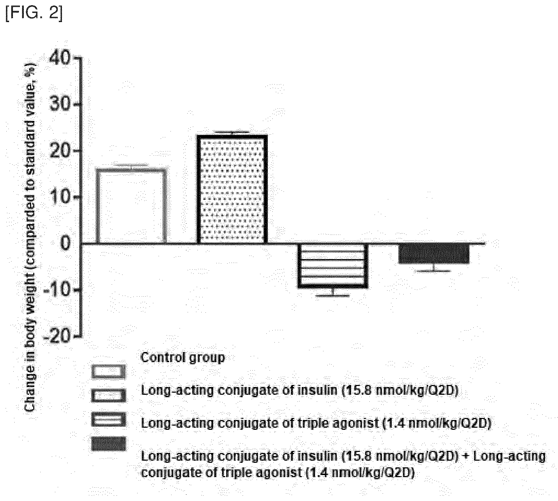

FIGS. 1 and 2 show a blood glucose control effect and a body weight change in type II diabetic model mice due to the combined administration of a long-acting conjugate of insulin and a long-acting conjugate of a triple agonist, respectively.

### [Best Mode for Carrying Out the Invention]

An aspect of the present invention is directed to a composition including: (i) insulin; and (ii) an isolated peptide having activity to glucagon, glucagon-like peptide-1 (GLP-1), and glucose-dependent insulinotropic polypeptide (GIP) receptors.

In an embodiment, the present invention is directed to a pharmaceutical composition for prevention or treatment of an insulin-associated disease, the composition including: (i) insulin; and (ii) an isolated peptide having activity to glucagon, GLP-1, and GIP receptors.

In another embodiment, the present invention is directed to a pharmaceutical composition for reducing weight gain caused by insulin administration, the composition including: (i) insulin; and (ii) an isolated peptide having activity to glucagon, GLP-1, and GIP receptors.

In the composition according to any one of the previous embodiments, the insulin is in the form of a long-acting conjugate in which a biocompatible substance capable of increasing the *in vivo* half-life of the insulin is conjugated to the insulin.

In the composition according to any one of the previous embodiments, the isolated peptide having activity to glucagon, GLP-1, and GIP receptors is in the form of a long-acting conjugate in which a biocompatible substance capable of increasing the *in vivo* half-life of the isolated peptide is conjugated to the isolated peptide.

In the composition according to any one of the previous embodiments, the insulin is in the form of a long-acting conjugate in which a biocompatible substance capable of increasing the *in vivo* half-life of the insulin is conjugated to the insulin; and
the isolated peptide having activity to glucagon, GLP-1, and GIP receptors is in the form of a long-acting conjugate in which a biocompatible substance capable of increasing the *in vivo* half-life of the isolated peptide is conjugated to the isolated peptide.

In the composition according to any one of the previous embodiments, the composition is administered to a subject in need of insulin administration.

In the composition according to any one of the previous embodiments, the insulin-associated disease is selected from the group consisting of insulin resistance disorders, diabetes, hyperglycemia, and obesity.

In the composition according to any one of the previous embodiments, the composition has both a blood glucose lowering effect and an effect of suppressing weight gain caused by administration of insulin alone.

In the composition according to any one of the previous embodiments, the composition includes both of: insulin or a long-acting conjugate thereof; and an isolated peptide having activity to glucagon, GLP-1, and GIP receptors or a long-acting conjugate thereof,
wherein the insulin or the conjugate thereof and the isolated peptide having activity to glucagon, GLP-1, and GIP receptors or the long-acting conjugate thereof are contained at a molar ratio of 1:1 to 100:1, or wherein the insulin or the conjugate thereof and the isolated peptide having activity to glucagon, GLP-1, and GIP receptors or the long-acting conjugate thereof are contained at a molar ratio of 1:1 to 1:100.

In the composition according to any one of the previous embodiments, the isolated peptide having activity to glucagon, GLP-1, and GIP receptors is an analog of native glucagon, which has an alteration selected from the group consisting of a substitution, an addition, a deletion, a modification, and a combination thereof of at least one amino acid in the sequence of the native glucagon.

In the composition according to any one of the previous embodiments, an amino acid sequence to be added is derived from the amino acid sequence of native GLP-1, native GIP, or native exendin-4.

In the composition according to any one of the previous embodiments, the isolated peptide having activity to glucagon, GLP-1, and GIP receptors contains an amino acid sequence represented by General Formula 1 below: 
Xaa1–Xaa2–Xaa3–Gly–Thr–Phe–Xaa7–Ser–Asp–Xaa10–Ser–Xaa12–
Xaa13–Xaa14–Xaa15–Xaa16–Xaa17–Xaa18–Xaa19–Xaa20–Xaa21–Phe–
Xaa23–Xaa24–Trp–Leu–Xaa27–Xaa28–Xaa29–Xaa30–R1 (General Formula 1,
SEQ ID NO: 103),
 wherein, in General Formula 1,
Xaa1 is histidine (His, H), 4-imidazoacetyl (CA), or tyrosine (Tyr, Y);
Xaa2 is glycine (Gly, G), α-methyl-glutamic acid, or aminoisobutyric acid (Aib);
Xaa3 is glutamic acid (Glu, E) or glutamine (Gln, Q);
Xaa7 is threonine (Thr, T) or isoleucine (Ile, I);
Xaa10 is leucine (Leu, L), tyrosine (Tyr, Y), lysine (Lys, K), cysteine (Cys, C), or valine (Val, V);
Xaa12 is lysine (Lys, K), serine (Ser, S), or isoleucine (Ile, I);
Xaa13 is glutamine (Gln, Q), tyrosine (Tyr, Y), alanine (Ala, A), or cysteine (Cys, C);
Xaa14 is leucine (Leu, L), methionine (Met, M), or tyrosine (Tyr, Y);
Xaa15 is cysteine (Cys, C), aspartic acid (Asp, D), glutamic acid (Glu, E), or leucine (Leu, L);
Xaa16 is glycine (Gly, G), glutamic acid (Glu, E), or serine (Ser, S);
Xaa17 is glutamine (Gln, Q), arginine (Arg, R), isoleucine (Ile, I), glutamic acid (Glu, E), cysteine (Cys, C), or lysine (Lys, K);
Xaa18 is alanine (Ala, A), glutamine (Gln, Q), arginine (Arg, R), or histidine (His, H);
Xaa19 is alanine (Ala, A), glutamine (Gln, Q), cysteine (Cys, C), or valine (Val, V);
Xaa20 is lysine (Lys, K), glutamine (Gln, Q), or arginine (Arg, R);
Xaa21 is glutamic acid (Glu, E), glutamine (Gln, Q), leucine (Leu, L), cysteine (Cys, C), or aspartic acid (Asp, D);
Xaa23 is isoleucine (Ile, I) or valine (Val, V);
XXaa24 is alanine (Ala, A), glutamine (Gln, Q), cysteine (Cys, C), asparagine (Asn, N), aspartic acid (Asp, D), or glutamic acid (Glu, E);
Xaa27 is valine (Val, V), leucine (Leu, L), lysine (Lys, K), or methionine (Met, M);
Xaa28 is cysteine (Cys, C), lysine (Lys, K), alanine (Ala, A), asparagine (Asn, N), or aspartic acid (Asp, D);
Xaa29 is cysteine (Cys, C), glycine (Gly, G), glutamine (Gln, Q), threonine (Thr, T), glutamic acid (Glu, E), or histidine (His, H);
Xaa30 is cysteine (Cys, C), glycine (Gly, G), lysine (Lys, K), or histidine (His, H), or is absent; and
R1 is cysteine (Cys, C), GKKNDWKHNIT (SEQ ID NO: 106), m-SSGAPPPS-n (SEQ ID NO: 107), or m-SSGQPPPS-n (SEQ ID NO: 108), or is absent,
wherein m is Cys, Pro, or Gly-Pro, and n is Cys, Gly, Ser, or His-Gly, or is absent.

In the composition according to any one of the previous embodiments, Xaa14 is leucine or methionine; and Xaa15 is cysteine, aspartic acid, or leucine.

In the composition according to any one of the previous embodiments, in General Formula 1,
Xaa2 is glycine, α-methyl-glutamic acid, or Aib;
Xaa7 is threonine,
Xaa10 is tyrosine, cysteine, or valine;
Xaa12 is lysine or isoleucine;
Xaa13 is tyrosine, alanine, glutamine, or cysteine;
Xaa14 is leucine, cysteine, or methionine;
Xaa15 is cysteine, leucine, glutamic acid, or aspartic acid;
Xaa17 is glutamine, arginine, isoleucine, cysteine, glutamic acid, or lysine;
Xaa18 is alanine, glutamine, arginine, or histidine;
Xaa19 is alanine, glutamine, valine, or cysteine;
Xaa20 is lysine, arginine, or glutamine;
Xaa21 is glutamic acid, glutamine, leucine, cysteine, or aspartic acid;
Xaa23 is isoleucine or valine;
Xaa24 is cysteine, alanine, glutamine, asparagine, glutamic acid, or aspartic acid; and
Xaa27 is leucine or lysine.

In the composition according to any one of the previous embodiments, the peptide contains an amino acid sequence represented by General Formula 2 below: 
Xaa1–Xaa2–Gln–Gly–Thr–Phe–Thr–Ser–Asp–Xaa10–Ser–Lys–Xaa13–
Xaa14–Xaa15–Xaa16–Xaa17–Xaa18–Xaa19–Xaa20–Xaa21–Phe–Xaa23–
Xaa24–Trp–Leu–Leu–Xaa28–Xaa29–Xaa30–Xaa31–Ser–Ser–Gly–Gln–Pro–Pro–
Pro–Ser–Xaa40 (General Formula 2, SEQ ID NO: 104),
 wherein, in General Formula 2,
Xaa1 is 4-imidazoacetyl, histidine, or tyrosine;
Xaa2 is glycine, α-methyl-glutamic acid, or Aib;
Xaa10 is tyrosine or cysteine;
Xaa13 is alanine, glutamine, tyrosine, or cysteine;
Xaa14 is leucine, methionine, or tyrosine;
Xaa15 is aspartic acid, glutamic acid, or leucine;
Xaa16 is glycine, glutamic acid, or serine;
Xaa17 is glutamine, arginine, isoleucine, glutamic acid, cysteine, or lysine;
Xaa18 is alanine, glutamine, arginine, or histidine;
Xaa19 is alanine, glutamine, cysteine, or valine;
Xaa20 is lysine, glutamine, or arginine;
Xaa21 is cysteine, glutamic acid, glutamine, leucine, or aspartic acid;
Xaa23 is isoleucine or valine;
Xaa24 is cysteine, alanine, glutamine, asparagine, or glutamic acid;
Xaa28 is lysine, cysteine, asparagine, or aspartic acid;
Xaa29 is glycine, glutamine, cysteine, or histidine;
Xaa30 is cysteine, glycine, lysine, or histidine;
Xaa31 is proline or cysteine; and
Xaa40 is cysteine or is absent.

In the composition according to any one of the previous embodiments, in General Formula 1,
Xaa2 is glycine, α-methyl-glutamic acid, or Aib;
Xaa7 is threonine;
Xaa10 is tyrosine, cysteine, or valine;
Xaa12 is lysine or isoleucine;
Xaa13 is tyrosine, alanine, or cysteine;
Xaa14 is leucine or methionine;
Xaa15 is cysteine or aspartic acid;
Xaa17 is glutamine, arginine, isoleucine, cysteine, or lysine;
Xaa18 is alanine, arginine, or histidine;
Xaa19 is alanine, glutamine, or cysteine;
Xaa20 is lysine or glutamine;
Xaa21 is glutamic acid, cysteine, or aspartic acid;
Xaa23 is valine;
Xaa24 is alanine, glutamine, cysteine, asparagine, or aspartic acid; and
Xaa27 is leucine or lysine.

In the composition according to any one of the previous embodiments, in General Formula 2,
Xaa13 is alanine, tyrosine, or cysteine;
Xaa15 is aspartic acid or glutamic acid;
Xaa17 is glutamine, arginine, cysteine, or lysine;
Xaa18 is alanine, arginine, or histidine;
Xaa21 is cysteine, glutamic acid, glutamine, or aspartic acid;
Xaa23 is isoleucine or valine;
Xaa24 is cysteine, glutamine, or asparagine;
Xaa28 is cysteine, asparagine, or aspartic acid;
Xaa29 is glutamine, cysteine, or histidine; and
Xaa30 is cysteine, lysine, or histidine.

In the composition according to any one of the previous embodiments, in General Formula 1,
Xaa2 is α-methyl-glutamic acid or Aib;
Xaa7 is threonine;
Xaa10 is tyrosine or cysteine;
Xaa12 is lysine or isoleucine;
Xaa13 is tyrosine, alanine, or cysteine;
Xaa14 is leucine or methionine;
Xaa15 is cysteine or aspartic acid;
Xaa16 is glutamic acid;
Xaa17 is arginine, isoleucine, cysteine, or lysine;
Xaa18 is alanine, arginine, or histidine;
Xaa19 is alanine, glutamine, or cysteine;
Xaa20 is lysine or glutamine;
Xaa21 is glutamic acid or aspartic acid;
Xaa23 is valine;
Xaa24 is glutamine, asparagine, or aspartic acid;
Xaa27 is leucine; and
Xaa28 is cysteine, alanine, asparagine, or aspartic acid.

In the composition according to any one of the previous embodiments, in General Formula 1,
Xaa1 is histidine or 4-imidazoacetyl;
Xaa2 is α-methyl-glutamic acid or Aib;
Xaa3 is glutamine;
Xaa7 is threonine;
Xaa10 is tyrosine;
Xaa12 is isoleucine;
Xaa13 is alanine or cysteine;
Xaa14 is methionine;
Xaa15 is aspartic acid;
Xaa16 is glutamic acid;
Xaa17 is isoleucine or lysine;
Xaa18 is alanine or histidine;
Xaa19 is glutamine or cysteine;
Xaa20 is lysine;
Xaa21 is aspartic acid;
Xaa23 is valine;
Xaa24 is asparagine;
Xaa27 is leucine;
Xaa28 is alanine or asparagine;
Xaa29 is glutamine or threonine; and
Xaa30 is cysteine or lysine, or is absent.

In the composition according to any one of the previous embodiments, the peptide contains an amino acid sequence represented by General Formula 3 below: 
Xaa1–Xaa2–Gln–Gly–Thr–Phe–Thr–Ser–Asp–Tyr–Ser–Lys–Xaa13–Leu–
Asp–Glu–Xaa17–Xaa18–Xaa19–Lys–Xaa21–Phe–Val–Xaa24–Trp–Leu–Leu–
Xaa28–Xaa29–Xaa30–Xaa31–Ser–Ser–Gly–Gln–Pro–Pro–Pro–Ser–Xaa40
(General Formula 3, SEQ ID NO: 105),
 wherein, in General Formula 3,
Xaa1 is histidine or tyrosine;
Xaa2 is α-methyl-glutamic acid or Aib;
Xaa13 is alanine, tyrosine, or cysteine;
Xaa17 is arginine, cysteine, or lysine;
Xaa18 is alanine or arginine;
Xaa19 is alanine or cysteine;
Xaa21 is glutamic acid or aspartic acid;
Xaa24 is glutamine or asparagine;
Xaa28 is cysteine or aspartic acid;
Xaa29 is cysteine, histidine, or glutamine;
Xaa30 is cysteine or histidine;
Xaa31 is proline or cysteine; and
Xaa40 is cysteine or is absent.

In the composition according to any one of the previous embodiments, R1 is cysteine, GKKNDWKHNIT (SEQ ID NO: 106), CSSGQPPPS (SEQ ID NO: 109), GPSSGAPPPS (SEQ ID NO: 110), GPSSGAPPPSC (SEQ ID NO: 111), PSSGAPPPS (SEQ ID NO: 112), PSSGAPPPSG (SEQ ID NO: 113), PSSGAPPPSHG (SEQ ID NO: 114), PSSGAPPPSS (SEQ ID NO: 115), PSSGQPPPS (SEQ ID NO: 116), or PSSGQPPPSC (SEQ ID NO: 117), or is absent.

In the composition according to any one of the previous embodiments, the isolated peptide having activity to glucagon, GLP-1, and GIP receptors contains an amino acid sequence selected from SEQ ID NOS: 1 to 102.

In the composition according to any one of the previous embodiments, the isolated peptide having activity to glucagon, GLP-1, and GIP receptors contains the amino acid sequence set forth in SEQ ID NO: 42.

In the composition according to any one of the previous embodiments, in the general formulas, the amino acids at positions 16 and 20 from the N-terminus form a ring with each other.

In the composition according to any one of the previous embodiments, the C-terminus of the isolated peptide having activity to glucagon, GLP-1, and GIP receptors is amidated.

In the composition according to any one of the previous embodiments, the insulin is native insulin, or an insulin analog which has an alteration selected from the group consisting of a substitution, an addition, a deletion, a modification, and a combination thereof of at least one amino acid in the native insulin.

In the composition according to any one of the previous embodiments, the insulin analog has an alteration of at least one amino acid selected from the group consisting of the amino acids at positions 1, 2, 3, 5, 8, 10, 12, 16, 23, 24, 25, 26, 27, 28, 29, and 30 in the B-chain and the amino acids at positions 1, 2, 5, 8, 10, 12, 14, 16, 17, 18, 19, and 21 in the A-chain of the native insulin, the alteration being a substitution with another amino acid, a deletion, or a combination thereof.

In the composition according to any one of the previous embodiments, the insulin analog includes: an A-chain of SEQ ID NO: 119 represented by General Formula 4; and a B-chain of SEQ ID NO: 120 represented by General Formula 5: 
[General Formula 4]
Xaa1–Xaa2–Val–Glu–Xaa5–Cys–Cys–Thr–Ser–Ile–Cys–Xaa12–Leu–
Xaa14–Gln–Xaa16–Glu–Asn–Xaa19–Cys–Xaa21 (SEQ ID NO: 119),
 wherein, in General Formula 4,
Xaa1 is alanine, glycine, glutamine, histidine, glutamic acid, or asparagine;
Xaa2 is alanine or isoleucine;
Xaa5 is alanine, glutamic acid, glutamine, histidine, or asparagine;
Xaa12 is alanine, serine, glutamine, glutamic acid, histidine, or asparagine;
Xaa14 is alanine, tyrosine, glutamic acid, histidine, lysine, aspartic acid, or asparagine;
Xaa16 is alanine, leucine, tyrosine, histidine, glutamic acid, or asparagine;
Xaa19 is alanine, tyrosine, serine, glutamic acid, histidine, threonine, or asparagine; and
Xaa21 is asparagine, glycine, histidine, or alanine, 
[General Formula 5]
Phe–Val–Asn–Gln–His–Leu–Cys–Xaa8–Ser–His–Leu–Val–Glu–Ala–Leu–
Xaa16–Leu–Val–Cys–Gly–Glu–Arg–Xaa23–Xaa24–Xaa25–Tyr–Xaa27–Xaa28–
Lys–Thr (SEQ ID NO: 120)
wherein, in General Formula 5,
Xaa8 is alanine or glycine;
Xaa16 is tyrosine, glutamic acid, serine, threonine, or aspartic acid, or is absent;
Xaa23 is glycine or alanine;
Xaa24 is alanine or phenylalanine;
Xaa25 is alanine, phenylalanine, aspartic acid, or glutamic acid, or is absent;
Xaa27 is threonine, or is absent; and
Xaa28 is proline, glutamic acid, or aspartic acid, or is absent
(wherein a peptide including the A-chain of SEQ ID NO: 121 and the B-chain of SEQ ID NO: 122 is excluded).

In the composition according to any one of the previous embodiments, the insulin analog has a substitution of at least one amino acid selected from the group consisting of the amino acids at positions 8, 23, 24, and 25 in the B-chain and the amino acids at positions 1, 2, and 19 in the A-chain of the native insulin with alanine, or a substitution of the amino acid at position 14 in the A-chain of the native insulin with glutamic acid or asparagine.

In the composition according to any one of the previous embodiments, the insulin analog contains an amino acid sequence selected from the group consisting of SEQ ID NOS: 124, 126, 128, 130, 132, 134, 136, 138, and 140.

In the composition according to any one of the previous embodiments, the insulin analog has an alteration by: a substitution of the amino acid at position 16 in the B-chain of the native insulin with glutamic acid; a deletion of the amino acid at position 25 in the B-chain of the native insulin; a substitution of the amino acid at position 14 in the A-chain of the native insulin with glutamic acid or alanine; or a combination thereof.

In the composition according to any one of the previous embodiments, the insulin analog contains an amino acid sequence of SEQ ID NO: 142 or 144.

In the composition according to any one of the previous embodiments, the insulin analog has an alteration by: a substitution of the amino acid at position 16 in the B-chain of the native insulin with glutamic acid, serine, threonine, or aspartic acid; a substitution of the amino acid at position 25 in the B-chain of the native insulin with aspartic acid or glutamic acid; a substitution of the amino acid at position 14 in the A-chain of the native insulin with histidine, lysine, alanine, or aspartic acid; a substitution of the amino acid at position 19 in the A-chain of the native insulin with glutamic acid, serine, or threonine; or a combination thereof.

In the composition according to any one of the previous embodiments, the insulin analog contains an amino acid sequence selected from the group consisting of SEQ ID NOS: 146, 148, 150, 152, 154, 156, 158, 160, 162, 164, 166, 168, and 170.

In the composition according to any one of the previous embodiments, the insulin analog is in the form of two polypeptide chains composed of an A-chain of SEQ ID NO: 119 represented by General Formula 4 and a B-chain of SEQ ID NO: 120 represented by General Formula 5.

In the composition according to any one of the previous embodiments, the A-chain and the B-chain are linked via a disulfide linkage.

In the composition according to any one of the previous embodiments, the conjugate is represented by Chemical Formula 1:

[Chemical Formula 1] X―Lₐ―F

wherein,
X is the insulin or the isolated peptide having activity to glucagon, GLP-1, and GIP receptors;
L is a linker;
a is 0 or a natural number, with the proviso that when a is 2 or greater, each L is independent from each other;
F is a substance capable of increasing the half-life of X; and
"―" is a covalent or non-covalent linkage.

In the composition according to any one of the previous embodiments, F is selected from the group consisting of polymers, fatty acids, cholesterol, albumin and fragments thereof, albumin-binding substances, polymers of repeating units of particular amino acid sequences, antibodies, antibody fragments, FcRn-binding substances, *in vivo* connective tissues, nucleotides, fibronectin, transferrin, saccharides, heparin, and elastin.

In the composition according to any one of the previous embodiments, the polymers are selected from the group consisting of polyethylene glycol, polypropylene glycol, ethylene glycol―propylene glycol copolymers, polyoxyethylated polyols, polyvinyl alcohols, polysaccharides, dextran, polyvinyl ethyl ether, biodegradable polymers, lipid polymers, chitin, hyaluronic acid, oligonucleotides, and a combination thereof.

In the composition according to any one of the previous embodiments, F is an immunoglobulin Fc region.

In the composition according to any one of the previous embodiments, F is an IgG Fc region.

In the composition according to any one of the previous embodiments, the immunoglobulin Fc region is aglycosylated.

In the composition according to any one of the previous embodiments, the immunoglobulin Fc region is selected from the group consisting of:
(a) a CH1 domain, a CH2 domain, a CH3 domain, and a CH4 domain;
(b) a CH1 domain and a CH2 domain;
(c) a CH1 domain and a CH3 domain;
(d) a CH2 domain and a CH3 domain;
(e) a combination of at least one of a CH1 domain, a CH2 domain, a CH3 domain, and a CH4 domain and an immunoglobulin hinge region or a moiety of the hinge region; and
(f) a dimer of each domain of a heavy chain constant region and a light chain constant region.

In the composition according to any one of the previous embodiments, the immunoglobulin Fc region has a deletion of a site capable of forming a disulfide linkage, a deletion of some amino acids at the N-terminus of native Fc, an addition of a methionine residue at the N-terminus of native Fc, a deletion of a complement-binding site, or deletion of an antibody-dependent cell-mediated cytotoxicity (ADCC) site.

In the composition according to any one of the previous embodiments, the immunoglobulin Fc region is an immunoglobulin Fc fragment derived from IgG, IgA, IgD, IgE, or IgM.

In the composition according to any one of the previous embodiments, the immunoglobulin Fc region is a hybrid of domains with different origins derived from immunoglobulins selected from the group consisting of IgG, IgA, IgD, IgE, and IgM.

In the composition according to any one of the previous embodiments, L is selected from the group consisting of peptides, fatty acids, sugars, polymers, low-molecular-weight compounds, nucleotides, and combinations thereof.

In the composition according to any one of the previous embodiments, the polymers are selected from the group consisting of polyethylene glycol, polypropylene glycol, ethylene glycol-propylene glycol copolymers, polyoxyethylated polyols, polyvinyl alcohols, polysaccharides, dextran, polyvinyl ethyl ether, biodegradable polymers, lipid polymers, chitin, hyaluronic acid, oligonucleotides, and combinations thereof.

In the composition according to any one of the previous embodiments, L is polyethylene glycol.

An aspect of the present invention is directed to a complex preparation for weight loss in an insulin-administered patient, the complex preparation including: (i) insulin; and (ii) an isolated peptide having activity to glucagon, GLP-1, and GIP receptors.

An aspect of the present invention is directed to a kit for prevention or treatment of an insulin-associated disease, the kit including: (i) insulin; and (ii) an isolated peptide having activity to glucagon, GLP-1, and GIP receptors.

An aspect of the present invention is directed to use of the composition in the preparation of a medicinal material.

An aspect of the present invention is directed to a method for prevention or treatment of an insulin-associated disease, the method including administering to a subject (i) insulin; and (ii) an isolated peptide having activity to glucagon, GLP-1, and GIP receptors.

An aspect of the present invention is directed to a method for reducing weight gain caused by insulin administration, the method including administering to a subject an isolated peptide having activity to glucagon, GLP-1, and GIP receptors.

An aspect of the present invention is directed to a method for reducing weight gain caused by insulin administration, the method including administering to a subject (i) insulin; and (ii) an isolated peptide having activity to glucagon, GLP-1, and GIP receptors.

### [Mode for Carrying Out the Invention]

Hereinafter, the present invention will be described in detail.

Each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description below.

Further, a person skilled in the art will recognize, or be able to ascertain, by using no more than routine experimentation, many equivalents to the specific embodiments of the invention described herein. Such equivalents are intended to be encompassed by the present invention.

Throughout this specification, conventional one-letter and three-letter codes for naturally occurring amino acids as well as three-letter codes generally allowed for other amino acids, such as α-aminoisobutyric acid (Aib), N-methylglycine (Sar), and α-methyl-glutamic acid, are used. The amino acids mentioned in abbreviations herein are described according to the IUPAC-IUB rules:
alanine (Ala, A), arginine (Arg, R)
asparagine (Asn, N), aspartic acid (Asp, D)
cysteine (Cys, C), glutamic acid (Glu, E)
glutamine (Gin, Q), glycine (Gly, G)
histidine (His, H), isoleucine (Ile, I)
leucine (Leu, L), lysine (Lys, K)
methionine (Met, M), phenylalanine (Phe, F)
proline (Pro, P), serine (Ser, S)
threonine (Thr, T), tryptophan (Trp, W)
tyrosine (Tyr, Y), valine (Val, V)

An aspect of the present invention is to provide a composition containing: (i) insulin; and (ii) an isolated peptide having activity to glucagon, glucagon-like peptide-1 (GLP-1), and glucose-dependent insulinotropic polypeptide (GIP) receptors (also referred to as "triple agonist").

In an embodiment, the present provides a pharmaceutical composition for prevention or treatment of an insulin-associated disease, the composition containing: (i) insulin; and (ii) an isolated peptide having activity to glucagon, GLP-1, and GIP receptors.

In another embodiment, the present invention provides a pharmaceutical composition for reducing weight loss caused by insulin administration, the composition containing: (i) insulin; and (ii) an isolated peptide having activity to glucagon, GLP-1, and GIP receptors.

Specifically, the composition may contain:
(a) insulin; and an isolated peptide having activity to glucagon, GLP-1, and GIP receptors;
(b) a long-acting conjugate of insulin in which insulin and a biocompatible substance capable of increasing the *in vivo* half-life of the insulin are conjugated to each other; and an isolated peptide having activity to glucagon, GLP-1, and GIP receptors;
(c) insulin; and a long-acting conjugate of a triple agonist in which an isolated peptide having activity to glucagon, GLP-1, and GIP receptors and a biocompatible substance capable of increasing the *in vivo* half-life of the isolated peptide are conjugated to each other; or
(d) a long-acting conjugate of insulin; and a long-acting conjugate of a triple agonist.

The pharmaceutical composition for prevention or treatment of an insulin-associated disease of the present invention contains: insulin or a long-acting conjugate thereof; and a triple agonist or a long-acting conjugate thereof, wherein the composition can mitigate side effects (*e*.*g*., weight gain) caused by insulin administration while exhibiting medicinal efficacy of insulin having a prevention or treatment effect of an insulin-associated disease occurring due to the lack, deficiency, or dysfunction of insulin.

Exemplary embodiments of the present invention verified a blood glucose lowering effect and a weight gain inhibitory effect in db/db mice administered the composition containing insulin or a long-acting conjugate thereof and a triple agonist or a long-acting conjugate thereof, indicating that the pharmaceutical composition according to the present invention can relieve side effects of insulin while maintaining medicinal effects of insulin.

As used herein, the term "insulin-associated disease" refers to a disease caused by an abnormality in blood glucose control function inherent to insulin, such as the lack, deficiency, or dysfunction of insulin, and any disease for which a prevention or treatment effect can be expected through the administration of insulin is included in the scope of the present invention. Specifically, the insulin-associated disease may be selected from the group consisting of insulin resistance disorders, diabetes, hyperglycemia, and obesity.

The administration of insulin for the treatment of insulin-associated diseases can achieve desired treatment effects, but causes unintended side effects, such as weight gain, thereby causing other types of diseases and suffering for patients. The pharmaceutical composition according to the present invention can show treatment effects for insulin-associated diseases, while inhibiting weight gain as a side effect of insulin, through the combined use of insulin and a triple agonist.

The composition of the present invention may contain insulin or a long-acting conjugate thereof and a triple agonist or a long-acting conjugate thereof at a content ratio at which treatment effects for insulin-associated diseases can be exhibited and side effects, especially weight gain, can be reduced.

Specifically, the composition may contain both insulin or a long-acting conjugate thereof and a triple agonist or a long-acting conjugate thereof, wherein the composition may contain the insulin and the triple agonist at a molar ratio of 1:1 to 100:1 or 1:1 to 1:100, but is not limited thereto.

In an embodiment of the present invention, the composition may be provided for a therapeutic use by way of the combined use of insulin or a conjugate thereof and a substance having activity to glucagon, GLP-1, and GIP receptors or a conjugate thereof. The term "insulin" is as described above.

Herein, the term "composition" may be used interchangeably with "combination", which contains insulin or a conjugate thereof and a substance having activity to glucagon, GLP-1, and GIP receptors. The composition may be provided in the form of a kit.

As used herein, the "combination" has uses for combined administration of insulin or a conjugate thereof and a substance having activity to glucagon, GLP-1, and GIP receptors or a conjugate thereof, and the term may be understood as being synonymous with "combined use". The composition may be administered:
a) in the form of one mixture in which (i) insulin or a conjugate thereof and (ii) a substance having activity to glucagon, GLP-1, and GIP receptors or a conjugate thereof are mixed; or
b) in the form that (i) insulin or a conjugate thereof and (ii) a substance having activity to glucagon, GLP-1, and GIP receptors or a conjugate thereof are separated, but is not limited thereto.

When the composition is in the form that insulin or a conjugate thereof and a substance having activity to glucagon, GLP-1, and GIP receptors or a conjugate thereof are separated, insulin or the conjugate thereof and the substance having activity to glucagon, GLP-1, and GIP receptors or the conjugate thereof may be prepared as separate preparations and administered simultaneously, separately, sequentially, or in reverse order.

In the present invention, combined administration is to be understood to mean not only simultaneous administration, but also an administration form in which insulin or a conjugate thereof and a substance having activity to glucagon, GLP-1, and GIP receptors or a conjugate thereof act together on a subject, so that each works at a level equal to or higher than its original function. Therefore, the use of the term "combined" is to be understood to represent simultaneous, separate, sequential, or reverse-order administration. When the administration is sequential, reverse-order, or separate, the order of administration is not particularly limited, but the delay in administering a second ingredient should not be such that the beneficial effect of the combined use is lost.

In the present invention, the term "composition" refers to a combination itself, containing insulin or a conjugate thereof and a substance having activity to glucagon, GLP-1, and GIP receptors or a conjugate thereof, or may be a composition which contains the combination and has therapeutic uses, but is not limited thereto. An example thereof may be a composition which has prevention or treatment uses for an insulin-associated disease, but is not limited thereto.

The composition according to the present invention is for combined administration of: insulin or a conjugate thereof; and a substance having activity to glucagon, GLP-1, and GIP receptors or a conjugate thereof, wherein the insulin or the conjugate thereof and the substance having activity to glucagon, GLP-1, and GIP receptors or the conjugate thereof may be prepared as one preparation or prepared separately. Specifically, insulin or a conjugate thereof and a substance having activity to glucagon, GLP-1, and GIP receptors or a conjugate thereof may be administered simultaneously, separately, sequentially, or in reverse order, but are not limited thereto.

In the present invention, the term "kit" may contain the combination or composition according to the present invention for combined administration of insulin or a conjugate thereof and a substance having activity to glucagon, GLP-1, and GIP receptors or a conjugate thereof. Specifically, the kit according to the present invention may contain: one preparation prepared of insulin or a conjugate thereof and a substance having activity to glucagon, GLP-1, and GIP receptors or a conjugate thereof; or separate preparations of insulin or a conjugate thereof and a substance having activity to glucagon, GLP-1, and GIP receptors or a conjugate thereof, and the kit may further contain a substance necessary for combined administration of the two substances, but is not limited thereto.

The insulin and the substance having activity to glucagon, GLP-1, and GIP receptors include insulin and various substances having significant levels of activity to glucagon, GLP-1, and GIP receptors, for example, compound- or peptide-type substances.

The "peptide having activity to glucagon, GLP-1, and GIP receptors" may be used interchangeably with the triple agonist in the present invention. As for triple agonists and long-acting conjugates thereof, the full text of International Patent Publication Nos. WO 2017/116204 and WO 2017/116205 are incorporated herein by reference.

Examples of the triple agonist include various substances, such as various peptides, with significant levels of activity to glucagon, GLP-1, and GIP receptors.

Although not particularly limited, as for the substances with significant levels of activity to glucagon, GLP-1, and GIP receptors, the *in vitro* activity of the substances to one or more, specifically two or more, and more specifically all the three among glucagon, GLP-1, and GIP receptors, is 0.1% or more, 1% or more, 2% or more, 3 % or more, 4% or more, 5% or more, 6% or more, 7% or more, 8% or more, 9% or more, 10% or more, 20% or more, 30% or more, 40% or more, 50% or more, 60% or more, 70% or more, 80% or more, 90% or more, or 100% or more compared with native ligands (native glucagon, native GLP-1, and native GIP) with respect to the corresponding receptors.

A method of measuring the *in vitro* activity of these triple agonists may refer to Test Example 1 herein, but is not particularly limited thereto.

Meanwhile, the triple agonist is characterized by having one or more, two or more, specifically three of the following activity i) to activity iii), specifically having significant levels of activity:
i) activation of the GLP-1 receptor; ii) activation of the glucagon receptor; and iii) activation of the GIP receptor.

The activation of a receptor may include, for example, showing 0.1% or more, 1% or more, 2% or more, 3% or more, 4% or more, 5% or more, 6% or more, 7% or more, 8% or more, 9% or more, 10% or more, 20% or more, 30% or more, 40% or more, 50% or more, 60% or more, 70% or more, 80% or more, 90% or more, and 100% or more of *in vitro* activity to the receptor, compared with the native form. However, the activation is not limited thereto.

The triple agonist may have an increased *in vivo* half-life compared with any one of native GLP-1, native glucagon, and native GIP, but is not particularly limited thereto.

Although not particularly limited, these peptides may be those which do not occur naturally.

Specifically, the triple agonist may be an analog of native glucagon, but is not particularly limited thereto.

The analog of native glucagon according to the present invention may include peptides having at least one difference in the amino acid sequence compared with native glucagon, peptides obtained by alteration through modification of the native glucagon sequence, and mimetics of native glucagon.

Meanwhile, although not particularly limited, the native glucagon may have the following amino acid sequence:

His–Ser–Gln–Gly–Thr–Phe–Thr–Ser–Asp–Tyr–Ser–Lys–Tyr–Leu–Asp–
Ser–Arg–Arg–Ala–Gln–Asp–Phe–Val–Gln–Trp–Leu–Met–Asn–Thr (SEQ ID
NO: 118)

Specifically, the triple agonist may be an analog of native glucagon having an alteration selected from the group consisting of a substitution, an addition, a deletion, a modification, and a combination thereof of at least one amino acid in the sequence of native glucagon, but is not particularly limited thereto.

The substitution of an amino acid may include both a substitution with an amino acid and a substitution with a non-native compound.

The addition may be made at the N-terminus and/or C-terminus of the triple agonist. The length of amino acids to be added is not particularly limited, but 1 or more, 2 or more, 3 or more, 4 or more, 5 or more, 6 or more, 7 or more, 8 or more, 9 or more, 10 or more, and 11 or more amino acids may be added, and in a broad sense, the addition may include an addition of a polypeptide, but is not particularly limited thereto.

The amino acid to be introduced into native glucagon may be selected from the group consisting of tyrosine, α-methyl-glutamic acid, Aib, methionine, glutamic acid, histidine, lysine, leucine, isoleucine, glutamine, valine, glycine, alanine, cysteine, serine, alanine, aspartic acid, and arginine, but is not particularly limited thereto.

For example, the amino acid sequence to be added may be at least one amino acid sequence derived from the amino acid sequence of native GLP-1, native GIP, or native exendin-4.

Such a triple agonist may include an intramolecular bridge (*e*.*g*., a covalent bridge or non-covalent bridge), and specifically, the triple agonist is in the form of including a ring, for example, in the form that a ring is formed between the amino acids at positions 16 and 20 in the triple agonist, but is not particularly limited thereto.

Non-limiting examples of the ring may include a lactam bridge (or a lactam ring).

In addition, examples of the triple agonist include all of those which are modified to contain amino acids capable of forming a ring at a target position, so as to include the ring.

For example, a pair of the amino acids at positions 16 and 25 in the triple agonist may be substituted with either glutamic acid or lysine capable of forming a ring, respectively, but is not limited thereto.

This ring may be formed between amino acid side chains within the triple agonist, for example, in the form of a lactam ring formation between a side chain of lysine and a side chain of glutamic acid, but is not particularly limited thereto.

Examples of the triple agonist prepared by way of a combination of these methods include: peptides which differ from natural glucagon in view of at least one amino acid sequence, in which the α-carbon of an amino acid residue at the N-terminus is removed, and which have activity to glucagon, GLP-1, and GIP receptors, and the like, but are not limited thereto, and triple agonists to be applied to the present invention can be prepared by way of a combination of various methods for analog preparation.

Although not particularly limited, in the triple agonist of the present invention, for increasing the *in vivo* half-life of the triple agonist, some amino acids may be substituted with other amino acids or non-natural compounds in order to avoid recognition by agonist degradation enzymes.

Specifically, the triple agonist of the present invention may be a peptide having an increased *in vivo* half-life by avoiding recognition by the degradation enzyme through the substitution of the second amino acid sequence among the amino acid sequences of the triple agonist, but any amino acid substitution or modification for avoiding recognition by *in vivo* degradation enzymes is included without limitation.

Such an alteration for the preparation of an analog of native glucagon includes: an alteration using L-type or D-type amino acid and/or non-native amino acid; and/or all of alterations through a modification of the sequence of the native form, for example, an alteration of a side chain functional group, an intramolecular covalent linkage such as a ring formation between side chains, methylation, acylation, ubiquitination, phosphorylation, aminohexanation, biotinylation, and the like.

In addition, such an alteration also includes all of additions of one or more amino acids at the amino and/or carboxy terminus of native glucagon.

Examples of the amino acids to be substituted or added may include not only the 20 amino acids commonly found in human proteins, but also atypical amino acids or those which do not occur naturally. Commercial sources of atypical amino acids may include Sigma-Aldrich, ChemPep, and Genzyme Pharmaceuticals. The sequences of peptides containing these amino acids and typical peptides can be synthesized by and purchased from commercial suppliers, e.g., American Peptide Company and Bachem in the USA, or Antigen in Korea.

The amino acid derivatives may also be accessible in a similar manner, and for example, 4-imidazoacetic acid or the like may be used.

The peptide having activity to glucagon, GLP-1, and GIP receptors according to the present invention may be in such a form that, for the protection from *in vivo* protein breaking enzymes and the increase in stability, the N-terminus and/or C-terminus is chemically modified or protected by organic groups or amino acids are added to the termini or the like of the peptide.

In particular, chemically synthesized peptides have electrically charged N- and C-termini, and thus for elimination of these charges, the N-terminus may be acetylated and/or the C-terminus may be amidated, but these are not particularly limited thereto. In addition, a form in which the C-terminus is not altered, that is, a form of having a carboxyl group, is also included.

The peptide having activity to glucagon, GLP-1, and GIP receptors according to the present invention, that is, the triple agonist, encompasses all of the peptide itself, a salt thereof (e.g., a pharmaceutically acceptable salt of the peptide), or a solvate thereof. The peptide having activity to glucagon, GLP-1, and GIP receptors may be in the form of any pharmaceutically acceptable salt.

The type of the salt is not particularly limited. However, the salt is preferably in a form that is safe and effective to a subject, e.g., a mammal, but is not particularly limited thereto.

The term "pharmaceutically acceptable" indicates a substance which can be effectively used for desired uses, within the range of pharmaco-medical decision, without inducing excessive toxicity, irritation, allergic responses, or the like.

As used herein, the "pharmaceutically acceptable salt" includes salts derived from pharmaceutically acceptable inorganic acids, organic acids, or bases. Suitable examples of the acids may include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, benzene sulfonic acid, and the like. Examples of the salts derived from suitable bases may include: alkali metals, such as sodium and potassium; alkaline earth metals, such as magnesium; ammonium; and the like.

As used herein, the term "solvate" refers to a complex formed between the peptide according to the present invention or a salt thereof and a solvent molecule.

It would be obvious that all the above description applies to insulin to be described later.

In a specific aspect, the peptide having activity to glucagon, GLP-1, and GIP receptors may contain an amino acid sequence represented by General Formula 1 below: 
Xaa1–Xaa2–Xaa3–Gly–Thr–Phe–Xaa7–Ser–Asp–Xaa10–Ser–Xaa12–
Xaa13–Xaa14–Xaa15–Xaa16–Xaa17–Xaa18–Xaa19–Xaa20–Xaa21–Phe–
Xaa23–Xaa24–Trp–Leu–Xaa27–Xaa28–Xaa29–Xaa30–R1 (General Formula 1,
SEQ ID NO: 103),
 wherein, in General Formula 1,
Xaa1 is histidine, 4-imidazoacetyl, or tyrosine;
Xaa2 is glycine, α-methyl-glutamic acid, or Aib;
Xaa3 is glutamic acid or glutamine;
Xaa7 is threonine or isoleucine;
Xaa10 is leucine, tyrosine, lysine, cysteine, or valine;
Xaa12 is lysine, serine, or isoleucine;
Xaa13 is glutamine, tyrosine, alanine, or cysteine;
Xaa14 is leucine, methionine, or tyrosine;
Xaa15 is cysteine, aspartic acid, glutamic acid, or leucine;
Xaa16 is glycine, glutamic acid, or serine;
Xaa17 is glutamine, arginine, isoleucine, glutamic acid, cysteine, or lysine;
Xaa18 is alanine, glutamine, arginine, or histidine;
Xaa19 is alanine, glutamine, cysteine, or valine;
Xaa20 is lysine, glutamine, or arginine;
Xaa21 is glutamic acid, glutamine, leucine, cysteine, or aspartic acid;
Xaa23 is isoleucine or valine;
XXaa24 is alanine, glutamine, cysteine, asparagine, aspartic acid, or glutamic acid;
Xaa27 is valine, leucine, lysine, or methionine;
Xaa28 is cysteine, lysine, alanine, asparagine, or aspartic acid;
Xaa29 is cysteine, glycine, glutamine, threonine, glutamic acid, or histidine;
Xaa30 is cysteine, glycine, lysine, or histidine, or is absent; and
R1 is cysteine, GKKNDWKHNIT (SEQ ID NO: 106), m-SSGAPPPS-n (SEQ ID NO: 107), or m-SSGQPPPS-n (SEQ ID NO: 108), or is absent,
wherein m is Cys, Pro, or Gly-Pro, and
n is Cys, Gly, Ser, or His-Gly, or is absent.

Examples of the triple agonist may include one which contains the amino acid sequence selected from the group consisting of SEQ ID NOS: 1 to 102 or one which may consist (essentially) of the amino acid sequence selected from the group consisting of SEQ ID NO: 1 to 102, but are not limited thereto.

Although described as "peptide consisting of a specific SEQ ID NO" herein, additions of nonsense sequences upstream or downstream of the amino acid sequence of the corresponding sequence number, naturally occurring mutations, and silent mutations thereof are not excluded as long as there exists the equivalent or corresponding activity to a peptide consisting of the amino acid sequence of the corresponding sequence number, and it is obvious that peptides having such a sequence addition or mutation are also within the scope of the present invention.

The above description can be applied to other embodiments or aspects of the present invention, but is not limited thereto.

Specifically, in General Formula 1 above, Xaa14 may be leucine or methionine; and Xaa15 may be cysteine, aspartic acid, or leucine. In such a case, the remaining variables except for Xaa14 and Xaa15 described in General Formula 1 may have a combination of the amino acids described above.

Examples of these peptides may be peptides containing or consisting (essentially) of an amino acid sequence selected from the group consisting of SEQ ID NOS: 1 to 12, 14 to 17, and 21 to 102, but are not limited thereto.

These peptides can significantly activate at least one of glucagon, GLP-1, and GIP receptors, but are not particularly limited thereto. Specifically, the peptides can significantly activate the GLP-1 receptor, or can further significantly activate the glucagon and/or GIP receptors, but are not particularly limited thereto.

Still more specifically,
in General Formula 1,
Xaa2 may be glycine, α-methyl-glutamic acid, or Aib;
Xaa7 may be threonine,
Xaa10 may be tyrosine, cysteine, or valine;
Xaa12 may be lysine or isoleucine;
Xaa13 may be tyrosine, alanine, glutamine, or cysteine;
Xaa14 may be leucine, cysteine, or methionine;
Xaa15 may be cysteine, leucine, glutamic acid, or aspartic acid;
Xaa17 may be glutamine, arginine, isoleucine, cysteine, glutamic acid, or lysine;
Xaa18 may be alanine, glutamine, arginine, or histidine;
Xaa19 may be alanine, glutamine, valine, or cysteine;
Xaa20 may be lysine, arginine, or glutamine;
Xaa21 may be glutamic acid, glutamine, leucine, cysteine, or aspartic acid;
Xaa23 may be isoleucine or valine;
Xaa24 may be cysteine, alanine, glutamine, asparagine, glutamic acid, or aspartic acid; and
Xaa27 may be leucine or lysine, but the variables are not particularly limited thereto.

The remaining variables of General Formula 1 except for the variables described in the present aspect may have a combination of the amino acids described above.

Still more specifically,
in General Formula 1,
Xaa2 may be glycine, α-methyl-glutamic acid, or Aib;
Xaa7 may be threonine,
Xaa10 may be tyrosine, cysteine, or valine;
Xaa12 may be lysine or isoleucine;
Xaa13 may be tyrosine, alanine, or cysteine;
Xaa14 may be leucine or methionine;
Xaa15 may be cysteine or aspartic acid;
Xaa17 may be glutamine, arginine, isoleucine, cysteine, or lysine;
Xaa18 may be alanine, arginine, or histidine;
Xaa19 may be alanine, glutamine, or cysteine;
Xaa20 may be lysine, or glutamine;
Xaa21 may be glutamic acid, cysteine, or aspartic acid;
Xaa23 may be valine;
Xaa24 may be alanine, glutamine, cysteine, asparagine, or aspartic acid; and
Xaa27 may be leucine or lysine, but the variables are not particularly limited thereto.

The remaining variables of General Formula 1 except for the variables described in the present aspect may have a combination of the amino acids described above.

Still more specifically,
in General Formula 1,
Xaa2 is α-methyl-glutamic acid or Aib;
Xaa7 is threonine,
Xaa10 is tyrosine or cysteine;
Xaa12 is lysine or isoleucine;
Xaa13 is tyrosine, alanine, or cysteine;
Xaa14 is leucine or methionine;
Xaa15 is cysteine or aspartic acid;
Xaa16 is glutamic acid;
Xaa17 is arginine, isoleucine, cysteine, or lysine;
Xaa18 is alanine, arginine, or histidine;
Xaa19 is alanine, glutamine, or cysteine;
Xaa20 is lysine, or glutamine;
Xaa21 is glutamic acid or aspartic acid;
Xaa23 is valine;
Xaa24 is glutamine, asparagine, or aspartic acid;
Xaa27 is leucine; and
Xaa28 is cysteine, alanine, asparagine, or aspartic acid.

The remaining variables of General Formula 1 except for the variables described in the present aspect may have a combination of the amino acids described above.

Specifically,
in General Formula 1,
Xaa1 may be histidine or 4-imidazoacetyl
Xaa2 may be α-methyl-glutamic acid or Aib;
Xaa3 may be glutamine;
Xaa7 may be threonine,
Xaa10 may be tyrosine;
Xaa12 may be isoleucine;
Xaa13 may be alanine or cysteine;
Xaa14 may be methionine;
Xaa15 may be aspartic acid;
Xaa16 may be glutamic acid;
Xaa17 may be isoleucine or lysine;
Xaa18 may be alanine or histidine;
Xaa19 may be glutamine or cysteine;
Xaa20 may be lysine;
Xaa21 may be aspartic acid;
Xaa23 may be valine;
Xaa24 may be asparagine;
Xaa27 may be leucine;
Xaa28 may be alanine or asparagine;
Xaa29 may be glutamine or threonine; and
Xaa30 may be cysteine or lysine, or may be absent.

The remaining variables of General Formula 1 except for the variables described in the present aspect may have a combination of the amino acids described above.

More specifically,
in General Formula 1,
Xaa2 may be glycine, α-methyl-glutamic acid or Aib;
Xaa3 may be glutamine;
Xaa7 may be threonine,
Xaa10 may be tyrosine, cysteine, or valine;
Xaa12 may be lysine;
Xaa13 may be tyrosine;
Xaa14 may be leucine;
Xaa15 may be aspartic acid;
Xaa16 may be glycine, glutamic acid, or serine;
Xaa17 may be glutamine, arginine, cysteine, or lysine;
Xaa18 may be alanine, arginine, or histidine;
Xaa19 may be alanine or glutamine;
Xaa20 may be lysine or glutamine;
Xaa21 may be glutamic acid, cysteine, or aspartic acid;
Xaa23 may be valine;
Xaa24 may be alanine, glutamine, or cysteine;
Xaa27 may be leucine or lysine; and
Xaa29 may be glycine, glutamine, threonine, or histidine, but the variables are not particularly limited thereto.

The remaining variables of General Formula 1 except for the variables described in the present aspect may have a combination of the amino acids described above.

These peptides having activity to glucagon, GLP-1, and GIP receptors may correspond to: peptides of which the activity to GLP-1 and glucagon receptors is significant and is higher than the activity to GIP receptor; peptides of which the activity to all of GLP-1, glucagon, and GIP receptors is significant; and peptides of which the activity to GLP-1 and GIP receptors is significant and is higher than the activity to the glucagon receptor, but the peptides are not limited thereto.

The peptides of which the activity to GLP-1 and glucagon receptors is significant and is higher than the activity to GIP receptor can provide a greater weight loss effect and a blood glucose control effect, and the peptides of which the activity to all of GLP-1, glucagon, and GIP receptors is significant can provide a maximized weight loss effect. However, the peptides are not limited thereto.

Examples of these peptides may include peptides containing or consisting (essentially) of an amino acid sequence selected from the group consisting of SEQ ID NOS: 8, 9, 21 to 37, 39, 42, 43, 49 to 61, 64 to 83, 85, 86, 88, 89, 91 to 93, and 95 to 102, but are not particularly limited thereto.

In a specific aspect, the peptide having activity to glucagon, GLP-1, and GIP receptors may contain an amino acid sequence represented by General Formula 2 below: 
Xaa1–Xaa2–Gln–Gly–Thr–Phe–Thr–Ser–Asp–Xaa10–Ser–Lys–Xaa13–
Xaa14–Xaa15–Xaa16–Xaa17–Xaa18–Xaa19–Xaa20–Xaa21–Phe–Xaa23–
Xaa24–Trp–Leu–Leu–Xaa28–Xaa29–Xaa30–Xaa31–Ser–Ser–Gly–Gln–Pro–Pro–
 Pro–Ser–Xaa40 (General Formula 2, SEQ ID NO: 104), wherein, in General Formula 2,
Xaa1 is 4-imidazoacetyl, histidine, or tyrosine;
Xaa2 is glycine, α-methyl-glutamic acid, or Aib;
Xaa10 is tyrosine or cysteine;
Xaa13 is alanine, glutamine, tyrosine, or cysteine;
Xaa14 is leucine, methionine, or tyrosine;
Xaa15 is aspartic acid, glutamic acid, or leucine;
Xaa16 is glycine, glutamic acid, or serine;
Xaa17 is glutamine, arginine, isoleucine, glutamic acid, cysteine, or lysine;
Xaa18 is alanine, glutamine, arginine, or histidine;
Xaa19 is alanine, glutamine, cysteine, or valine;
Xaa20 is lysine, glutamine, or arginine;
Xaa21 is cysteine, glutamic acid, glutamine, leucine, or aspartic acid;
Xaa23 is isoleucine or valine;
Xaa24 is cysteine, alanine, glutamine, asparagine, or glutamic acid;
Xaa28 is lysine, cysteine, asparagine, or aspartic acid;
Xaa29 is glycine, glutamine, cysteine, or histidine;
Xaa30 is cysteine, glycine, lysine, or histidine;
Xaa31 is proline or cysteine; and
Xaa40 is cysteine or is absent.

More specifically, in General Formula 2,
Xaa13 may be alanine, tyrosine, or cysteine;
Xaa15 may be aspartic acid or glutamic acid;
Xaa17 may be glutamine, arginine, cysteine, or lysine;
Xaa18 may be alanine, arginine, or histidine;
Xaa21 may be cysteine, glutamic acid, glutamine, or aspartic acid;
Xaa23 may be isoleucine or valine;
Xaa24 may be cysteine, glutamine, or asparagine;
Xaa28 may be cysteine, asparagine, or aspartic acid;
Xaa29 may be glutamine, cysteine, or histidine; and
Xaa30 may be cysteine, lysine, or histidine.

The remaining variables of General Formula 2 except for the variables described in the present aspect may have a combination of the amino acids described above.

Examples of the peptides having activity to glucagon, GLP-1, and GIP receptors may be peptides containing or consisting (essentially) of an amino acid sequence selected from the group consisting of SEQ ID NOS: 21, 22, 42, 43, 50, 64 to 77, and 95 to 102, and more specifically SEQ ID NOS: 21, 22, 42, 43, 50, 64 to 77, and 96 to 102, but are not particularly limited thereto.

In a specific aspect, the peptide having activity to glucagon, GLP-1, and GIP receptors may contain an amino acid sequence represented by General Formula 3 below: 
Xaa1–Xaa2–Gln–Gly–Thr–Phe–Thr–Ser–Asp–Tyr–Ser–Lys–Xaa13–Leu–
Asp–Glu–Xaa17–Xaa18–Xaa19–Lys–Xaa21–Phe–Val–Xaa24–Trp–Leu–Leu–
Xaa28–Xaa29–Xaa30–Xaa31–Ser–Ser–Gly–Gln–Pro–Pro–Pro–Ser–Xaa40
(General Formula 3, SEQ ID NO: 105),
 wherein, in General Formula 3,
Xaa1 is histidine or tyrosine;
Xaa2 is α-methyl-glutamic acid or Aib;
Xaa13 is alanine, tyrosine, or cysteine;
Xaa17 is arginine, cysteine, or lysine;
Xaa18 is alanine or arginine;
Xaa19 is alanine or cysteine;
Xaa21 is glutamic acid or aspartic acid;
Xaa24 is glutamine or asparagine;
Xaa28 is cysteine or aspartic acid;
Xaa29 is cysteine, histidine, or glutamine;
Xaa30 is cysteine or histidine;
Xaa31 is proline or cysteine; and
Xaa40 is cysteine, or is absent.

Examples of the peptides having activity to glucagon, GLP-1, and GIP receptors may be peptides containing or consisting (essentially) of an amino acid sequence selected from the group consisting of SEQ ID NOS: 21, 22, 42, 43, 50, 64 to 71, 75 to 77, and 96 to 102, but are not particularly limited thereto.

In General Formula 1, R1 may be cysteine, GKKNDWKHNIT (SEQ ID NO: 106), CSSGQPPPS (SEQ ID NO: 109), GPSSGAPPPS (SEQ ID NO: 110), GPSSGAPPPSC (SEQ ID NO: 111), PSSGAPPPS (SEQ ID NO: 112), PSSGAPPPSG (SEQ ID NO: 113), PSSGAPPPSHG (SEQ ID NO: 114), PSSGAPPPSS (SEQ ID NO: 115), PSSGQPPPS (SEQ ID NO: 116), or PSSGQPPPSC (SEQ ID NO: 117), or may be absent, but is not particularly limited thereto.

In addition, the peptide of the present invention may be synthesized by way of a method well known in the art, for example, an automatic peptide synthesizer, depending on the length thereof, and may be produced using genetic engineering technology.

Specifically, the peptide of the present invention may be prepared by way of a standard synthesis method, a recombinant expression system, or any other method known in the art. Therefore, the peptide according to the present invention may be synthesized by way of a plurality of methods including, for example, the following methods:
(a) a method of synthesizing peptides by way of a solid-phase or liquid-phase method, either stepwise or by fragment assembly, and isolating and purifying a final peptide product;
(b) a method of expressing a nucleic acid construct encoding a peptide in a host cell and recovering the expression product from the host cell culture;
(c) a method of performing *in vitro* cell-free expression of a nucleic acid construct encoding a peptide and recovering the expression product therefrom; or
a method of obtaining peptide fragments by way of any combination of the methods (a), (b), and (c), obtaining the peptide by linking the fragments, and then recovering the peptide.

As used herein, the term "insulin" refers to a kind of hormone secreted in the beta-cells of pancreas, and generally serves to control blood sugar in the body through the promotion of intracellular glucose absorption and the inhibition of fat decomposition. A proinsulin precursor, which has no blood glucose control function, is processed to become insulin, which has a blood glucose control function. Insulin is composed of two polypeptide chains, *i.e.,* the A-chain and the B-chain, which contain 21 and 30 amino acids, respectively, and are interlinked by two disulfide bridges. The insulin may be human insulin. The A-chain and B-chain of native human insulin contain the amino acid sequences set forth in SEQ ID NOS: 121 and 122 below, respectively.
A-chain: 
Gly–Ile–Val–Glu–Gln–Cys–Cys–Thr–Ser–Ile–Cys–Ser–Leu–Tyr–Gln–Leu–
Glu–Asn–Tyr–Cys–Asn (SEQ ID NO: 121)
B-chain:

Phe–Val–Asn–Gln–His–Leu–Cys–Gly–Ser–His–Leu–Val–Glu–Ala–Leu–
Tyr–Leu–Val–Cys–Gly–Glu–Arg–Gly–Phe–Phe–Tyr–Thr–Pro–Lys–Thr (SEQ ID
NO: 122)

As used herein, the term "proinsulin" refers to a precursor molecule of insulin. The proinsulin may contain the insulin A-chain and B-chain, and a C-peptide therebetween. The proinsulin may be human proinsulin.

In the present invention, the insulin may be native insulin, or an analog, derivative, or fragment thereof, which has an alteration selected from the group consisting of a substitution, an addition, a deletion, a modification, and a combination thereof of at least one amino acid in native insulin, but is not limited thereto.

As used herein, the term "insulin analog" refers to non-native insulin which is different from native insulin.

Examples of the insulin analog include an analog obtained by altering some amino acids of native insulin through addition, deletion, or substitution. For example, the insulin analog may be one in which at least one amino acid selected from the group consisting of the amino acids at positions 1, 2, 3, 5, 8, 10, 12, 16, 23, 24, 25, 26, 27, 28, 29, and 30 in the B-chain and the amino acids at positions 1, 2, 5, 8, 10, 12, 14, 16, 17, 18, 19, and 21 in the A-chain of native insulin is substituted with another amino acid or deleted, but is not limited thereto. The insulin analogs of the present invention may refer to the disclosures of Korean Patent Publication No. 10-2014-0106452 or 10-2017-0026284 (or WO 2014/133324 A1 or WO 2017/039267), the full text of each specification of which is incorporated herein by reference.

The insulin analogs to be applied in the present invention may be in the form of a single polypeptide chain or two polypeptide chains, more preferably two polypeptide chains, but are not limited thereto. The two polypeptide chains may be composed of two polypeptides—a polypeptide corresponding to the A-chain of native insulin and a polypeptide corresponding to the B-chain of native insulin. The expression corresponding to the A-chain or B-chain of native insulin may refer to having a sequence identity of at least 60%, at least 65%, at least 70%, at least 75%, at least 80%, at least 85%, at least 90%, at least 91%, at least 92%, at least 93%, at least 94%, or at least 95% when any one chain of two polypeptide chains is compared with the A-chain or B-chain of native insulin in view of sequence identity, but is not particularly limited thereto, and a person skilled in the art can easily understand by comparing a sequence constituting two polypeptide chains with the sequence of the A-chain or B-chain of native insulin.

As used herein, the term "homology" is intended to represent the degree of similarity to the amino acid sequence of a wild-type protein or the nucleotide sequence encoding the same, and the term encompasses sequences having sequence identity of at least the above-described percentage levels to the amino acid sequences or nucleotide sequences of the present invention. The homology may be determined through the comparison of two given sequences by the naked eye, or may be determined using a bioinformatic algorithm whereby the degree of homology is analyzed by arranging the subject sequences of comparison. The homology between the two given amino acid sequences may be expressed as a percentage. Useful automated algorithms are available for use in GAP, BESTFIT, FASTA, and TFASTA computer software modules of the Wisconsin Genetics Software Package (Genetics Computer Group, Madison, WI, USA). The arrangement algorithms automated in the above modules encompass sequence arrangement algorithms by Needleman & Wunsch, Pearson & Lipman, and Smith & Waterman. Other useful algorithms for sequence arrangement and the determination of homology are automated in software including FASTP, BLAST, BLAST2, PSIBLAST, and CLUSTAL W.

The information on sequences of insulin and nucleotide sequences encoding the same used in the present invention may be obtained from a known database, such as the NCBI.

In a specific exemplary embodiment, the insulin analog of the present invention contains: an A-chain of SEQ ID NO: 119 represented by General Formula 4 below; and a B-chain of SEQ ID NO: 120 represented by General Formula 5 below: [General Formula 4] 
Xaa1-Xaa2-Val-Glu-Xaa5-Cys-Cys-Thr-Ser-Ile-Cys-Xaa12-Leu-
Xaa14-Gln-Xaa16-Glu-Asn-Xaa19-Cys-Xaa21(SEQ ID NO: 119),
 wherein, in General Formula 4,
Xaa1 is alanine, glycine, glutamine, histidine, glutamic acid, or asparagine;
Xaa2 is alanine or isoleucine;
Xaa5 is alanine, glutamic acid, glutamine, histidine, or asparagine;
Xaa12 is alanine, serine, glutamine, glutamic acid, histidine, or asparagine;
Xaa14 is alanine, tyrosine, glutamic acid, histidine, lysine, aspartic acid, or asparagine;
Xaa16 is alanine, leucine, tyrosine, histidine, glutamic acid, or asparagine;
Xaa19 is alanine, tyrosine, serine, glutamic acid, histidine, threonine, or asparagine; and
Xaa21 is asparagine, glycine, histidine, or alanine, 
[General Formula 5]
Phe–Val–Asn–Gln–His–Leu–Cys–Xaa8–Ser–His–Leu–Val–Glu–Ala–Leu–
Xaa16–Leu–Val–Cys–Gly–Glu–Arg–Xaa23–Xaa24–Xaa25–Tyr–Xaa27–Xaa28–
Lys–Thr (SEQ ID NO: 120)
wherein, in General Formula 5,
Xaa8 is alanine or glycine;
Xaa16 is tyrosine, glutamic acid, serine, threonine, or aspartic acid, or is absent;
Xaa23 is glycine or alanine;
Xaa24 is alanine or phenylalanine;
Xaa25 is alanine, phenylalanine, aspartic acid, or glutamic acid, or is absent;
Xaa27 is threonine, or is absent; and
Xaa28 is proline, glutamic acid, or aspartic acid, or is absent
(wherein a peptide including the A-chain of SEQ ID NO: 121 and the B-chain of SEQ ID NO: 122 is excluded).

More specifically, the insulin analog may have a substitution of at least one amino acid selected from the group consisting of the amino acids at positions 8, 23, 24, and 25 in the B-chain and the amino acids at positions 1, 2, and 19 in the A-chain of the native insulin with alanine, and/or a substitution of the amino acid at position 14 in the A-chain of the native insulin with glutamic acid or asparagine, and in particular, the insulin analog may contain or consist of an amino acid sequence selected from the group consisting of SEQ ID NOS: 124, 126, 128, 130, 132, 134, 136, 138, and 140, but the insulin analog is not limited thereto.

Alternatively, the insulin analog may have a substitution of the amino acid at position 16 in the B-chain of the native insulin with glutamic acid, and/or a deletion of the amino acid at position 25 in the B-chain of the native insulin, and/or a substitution of the amino acid at position 14 in the A-chain of the native insulin with glutamic acid or alanine, and in particular, the insulin analog may contain or consist of an amino acid sequence of SEQ ID NO: 142 or 144, but the insulin analog is not limited thereto.

Alternatively, the insulin analog may have a substitution of the amino acid at position 16 in the B-chain of the native insulin with glutamic acid, serine, threonine, or aspartic acid, and/or a substitution of the amino acid at position 25 in the B-chain of the native insulin with aspartic acid or glutamic acid, and/or a substitution of the amino acid at position 14 in the A-chain of the native insulin with histidine, lysine, alanine, or aspartic acid, and/or a substitution of the amino acid at position 19 in the A-chain of the native insulin with glutamic acid, serine, or threonine, and in particular, the insulin analog may contain or consist of an amino acid sequence selected from the group consisting of SEQ ID NOS: 146, 148, 150, 152, 154, 156, 158, 160, 162, 164, 166, 168, and 170, but the insulin analog is not limited thereto.

Meanwhile, the insulin analog of the present invention may be a substance which is in the form of a short-chain insulin without removal of the C-peptide, contains an A-chain of SEQ ID NO: 119 represented by General Formula 4 and a B-chain of SEQ ID NO: 120 represented by General Formula 5, and has activity and functions of insulin.

In addition, the insulin analog of the present invention may be prepared by removing the C-peptide from the proinsulin containing the C-peptide between the A-chain and the B-chain. The removal of the C-peptide may be conducted through a method known in the art, and specifically, the insulin analog may be prepared by treatment with trypsin and carboxypeptidase B, but is not limited thereto.

Specifically, the insulin analog of the present invention may be composed of an A-chain of SEQ ID NO: 119 represented by General Formula 4 and a B-chain of SEQ ID NO: 120 represented by General Formula 5, and more specifically, the insulin analog may be in the form of two polypeptide chains in which the A-chain and the B-chain are linked to each other via two disulfide bridges, but is not limited thereto.

It is obvious that an insulin analog which is set forth in particular sequence numbers and is prepared in the form of two polypeptide chains by removing the C-peptide from the proinsulin-form insulin analog is also included in the scope of the present invention.

Specifically, the insulin analogs may have the following alterations in the sequence of native insulin, particularly native human insulin, and may be selected from Analogs 1 to 24 below.

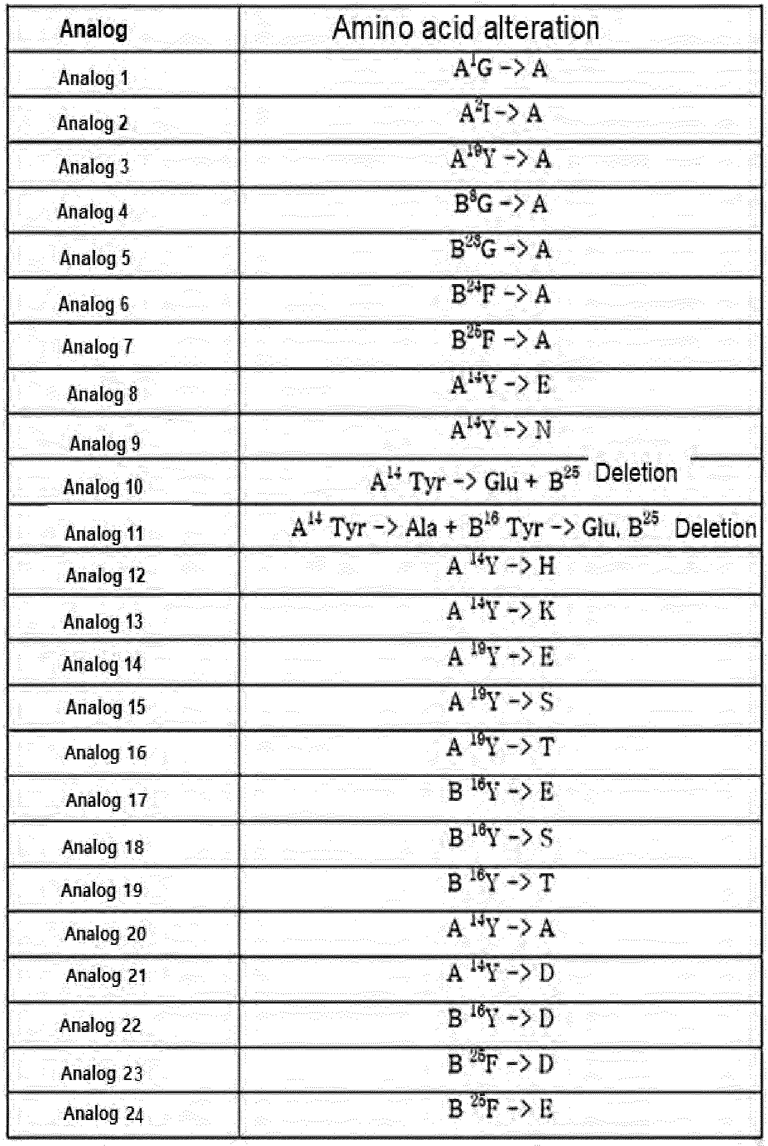

As used herein, the term "derivative of insulin" encompasses a peptide which has at least one difference in the amino acid sequence compared with native insulin, a peptide which is prepared by altering the sequence of native insulin through modification, and a mimetic of native insulin which can regulate the *in vivo* blood glucose control function like native insulin. Such a derivative of native insulin may have an *in vivo* blood glucose control function.

Specifically, the derivative of insulin may be obtained by alteration of any one method of substitution, addition, deletion, and modification of some amino acids in native insulin or a combination of these methods.

Specifically, the derivative of native insulin may show an amino acid sequence homology of at least 80% to each of the A-chain and the B-chain of native insulin and/or may be in such a form that a group of one amino acid residue of insulin is subjected to chemical substitution (*e*.*g*., alpha-methylation or alpha-hydroxylation), deletion *(e.g.,* deamination), or modification *(e.g.,* N-methylation), but is not limited thereto.

The derivatives of native insulin to be applied in the present invention may be prepared by a combination of various methods used for preparing derivatives.

Such an alteration for the preparation of derivatives of insulin includes: an alteration using an L-type or D-type amino acid and/or a non-native amino acid; and/or an alteration by modification or post-translational modification (*e*.*g*., methylation, acylation, ubiquitination, intermolecular covalent bond, *etc*.) of the sequence of the native form.

In addition, such an alteration also includes all of the additions of one or more amino acids at the amino and/or carboxy terminus of insulin.

The amino acids to be substituted or added may be not only the 20 amino acids commonly found in human proteins, but also atypical amino acids or those which do not occur naturally. Commercial sources of atypical amino acids may include Sigma-Aldrich, ChemPep, and Genzyme Pharmaceuticals. The sequences of peptides containing these amino acids and typical peptides can be synthesized by and purchased from commercial suppliers, for example, American Peptide Company and Bachem in the USA, or Antigen in Korea, but are not particularly limited.

As used herein, the term "fragment of native insulin, an insulin analog, or a derivative of insulin" refers to a form in which one or more amino acids are removed from the amino terminus or carboxy terminus of native insulin, an insulin analog, or a derivative of native insulin. These fragments may retain a blood glucose control function in the body.

In addition, the insulin analog of the present invention may be prepared by using respective methods used in the preparation of derivatives and fragments of native insulin independently or in combination.

Specifically, the insulin analog according to the present invention includes an alteration of a particular amino acid residue in the A-chain and B-chain of the native insulin described above, and specifically, the insulin analog may have an alteration of a particular amino acid residue in the A-chain of native insulin and/or an alteration of a particular amino acid residue in the B-chain of native insulin.

The pharmaceutical composition according to the present invention may contain, as insulin, which is one of the active ingredients, (a) native insulin, (b) an insulin analog, (c) a derivative of insulin, (d) a fragment thereof, or (e) a combination thereof.

In one specific embodiment, the insulin or the isolated peptide having activity to glucagon, GLP-1, and GIP receptors of the present invention may be in the form of a long-acting conjugate in which a biocompatible substance capable of increasing the *in vivo* half-life of the insulin or isolated peptide is conjugated to the insulin or isolated peptide.

As used herein, the term "long-acting conjugate" or "conjugate" of insulin or an isolated peptide having activity to glucagon, GLP-1, and GIP receptors has a structure in which a biocompatible substance is conjugated to the insulin or the isolated peptide having activity to glucagon, GLP-1, and GIP receptors, and can exhibit increased duration of efficacy compared with insulin or an isolated peptide having activity to glucagon, GLP-1, and GIP receptors to which the biocompatible substance is not conjugated.

The biocompatible substance in the long-acting conjugate may be linked by a covalent linkage to the insulin or the isolated peptide having activity to glucagon, GLP-1, and GIP receptors, but is not particularly limited thereto.

In the present invention, insulin, which is one element of the conjugate, may be insulin having the sequence of the native form, or may be an analog, derivative, or fragment of insulin which has an alteration of at least one amino acid in the sequence of the native form by a substitution, an addition, a deletion, a modification, or a combination thereof, but any form of insulin may be used as one element of the conjugate of the present invention without limitation as long as it has blood glucose lowering /increasing effects of native insulin.

As used herein, the term "biocompatible substance" refers to a substance which can be linked to a physiologically active substance (*e*.*g*., an isolated peptide having activity to glucagon, GLP-2, and GIP receptors, insulin, *etc*.) to thereby increase the duration of efficacy of the physiologically active substance compared with a physiologically active substance to which a biocompatible substance moiety or carrier is not conjugated. The biocompatible substance may be covalently linked to a physiologically active substance, but is not particularly limited thereto.

Specifically, the conjugate is represented by Chemical Formula 1:

[Chemical Formula 1] X―L_{a―}F,

wherein,
X is the insulin, or the isolated peptide having activity to glucagon, GLP-1, and GIP receptors (*i.e.,* a triple agonist);
L is a linker;
a is 0 or a natural number, with the proviso that when a is 2 or greater, each L is independent from each other; and
F is a substance capable of increasing the half-life of X.

The composition of the present invention may contain (a) insulin and a triple agonist, (b) a long-acting conjugate of insulin and a triple agonist, (c) insulin and a long-acting conjugate of a triple agonist, or (d) a long-acting conjugate of insulin and a long-acting conjugate of a triple agonist, wherein the long-acting conjugate form of the insulin or triple agonist exhibits excellent blood glucose control effects on the basis of increased duration in the body, and can relieve side effects of insulin.

In the conjugate, F is X, that is, a substance capable of increasing the half-life of insulin or a triple agonist, and corresponds to one element of a moiety constituting the conjugate of the present invention.

F and X may be linked to each other by way of a covalent chemical linkage or a non-covalent chemical linkage, or F and X may be linked to each other via L by way of a covalent chemical linkage, a non-covalent chemical linkage, or a combination thereof.

The substance capable of increasing the half-life of X may be a biocompatible substance and, for example, may be selected from the group consisting of polymers, fatty acids, cholesterol, albumin and fragments thereof, albumin-binding substances, polymers of repeating units of particular amino acid sequences, antibodies, antibody fragments, FcRn-binding substances, *in vivo* connective tissues, nucleotides, fibronectin, transferrin, saccharides, heparin, and elastin, but is not limited thereto.

The elastin may be human tropoelastin, which is a water-soluble precursor, and may be a moiety of the sequence thereof or a polymer of some repeating units thereof, and examples thereof include all elastin-like polypeptides, but are not particularly limited thereto.

An example of the high-molecular polymer is one which is selected from the group consisting of polyethylene glycol, polypropylene glycol, ethylene glycol― propylene glycol copolymers, polyoxyethylated polyols, polyvinyl alcohols, polysaccharides, dextran, polyvinyl ethyl ether, biodegradable polymers, lipid polymers, chitin, hyaluronic acid, oligonucleotides, and combinations thereof, but is not particularly limited thereto.

The polyethylene glycol corresponds to a term encompassing all of the forms of homopolymers of ethylene glycol, PEG copolymers, and monomethyl-substituted PEG polymers (mPEG), but is not particularly limited thereto.

Examples of the biocompatible substance may include poly-amino acids, such as poly-lysine, poly-aspartic acid, and poly-glutamic acid, but are not limited thereto.

The fatty acid may have binding affinity to albumin *in vivo,* but is not particularly limited thereto.

In a more specific embodiment, the FcRn-binding substance may be an immunoglobulin Fc region, and more specifically an IgG Fc region, but is not particularly limited thereto.

One or more amino acid side chains within the peptide of the present invention may be conjugated to these biocompatible substances in order to increase solubility and/or half-life *in vivo,* and/or increase bioavailability thereof. Such an alteration may also reduce the clearance of therapeutic proteins and peptides.

The biocompatible substances described above may be water-soluble (amphipathic or hydrophilic) and/or non-toxic and/or pharmaceutically acceptable.

F and X may be directly linked to each other (*i.e.,* a is 0 in Chemical Formula 1) or may be linked via a linker (L).

In the present invention, "immunoglobulin Fc region" refers to a region including heavy chain constant region 2 (CH2) and/or heavy chain constant region 3 (CH3), excluding the heavy chain and light chain variable regions of an immunoglobulin. The immunoglobulin Fc region may be one element that constitutes a moiety of the conjugate of the present invention.

This immunoglobulin Fc region may include a hinge region in the heavy chain constant region, but is not limited thereto. The immunoglobulin Fc region of the present invention may be an extended Fc region including a part or the entirety of heavy chain constant region 1 (CH1) and/or light chain constant region 1 (CT1), excluding the heavy chain and light chain variable regions of the immunoglobulin, as long as the immunoglobulin Fc region has a substantially equivalent or improved effect compared with the native form. Alternatively, the immunoglobulin Fc region of the present invention may be a region having a deletion of a relatively long amino acid sequence corresponding to CH2 and/or CH3.

For example, the immunoglobulin Fc region of the present invention may be 1) a CH1 domain, a CH2 domain, a CH3 domain, and a CH4 domain; 2) a CH1 domain and a CH2 domain; 3) a CH1 domain and a CH3 domain; 4) a CH2 domain and a CH3 domain; 5) a combination of one or more domains among a CH1 domain, a CH2 domain, a CH3 domain, and a CH4 domain and an immunoglobulin hinge region (or a moiety of the hinge region); and 6) a dimer between each domain of the heavy chain constant regions and the light chain constant region. However, the immunoglobulin Fc region of the present invention is not limited thereto.

In a specific embodiment, the immunoglobulin Fc region may be in a dimeric form, and one X molecule may be covalently linked to one Fc region in a dimeric form, wherein the immunoglobulin Fc and X may be linked to each other by a non-peptide polymer. On the other hand, two X molecules can also be symmetrically linked to one Fc region in a dimeric form. The immunoglobulin Fc and X may be linked to each other by way of a non-peptide linker. However, the immunoglobulin Fc region of the present invention is not limited thereto.

In addition, the immunoglobulin Fc region of the present invention contains not only the amino acid sequence of the native form but also a derivative thereof. The amino acid sequence derivative refers to an amino acid sequence which is different from the amino acid sequence of the native form by a deletion, an insertion, a non-conservative or conservative substitution, or a combination thereof of at least one amino acid residue.

For example, for immunoglobulin Fc, the amino acid residues at positions 214 to 238, 297 to 299, 318 to 322, or 327 to 331, which are known to have an important role in conjugating, may be used as appropriate sites for alteration.

A variety of derivatives are possible, for example, by removing a site capable of forming a disulfide linkage, deleting some amino acid residues at the N-terminus of native Fc, or adding a methionine residue at the N-terminus of native Fc. In addition, in order to eliminate effector functions, a complement-binding site, for example, a C1q-binding site, may be removed, and an antibody-dependent cell-mediated cytotoxicity (ADCC) site may be removed. Techniques for preparing such sequence derivatives of the immunoglobulin Fc region are disclosed in International Patent Publication Nos. WO 97/34631 and WO 96/32478, and the like.

Amino acid exchanges in proteins and peptides, which do not entirely change the activity of the proteins or peptides, are known in the art (H. Neurath, R. L. Hill, The Proteins, Academic Press, New York, 1979). The most commonly occurring exchanges are exchanges between amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In some cases, the change may be conducted by phosphorylation, sulfation, acrylation, glycosylation, methylation, farnesylation, acetylation, aminations, or the like.

The above-described Fc derivatives exhibit biological activity equivalent to that of the Fc region of the present invention, and may improve the structural stability of the Fc region against heat, pH, and the like.

Furthermore, this Fc region may be obtained from a native form isolated from the living bodies of humans or animals, such as cows, goats, pigs, mice, rabbits, hamsters, rats, and guinea pigs, or may be a recombinant form obtained from transformed animal cells or microorganisms, or derivatives thereof. In such a case, the Fc region may be obtained from the native form by isolating the whole immunoglobulin from the living body of a human or animal and then treating the isolated immunoglobulin with protease. The isolated immunoglobulin is cleaved into Fab and Fc by treatment with papain, and cleaved into pF'c and F(ab)₂ by treatment with pepsin. These may be subjected to size-exclusion chromatography or the like to separate Fc or pF'c. In a more specific embodiment, the immunoglobulin Fc region is a recombinant immunoglobulin Fc region in which a human-derived Fc region is obtained from microorganisms.

In addition, the immunoglobulin Fc region may be in the form of natural glycans, increased glycans compared with the native form, or decreased glycans compared with the native form, or in a deglycosylated form. The increase, decrease, or removal of the immunoglobulin Fc glycans may be attained by using conventional methods, such as a chemical method, an enzymatic method, and a genetic engineering method using a microorganism. The immunoglobulin Fc region with glycans removed from Fc exhibits a significant deterioration in binding affinity to complement C1q and a reduction or elimination of antibody-dependent cytotoxicity or complement-dependent cytotoxicity, and thus causes no unnecessary immune responses *in vivo.* In this regard, a deglycosylated or aglycosylated immunoglobulin Fc region may be a more suitable form to fulfill the original purpose of the present invention as a drug carrier.

As used herein, the term "deglycosylation" refers to enzymatic removal of glycans from an Fc region, and the term "aglycosylation" means an unglycosylated Fc region produced in prokaryotes, more specifically E. *coli.*

Meanwhile, the immunoglobulin Fc region may originate from humans, or other animals including cows, goats, pigs, mice, rabbits, hamsters, rats, and guinea pigs, and in a more specific embodiment, the immunoglobulin Fc region originates from humans.

In addition, the immunoglobulin Fc region may be an Fc region derived from IgG, IgA, IgD, IgE, IgM, or a combination or hybrid thereof. In a still more specific embodiment, the immunoglobulin Fc region is derived from IgG or IgM, which are most abundant in human blood, and in a still more specific embodiment, the immunoglobulin Fc region is derived from IgG, which is known to increase the half-lives of ligand-binding proteins. In a still more specific embodiment, the immunoglobulin Fc region is an IgG4 Fc region, and in the most specific embodiment, the immunoglobulin Fc region is an aglycosylated Fc region derived from human IgG4, but is not limited thereto.

As used herein, the term "combination" means that when a dimer or multimer is formed, a polypeptide encoding a single-chain immunoglobulin Fc region of the same origin is linked to a single-chain polypeptide of a different origin. That is, a dimer or multimer may be prepared from two or more fragments selected from the group consisting of IgG Fc, IgA Fc, IgM Fc, IgD Fc, and IgE Fc fragments.

In addition, the above-described conjugate may have increased duration of efficacy compared with native insulin or a triple agonist, or compared with X not modified with F, and such a conjugate encompasses not only the above-described forms, but also a form of being encapsulated in a biodegradable nanoparticle.

In addition, L may be a peptide linker or a non-peptide linker.

When L is a peptide linker, L may contain one or more amino acids, for example, 1 to 1000 amino acids, but is not particularly limited thereto. In the present invention, various known peptide linkers may be used to link F and X, and examples thereof may include a [GS]ₓ linker, a [GGGS]ₓ linker, a [GGGGS]ₓ linker, and the like, wherein x may be a natural number of at least 1. However, the peptide linkers are not limited to the above examples.

In the present invention, the "non-peptide linker" includes a biocompatible polymer having two or more repeating units linked to each other. The repeating units are linked to each other by way of any covalent linkage but not a peptide linkage. The non-peptide linker may be one element constituting a moiety of the conjugate of the present invention, and corresponds to L in Chemical Formula 1. In the present invention, the non-peptide linker may be used interchangeably with a non-peptide polymer.

In the present invention, the peptide linker contains a reactive group at an end thereof, and thus may form a conjugate by way of a reaction with another element constituting the conjugate. When a non-peptide linker having reactive functional groups at both ends binds to X and F in Chemical Formula 1 through the respective reactive groups to form a conjugate, the non-peptide linker or non-peptide polymer may be named a non-peptide polymer linker moiety or a non-peptide linker moiety.

In Lₐ, a may be 1 or greater, and each L may be independent when a is 2 or greater.

In one specific embodiment, in the conjugate, F and X are covalently linked to each other via a non-peptide linker including reactive groups, at both ends thereof, capable of being linked to F, specifically an immunoglobulin Fc region, and X, specifically a peptide drug.

Specifically, the non-peptide linker may be selected from the group consisting of fatty acids, saccharides, high-molecular-weight polymers, low-molecular-weight compounds, nucleotides, and combinations thereof.

Although not particularly limited, the high-molecular-weight polymer in the present invention may be in the range of more than 0 kDa to about 100 kDa, specifically in the range of about 1 kDa to about 100 kDa, and more specifically in the range of about 1 kDa to about 20 kDa, but is not particularly limited thereto.

As used herein, the term "about" refers to a range including ±0.5, ±0.4, ±0.3, ±0.2, ±0.1, and the like, and thus includes all of the values in the range equivalent or similar to those stated after this term, but is not limited thereto.

Although not particularly limited, the high-molecular-weight polymer may be selected from the group consisting of polyethylene glycol, polypropylene glycol, ethylene glycol-propylene glycol copolymers, polyoxyethylated polyols, polyvinyl alcohols, polysaccharides, dextran, polyvinyl ethyl ether, biodegradable polymers, lipid polymers, chitin, hyaluronic acid, oligonucleotides, and combinations thereof, but is not particularly limited thereto. In a more specific embodiment, L may be polyethylene glycol, but is not limited thereto. In addition, derivatives thereof which are already known in the art and derivatives which can be easily prepared within the skill in the art are also included in the scope of the present invention.

As the non-peptide linker usable in the present invention, any polymer that has resistance to *in vivo* protease can be used without limitation. The molecular weight of the non-peptide polymer is in the range of about 1 kDa to about 100 kDa, and specifically in the range of about 1 kDa to about 20 kDa, but is not limited thereto. In addition, as the non-peptide linker of the present invention, which is to be linked to a polypeptide corresponding to F, one type of polymer as well as a combination of different types of polymers may be used.

In one specific embodiment, both ends of the non-peptide linker may be linked to F, for example, an amine group or thiol group of the immunoglobulin Fc region, and an amine group or thiol group of X, respectively.

Specifically, the non-peptide polymer may contain, at both ends thereof, reactive groups capable of being linked to F (*e.g.,* an immunoglobulin Fc region) and X, respectively, and specifically reactive groups capable of being linked to an amine group located at the N-terminus or lysine, or a thiol group of cysteine, in X or F (*e.g.,* an immunoglobulin Fc region), but is not limited thereto.

Alternatively, the reactive groups of the non-peptide polymer capable of being linked to F, for example, an immunoglobulin Fc region, and X may be selected from the group consisting of an aldehyde group, a maleimide group, and a succinimide derivative, but are not limited thereto.

Examples of the aldehyde group may include a propionaldehyde group or a butyraldehyde group, but are not limited thereto.

Examples of the succinimide derivative may include succinimidyl valerate, succinimidyl methylbutanoate, succinimidyl methylpropionate, succinimidyl butanoate, succinimidyl propionate, *N*-hydroxysuccinimide, hydroxy succinimidyl, succinimidyl carboxymethyl, or succinimidyl carbonate, but are not limited thereto.

The non-peptide linker may be linked to X and F via these reactive groups, but is not particularly limited thereto.

In addition, a final product produced through reductive alkylation by way of an aldehyde linkage is significantly more stable than the linking by way of an amide linkage. The aldehyde reactive group selectively reacts with the N-terminus at a low pH, and may form a covalent linkage with a lysine residue at a high pH, for example, pH 9.0.

The reactive groups at both ends of the non-peptide linker may be the same as or different from each other, and for example, the non-peptide linker may have a maleimide group at one end; and an aldehyde group, a propionaldehyde group, or a butyraldehyde group at the other end. However, the reactive groups are not particularly limited thereto as long as F, specifically an immunoglobulin Fc region, and X can be linked to both ends of the non-peptide linker.

For example, the non-peptide linker may have a maleimide group as a reactive group at one end, and an aldehyde group, a propionaldehyde group, a butyraldehyde group, or the like at the other end.

The long-acting protein conjugate of the present invention can be prepared by activating hydroxy groups to various reactive groups through known chemical reactions when polyethylene glycol having the hydroxy reactive groups at both ends is used as a non-peptide polymer, or by using commercially available polyethylene glycol having modified reactive groups.

In one specific embodiment, the non-peptide polymer may be linked to a cysteine residue of the triple agonist, and more specifically the -SH group of cysteine, but is not limited thereto.

For example, the non-peptide polymer may be linked to the cysteine residue at position 10, the cysteine residue at position 13, the cysteine residue at position 15, the cysteine residue at position 17, the cysteine residue at position 19, the cysteine residue at position 21, the cysteine residue at position 24, the cysteine residue at position 28, the cysteine residue at position 29, the cysteine residue at position 30, the cysteine residue at position 31, the cysteine residue at position 40, or the cysteine residue at position 41 in the triple agonist, but is not particularly limited.

Specifically, a reactive group of the non-peptide polymer may be linked to the -SH group of the cysteine residue, and all of the above descriptions are applied to the reactive group. When maleimide-PEG-aldehyde is used, the maleimide group may be linked to the -SH group of X by way of a thioether linkage, and the aldehyde group may be linked to F, specifically the ―NH₂ group of immunoglobulin Fc through reductive amidation, but is not limited thereto, and this case corresponds to one example.

In one specific embodiment, the non-peptide polymer may be linked to an amine group of insulin or an analog thereof, more specifically an amine group located at the N-terminus or an amine group located at the side chain of lysine, but is not limited thereto, and this case corresponds to one example.

In the conjugate, the reactive group of the non-peptide polymer may be linked to a ―NH₂ group located at the N-terminus of the immunoglobulin Fc region, but this case corresponds to one example.

As used herein, the term "prevention" refers to all actions that inhibit or delay the outbreak of an insulin-associated disease by administration of the composition, and the term "treatment" refers to all actions that alleviate or beneficially change the symptoms of an insulin-associated disease due to administration of the composition.

As used herein, the term "administration" refers to the introduction of a predetermined substance into a patient by way of any appropriate method, and the administration route of the composition may be, but is not limited to, any general route through which the composition can arrive at the target *in vivo,* and examples of the administration route may include intraperitoneal administration, intravenous administration, intramuscular administration, subcutaneous administration, intradermal administration, oral administration, topical administration, intranasal administration, intrapulmonary administration, intrarectal administration, and the like.

The pharmaceutical composition according to the present invention may contain a pharmaceutically acceptable carrier.

As used herein, the term "pharmaceutical acceptable" refers to the properties of having a sufficient amount to exhibit a therapeutic effect and causing no side effects, and the amount may be easily determined by a person skilled in the art according to factors that are well known in the medical field, including the type of disease, patient's age, body weight, health condition, and sex, the sensitivity of a patient to drugs, the route of administration, the manner of administration, the number of times of administration, the duration of treatment, drugs used in combination or at the same time, and the like.

As for the pharmaceutically acceptable carriers, a binder, a lubricant, a disintegrant, an excipient, a solubilizer, a dispersant, a stabilizer, a suspending agent, a colorant, a flavor, and the like may be used for oral administration; a buffer, a preservative, an analgesic, a solubilizer, an isotonic agent, a stabilizer, and the like may be used in a mixture for injections; and a substrate, an excipient, a lubricant, a preservative, and the like may be used for topical administration. The formulations of the composition according to the present invention may be variously prepared by mixing with the pharmaceutically acceptable carriers described above. For example, for oral administration, the composition may be prepared in the form of a tablet, a troche, a capsule, an elixir, a suspension, a syrup, a wafer, or the like; and for injections, the composition may be prepared in the form of a unit-dose ampoule or a multi-dose container. Besides, the composition may also be formulated in the form of a solution, a suspension, a tablet, a pill, a capsule, a sustained-release preparation, or the like.

Meanwhile, examples of carriers, excipients, and diluents suitable for preparation may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, and the like. In addition, the composition may further contain a filler, an anti-coagulant, a lubricant, a humectant, a flavor, a preservative, and the like.

The pharmaceutical composition of the present invention may have any one formulation selected from the group consisting of a tablet, a pill, a powder, granules, a capsule, a suspension, a liquid medicine for internal use, an emulsion, a syrup, a sterile aqueous solution, a non-aqueous solvent, a lyophilized preparation, and a suppository.

The conjugate may be used by mixing with various carriers approved as medicinal materials, such as physiological saline or organic solvents, and for increasing stability or absorbency, a carbohydrate, such as glucose, sucrose, or dextran; an antioxidant, such as ascorbic acid or glutathione; a chelating agent; a low-molecular-weight protein; other stabilizers; and the like may be used as a medicinal material.

The dose and frequency of the pharmaceutical composition of the present invention are determined according to the type of drug as an active ingredient, together with various factors, such as a disease to be treated, a route of administration, patient's age, sex, and body weight, and severity of a disease.

A total effective amount of the composition of the present invention may be administered to a patient in a single dose, or may be administered in multiple doses for a long period of time by way of a fractionated treatment protocol. The pharmaceutical composition of the present invention may contain an active ingredient, the content of which may vary depending on the severity of a disease. Specifically, the total dose of the conjugate of the present invention may be about 0.0001 mg to 500 mg per 1 kg of body weight of a patient per day. However, the effective dose of the conjugate for a patient is determined considering various factors, such as patient's age, body weight, health condition, and sex, the severity of a disease, a diet, and an excretion rate, in addition to the route of administration and the frequency of treatment of the pharmaceutical composition, and therefore, considering these, a person skilled in the art can easily determine effective doses suitable for particular uses of the pharmaceutical composition of the present invention. The pharmaceutical composition according to the present invention is not particularly limited to the formulation, the route of administration, and the manner of administration as long as the pharmaceutical composition exhibits the effects of the present invention.

In addition, the pharmaceutical composition of the present invention may contain the combination, that is, a triple agonist or a long-acting conjugate thereof; and insulin or a long-acting conjugate thereof (or each component of the combination) at 0.01% to 99% weight per volume.

In an aspect of the present invention provides a complex preparation for weight loss in an insulin-administered patient, the complex preparation containing: (i) insulin; and (ii) an isolated peptide having activity to glucagon, GLP-1, and GIP receptors.

All of the above descriptions are applied to the insulin, the isolated peptide having activity to glucagon, GLP-1, and GIP receptors, and the ingredients contained in the composition.

Specifically, the complex preparation may contain:
(a) insulin, and a triple agonist;
(b) a long-acting conjugate of insulin in which the insulin is conjugated to a biocompatible substance capable of increasing the *in vivo* half-life of the insulin, and a triple agonist;
(c) insulin, and a long-acting conjugate of a triple agonist in which the triple agonist is conjugated to a biocompatible substance capable of increasing the *in vivo* half-life of the triple agonist; or
(d) a long-acting conjugate of insulin, and a long-acting conjugate of a triple agonist.

Specifically, the complex preparation may contain: insulin or a long-acting conjugate thereof; and a triple agonist or a long-acting conjugate thereof, wherein the insulin and the triple agonist may be contained at a molar ratio of 1:1 to 100:1; the insulin and the triple agonist may be contained at a molar ratio of 1:1 to 1:100; the insulin or the long-acting conjugate thereof and the triple agonist or the long-acting conjugate thereof may be contained at a molar ratio of 1:1 to 100:1; or the insulin or the long-acting conjugate thereof and the triple agonist or the long-acting agonist thereof may be contained at a molar ratio of 1:1 to 1:100, but the complex preparation is not limited thereto.

An aspect of the present invention provides a kit including: (i) insulin; and (ii) an isolated peptide having activity to glucagon, GLP-1, and GIP receptors.

The insulin, and the isolated peptide having activity to glucagon, GLP-1, and GIP receptors are as described above.

The kit may include instructions for administration of: (i) insulin; and (ii) an isolated peptide having activity to glucagon, GLP-1, and GIP receptors.

Specifically, the kit may include:
(a) insulin, and a triple agonist;
(b) a long-acting conjugate of insulin in which the insulin is conjugated to a biocompatible substance capable of increasing the *in vivo* half-life of the insulin, and a triple agonist;
(c) insulin, and a long-acting conjugate of a triple agonist in which the triple agonist is conjugated to a biocompatible substance capable of increasing the *in vivo* half-life of the triple agonist; or
(d) a long-acting conjugate of insulin, and a long-acting conjugate of a triple agonist.

Specifically, the kit may include: insulin or a long-acting conjugate thereof; and a triple agonist or a long-acting conjugate thereof, wherein the kit may include an instruction for administering to a subject the insulin and the triple agonist at a molar ratio of 1:1 to 100:1 or 1:1 to 1:100, for administering to a subject the insulin or the long-acting conjugate thereof and the triple agonist or the long-acting conjugate thereof at a molar ratio of 1:1 to 100:1, or for administering to a subject the insulin or the long-acting conjugate thereof and the triple agonist or the long-acting agonist thereof at a molar ratio of 1:1 to 1:100, but the kit is not limited thereto.

An aspect of the present invention provides uses of a composition or complex preparation containing: (i) insulin; and (ii) an isolated peptide having activity to glucagon, GLP-1, and GIP receptors, in the preparation of medicinal materials.

The composition or complex preparation are as described above. The medicinal materials can all be used for the above-described uses.

An aspect of the present invention provides a method for prevention or treatment of an insulin-associated disease, the method including administering to a subject administering (i) insulin and (ii) an isolated peptide having activity to glucagon, GLP-1, and GIP receptors.

An aspect of the present invention provides a method for reducing weight gain caused by insulin administration, the method including administering to a subject an isolated peptide having activity to glucagon, GLP-1, and GIP receptors.

Specifically, an aspect of the present invention provides a method for reducing weight gain caused by insulin administration, the method including administering to a subject (i) insulin and (ii) an isolated peptide having activity to glucagon, GLP-1, and GIP receptors.

The subject may be at risk of or develop an insulin-associated disease. The subject may also be a subject in need of insulin administration, or a subject in need of insulin administration as well as weight loss. However, the subject is not particularly limited thereto.

The administering step may be performed by combined administration of (a) insulin, and a triple agonist; (b) a long-acting conjugate of insulin, and a triple agonist; (c) insulin, and a long-acting conjugate of a triple agonist; or (d) a long-acting conjugate of insulin, and a long-acting conjugate of a triple agonist.

Herein, the use of the term "combined" is to be understood as referring to simultaneous, separate, or sequential administration. When the administration is sequential or separate, the delay in administering a second ingredient should not be such that the beneficial effect of the combination is lost.

The substances may be administered simultaneously, separately, sequentially, or in reverse order, and may be simultaneously administered in an appropriate combination of effective amounts, but such administration is not limited to a particular method or order of administration.

Furthermore, the method for prevention or treatment of the present invention may include administering to a subject a composition or complex preparation containing (a) to (d) above, but is not limited thereto.

The composition or complex preparation of the present invention containing insulin or a long-acting conjugate thereof and a triple agonist or a long-acting conjugate thereof supplements the activity or function of insulin to thereby suppress side effects of insulin, such as weight gain, while remarkably lowering blood sugar, and thus exhibits excellent effects on the treatment of insulin-associated diseases.

In addition, the composition or complex preparation may be formulated in the form of a unit dose of preparation suitable for the administration into the patient's body, specifically a preparation useful for the administration of protein medicines, according to a typical method in the pharmaceutical field, and may be administered by way of an administration method that is typically used in the art through an oral administration route, or a parenteral administration route including skin, intravenous, intramuscular, intraarterial, intramedullary, intrathecal, intraventricular, pulmonary, transdermal, subcutaneous, intraperitoneal, intranasal, intragastrical, topical, sublingual, vaginal, or rectal routes, but is not limited thereto.

The method of the present invention may include administering a pharmaceutically effective amount of the pharmaceutical composition containing the active ingredients. The appropriate total daily dose of the pharmaceutical composition may be determined by a practitioner within the scope of correct medical judgment, and the pharmaceutical composition may be administered once or several times in divided doses. However, for the purpose of the present invention, the specific therapeutically effective amount for a certain patient is preferably differently applied according to: the type and degree of response to be achieved; in some cases, the use or non-use of another preparation; various factors including a specific composition, patient's age, body weight, general physical condition, sex, and a diet, the time of administration, the route of administration, the secretion rate of a composition, the duration of treatment, and a drug used in combination or simultaneously with a specific composition; and similar factors well known in the field of medicine.

Specifically, for insulin or a long-acting conjugate thereof and a triple agonist or a long-acting conjugate thereof, the insulin and the triple agonist may be administered to a subject at a molar ratio of 1:1 to 100:1 or a molar ratio of 1:1 to 1:100; or the insulin or the long-acting conjugate thereof and the triple agonist or the long-acting conjugate thereof may be administered to a subject at a molar ratio of 1:1 to 100:1; or the insulin or the long-acting conjugate thereof and the triple agonist or the long-acting agonist thereof may be administered to a subject at a molar ratio of 1:1 to 1:100, but this is not limited thereto.

Hereinafter, the present invention will be described in detail with reference to the following exemplary embodiments. However, the following exemplary embodiments are merely for illustrating the present invention and are not intended to limit the scope of the present invention.

### Example 1: Preparation of triple agonists and long-acting conjugates thereof

### (1) Preparation of triple agonists

Triple agonists exhibiting activity to all of glucagon, GLP-1, and GIP receptors were prepared, and the sequences thereof are shown in Table 1 below.

**TABLE 1**

| **SEQ ID NO** | **Sequence** | **Information** |
|---|---|---|
| 1 | HXQGTFTSDVSSYLDGQAAKEFIAWLVKGC | |
| 2 | HXQGTFTSDVSSYLDGQAQKEFIAWLVKGC | |
| 3 | HXQGTFTSDVSSYLLGQAAKQFIAWLVKGGGP SSGAPPPSC | |
| 4 | HXQGTFTSDVSSYLLGQQQKEFIAWLVKGC | |
| 5 | H X Q G T F T S D V S S Y L L G Q Q Q K E F I A W L V K G G G P S S G A P P P S C | |
| 6 | HXQGTFTSDVSSYLDGQAAKEFVAWLLKGC | |
| 7 | HXQGTFTSDVSKYLDGQAAKEFVAWLLKGC | |
| 8 | HXQGTFTSDVSKYLDGQAAQEFVAWLLKGC | |
| 9 | HXQGTFTSDVSKYLDGQAAQEFVAWLLAGC | |
| 10 | H X Q G T F T S D V S K Y L D G Q A A Q E F V A W L L A G G G P S S G A P P P S C | |
| 11 | CAGEGTFTSDLSKYLDSRRQQLFVQWLKAGGP SSGAPPPSHG | |
| 12 | CAGEGTFISDLSKYMDEQAVQLFVEWLMAGGP SSGAPPPSHG | |
| 13 | CAGEGTFISDYSIQLDEIAVQDFVEWLLAQKPS SGAPPPSHG | |
| 14 | CAGQGTFTSDYSIQLDEIAVRDFVEWLKNGGPS SGAPPPSHG | |
| 15 | CAGQGTFTSDLSKQMDEEAVRLFIEWLKNGGP SSGAPPPSHG | |
| 16 | CA G Q G T F T S D L S K Q M D S E A Q Q L F I E W L K N G G P S S G A P P P S H G | |
| 17 | CAGQGTFTSDLSKQMDEERAREFIEWLLAQKP SSGAPPPSHG | |
| 18 | CAGQGTFTSDLSKQMDSERAREFIEWLKNTGP SSGAPPPSHG | |
| 19 | CA G Q G T F T S D L S I Q Y D S E H Q R D F I E W L K D T G P S S G A P P P S H G | |
| 20 | CAGQGTFTSDLSIQYEEEAQQDFVEWLKDTGP SSGAPPPSHG | |
| 21 | Y X Q G T F T S D Y S K Y L D E C R A K E F V Q W L L D H H P S S G Q P P P S | Ring formation |
| 22 | Y X Q G T F T S D Y S K C L D E K R A K E F V Q W L L D H H P S S G Q P P P S | Ring formation |
| 23 | Y X Q G T F T S D Y S K Y L D E C R A K E F V Q W L L A Q K G K K N D W K H N I T | Ring formation |
| 24 | Y X Q G T F T S D Y S K Y L D E C R A K E F V Q W L K N G G P S S G A P P P S | Ring formation |
| 25 | H X Q G T F T S D C S K Y L D E R A A Q D F V Q W L L D G G P S S G A P P P S | |
| 26 | H X Q G T F T S D C S K Y L D S R A A Q D F V Q W L L D G G P S S G A P P P S | |
| 27 | H X Q G T F T S D Y S K Y L D E R A C Q D F V Q W L L D Q G G P S S G A P P P S | |
| 28 | H X Q G T F T S D Y S K Y L D E K R A Q E F V C W L L A Q K G K K N D W K H N I T | |
| 29 | H X Q G T F T S D Y S K Y L D E K A A K E F V Q W L L N T C | Ring formation |
| 30 | H X Q G T F T S D Y S K Y L D E K A Q K E F V Q W L L D T C | Ring formation |
| 31 | H X Q G T F T S D Y S K Y L D E K A C K E F V Q W L L A Q | Ring formation |
| 32 | H X Q G T F T S D Y S K Y L D E K A C K D F V Q W L L D G G P S S G A P P P S | Ring formation |
| 33 | H X Q G T F T S D Y S I A M D E I H Q K D F V N W L L A Q K C | Ring formation |
| 34 | H X Q G T F T S D Y S K Y L D E K R Q K E F V N W L L A Q K C | Ring formation |
| 35 | H X Q G T F T S D Y S I A M D E I H Q K D F V N W L L N T K C | Ring formation |
| 36 | H X Q G T F T S D Y S K Y L C E K R Q K E F V Q W L L N G G P S S G A P P P S G | Ring formation |
| 37 | H X Q G T F T S D Y S K Y L D E C R Q K E F V Q W L L N G G P S S G A P P P S G | Ring formation |
| 38 | CA X Q G T F T S D K S S Y L D E R A A Q D F V Q W L L D G G P S S G A P P P S S | |
| 39 | H X Q G T F T S D Y S K Y L D G Q H A Q C F V A W L L A G G G P S S G A P P P S | |
| 40 | H X Q G T F T S D K S K Y L D E R A C Q D F V Q W L L D G G P S S G A P P P S | |
| 41 | H X Q G T F T S D K S K Y L D E C A A Q D F V Q W L L D G G P S S G A P P P S | |
| 42 | Y X Q G T F T S D Y S K Y L D E K R A K E F V Q W L L D H H P S S G Q P P P S C | Ring formation |
| 43 | Y X Q G T F T S D Y S K Y L D E K R A K E F V Q W L L D H H C S S G Q P P P S | Ring formation |
| 44 | HGQGTFTSDCSKQLDGQAAQEFVAWLLAGGP SSGAPPPS | |
| 45 | H G Q G T F T S D C S K Y M D G Q A A Q D F V A W L L A G G P S S G A P P P S | |
| 46 | HGQGTFTSDCSKYLDEQHAQEFVAWLLAGGP SSGAPPPS | |
| 47 | HGQGTFTSDCSKYLDGQRAQEFVAWLLAGGP SSGAPPPS | |
| 48 | H G Q G T F T S D C S K Y L D G Q R A Q D F V N W L L A G G P S S G A P P P S | |
| 49 | C A X Q G T F T S D Y S I C M D E I H Q K D F V N W L L N T K | Ring formation |
| 50 | H X Q G T F T S D Y S K Y L D E K R A K E F V Q W L L D H H P S S G Q P P P S C | Ring formation |
| 51 | H X Q G T F T S D Y S K Y L D E K R Q K E F V Q W L L N T C | Ring formation |
| 52 | H X Q G T F T S D Y S K Y L D E K R Q K E F V Q W L L D T C | Ring formation |
| 53 | H X E G T F T S D Y S I A M D E I H Q K D F V N W L L A Q C | Ring formation |
| 54 | H X E G T F T S D Y S I A M D E I H Q K D F V D W L L A E C | Ring formation |
| 55 | H X Q G T F T S D Y S I A M D E I H Q K D F V N W L L A Q C | Ring formation |
| 56 | H X Q G T F T S D Y S K Y L D E K R Q K E F V N W L L A Q C | Ring formation |
| 57 | H X Q G T F T S D Y S I A M D E I H Q K D F V N W L L N T C | Ring formation |
| 58 | H X Q G T F T S D Y S K Y L D E K R Q K E F V Q W L L N T K C | Ring formation |
| 59 | CA X Q G T F T S D Y S I C M D E K H Q K D F V N W L L N T K | Ring formation |
| 60 | C A X Q G T F T S D Y S I A M D E K H C K D F V N W L L N T K | Ring formation |
| 61 | C A X Q G T F T S D Y S I A M D E I A C K D F V N W L L N T K | Ring formation |
| 62 | CAXQGTFTSDKSKYLDERAAQDFVQWLLDGGP SSGAPPPS | - |
| 63 | CA X Q G T F T S D C S K Y L D E R A A Q D F V Q W L L D G G P S S G A P P P S | - |
| 64 | Y X Q G T F T S D Y S K Y L D E C A A K E F V Q W L L D H H P S S G Q P P P S | Ring formation |
| 65 | H X Q G T F T S D Y S K C L D E K R A K E F V Q W L L D H H P S S G Q P P P S | Ring formation |
| 66 | Y X Q G T F T S D Y S K Y L D E C R A K D F V Q W L L D H H P S S G Q P P P S | Ring formation |
| 67 | Y X Q G T F T S D Y S K Y L D E C A A K D F V Q W L L D H H P S S G Q P P P S | Ring formation |
| 68 | Y X Q G T F T S D Y S K C L D E K A A K E F V Q W L L D H H P S S G Q P P P S | Ring formation |
| 69 | Y X Q G T F T S D Y S K C L D E R A A K E F V Q W L L D H H P S S G Q P P P S | Ring formation |
| 70 | Y X Q G T F T S D Y S K C L D E K R A K D F V Q W L L D H H P S S G Q P P P S | Ring formation |
| 71 | Y X Q G T F T S D Y S K Y L D E R A C K D F V Q W L L D H H P S S G Q P P P S | Ring formation |
| 72 | Y X Q G T F T S D C S K Y L D E R A A K D F V Q W L L D H H P S S G Q P P P S | Ring formation |
| 73 | C A X Q G T F T S D Y S K Y L D E C R A K E F V Q W L L D H H P S S G Q P P P S | Ring formation |
| 74 | C A X Q G T F T S D Y S K C L D E K R A K E F V Q W L L D H H P S S G Q P P P S | Ring formation |
| 75 | Y X Q G T F T S D Y S K Y L D E K A A K E F V Q W L L D H H P S S G Q P P P S C | Ring formation |
| 76 | Y X Q G T F T S D Y S K Y L D E K R A K D F V Q W L L D H H P S S G Q P P P S C | Ring formation |
| 77 | Y X Q G T F T S D Y S K Y L D E K A A K D F V Q W L L D H H P S S G Q P P P S C | Ring formation |
| 78 | H X Q G T F T S D Y S K Y L D E K R Q K E F V Q W L L D T K C | Ring formation |
| 79 | H X E G T F T S D Y S I A M D E I H Q K D F V N W L L A Q K C | Ring formation |
| 80 | H X E G T F T S D Y S I A M D E I H Q K D F V D W L L A E K C | Ring formation |
| 81 | CA X Q G T F T S D Y S K Y L D E K R Q K E F V Q W L L N T C | Ring formation |
| 82 | CA X Q G T F T S D Y S K Y L D E K R Q K E F V Q W L L D T C | Ring formation |
| 83 | CA X E G T F T S D Y S I A M D E I H Q K D F V N W L L A Q C | Ring formation |
| 84 | CA X E G T F T S D Y S I A M D E I H Q K D F V D W L L A E C | Ring formation |
| 85 | CA X Q G T F T S D Y S I A M D E I H Q K D F V N W L L A Q C | Ring formation |
| 86 | CA X Q G T F T S D Y S K Y L D E K R Q K E F V N W L L A Q C | Ring formation |
| 87 | CA X Q G T F T S D Y S I A M D E I H Q K D F V N W L L N T C | Ring formation |
| 88 | CA X Q G T F T S D Y S K Y L D E K R Q K E F V Q W L L N T K C | Ring formation |
| 89 | CA X Q G T F T S D Y S K Y L D E K R Q K E F V Q W L L D T K C | Ring formation |
| 90 | CA X E G T F T S D Y S I A M D E I H Q K D F V N W L L A Q K C | Ring formation |
| 91 | CA X E G T F T S D Y S I A M D E I H Q K D F V D W L L A E K C | Ring formation |
| 92 | CA X Q G T F T S D Y S I A M D E I H Q K D F V N W L L A Q K C | Ring formation |
| 93 | CA X Q G T F T S D Y S K Y L D E K R Q K E F V N W L L A Q K C | Ring formation |
| 94 | CA X Q G T F T S D Y S I A M D E I H Q K D F V N W L L N T K C | Ring formation |
| 95 | Y X Q G T F T S D Y S K Y L D E K R A K E F V Q W L L C H H P S S G Q P P P S | Ring formation |
| 96 | Y X Q G T F T S D Y S K Y L D E K R A K E F V Q W L L D H C P S S G Q P P P S | Ring formation |
| 97 | Y X Q G T F T S D Y S K Y L D E K R A K E F V Q W L L D C H P S S G Q P P P S | Ring formation |
| 98 | Y X Q G T F T S D Y S K A L D E K A A K E F V N W L L D H H P S S G Q P P P S C | Ring formation |
| 99 | Y X Q G T F T S D Y S K A L D E K A A K D F V N W L L D H H P S S G Q P P P S C | Ring formation |
| 100 | Y X Q G T F T S D Y S K A L D E K A A K E F V Q W L L D Q H P S S G Q P P P S C | Ring formation |
| 101 | Y X Q G T F T S D Y S K A L D E K A A K E F V N W L L D Q H P S S G Q P P P S C | Ring formation |
| 102 | Y X Q G T F T S D Y S K A L D E K A A K D F V N W L L D Q H P S S G Q P P P S C | Ring formation |

In the sequences shown in Table 1, the amino acid marked with X represents aminoisobutyric acid (Aib), which is a non-native amino acid, and the underlined amino acids represent amino acids that form a ring together. In Table 1, CA represents 4-imidazoacetyl, and Y represents tyrosine.

### (2) Preparation of long-acting conjugates of triple agonists

To PEGylate 10 kDa PEG having a maleimide group and an aldehyde group at both ends thereof, that is, maleimide―PEG―aldehyde (10 kDa, NOF, Japan), to the cysteine residue of the triple agonists (SEQ ID NOS: 21, 22, 42, 43, 50, 77, and 96) in Example 1, each of the triple agonists and maleimide―PEG― aldehyde were reacted at a molar ratio of 1:1―3 with a protein concentration of 1-5 mg/mL at a low temperature for 0.5―3 hours. The reaction was conducted in an environment in which 20―60% isopropanol was added to 50 mM Tris buffer (pH 7.5). Upon completion of the reaction, the reaction solution was applied to SP Sepharose HP (GE Healthcare, USA) to purify the cysteine-mono-PEGylated triple agonist.

Then, the purified mono-PEGylated triple agonist and immunoglobulin Fc were reacted at a molar ratio of 1:1―5 with a protein concentration of 10-50 mg/mL at 4―8°C for 12―18 hours. The reaction was conducted in an environment in which 10―50 mM sodium cyanoborohydride as a reducing agent and 10―30% isopropanol were added to 100 mM potassium phosphate buffer (pH 6.0). Upon completion of the reaction, the reaction solution was applied to a Butyl Sepharose FF purification column (GE Healthcare, USA) and a Source ISO purification column (GE Healthcare, USA) to purify a conjugate of the triple agonist and immunoglobulin Fc.

The purity analyzed by reverse-phase chromatography, size-exclusion chromatography, and ion-exchange chromatography after the preparation was 95% or more.

A conjugate in which the triple agonist of SEQ ID NO: 21 and immunoglobulin Fc were linked via PEG was named "conjugate containing SEQ ID NO: 21 and immunoglobulin Fc", "long-acting conjugate of SEQ ID NO: 21", or "SEQ ID NO: 22 long-acting conjugate", and these may be used interchangeably herein.

A conjugate in which the triple agonist of SEQ ID NO: 22 and immunoglobulin Fc were linked via PEG was named "conjugate containing SEQ ID NO: 22 and immunoglobulin Fc", "long-acting conjugate of SEQ ID NO: 22", or "SEQ ID NO: 22 long-acting conjugate", and these may be used interchangeably herein.

A conjugate in which the triple agonist of SEQ ID NO: 42 and immunoglobulin Fc were linked via PEG was named "conjugate containing SEQ ID NO: 42 and immunoglobulin Fc", "long-acting conjugate of SEQ ID NO: 42", or "SEQ ID NO: 42 long-acting conjugate", and these may be used interchangeably herein.

A conjugate in which the triple agonist of SEQ ID NO: 43 and immunoglobulin Fc were linked via PEG was named "conjugate containing SEQ ID NO: 43 and immunoglobulin Fc", "long-acting conjugate of SEQ ID NO: 43", or "SEQ ID NO: 43 long-acting conjugate", and these may be used interchangeably herein.

A conjugate in which the triple agonist of SEQ ID NO: 50 and immunoglobulin Fc were linked via PEG was named "conjugate containing SEQ ID NO: 50 and immunoglobulin Fc", "long-acting conjugate of SEQ ID NO: 50", or "SEQ ID NO: 50 long-acting conjugate", and these may be used interchangeably herein.

A conjugate in which the triple agonist of SEQ ID NO: 77 and immunoglobulin Fc were linked via PEG was named "conjugate containing SEQ ID NO: 77 and immunoglobulin Fc", "long-acting conjugate of SEQ ID NO: 77", or "SEQ ID NO: 77 long-acting conjugate", and these may be used interchangeably herein.

A conjugate in which the triple agonist of SEQ ID NO: 96 and immunoglobulin Fc were linked via PEG was named "conjugate containing SEQ ID NO: 96 and immunoglobulin Fc", "long-acting conjugate of SEQ ID NO: 96", or "SEQ ID NO: 96 long-acting conjugate", and these may be used interchangeably herein.

### Example 2: Preparation of long-acting conjugates of native insulin

To PEGylate 3.4K propion-ALD(2) PEG (3.4 kDa PEG having one propionaldehyde group at each end, NOF, Japan) at the N-terminus of the B-chain of the human native insulin (India, Biocon), native insulin and PEG were reacted at a molar ratio of 1:4 with a native insulin concentration of 5 mg/mL at 25°C for 2 hours. The reaction was conducted by adding 3 mM sodium cyanoborohydride (NaCNBH₃) as a reducing agent in a mixture solvent of 50 mM sodium citrate buffer (pH 5.0) and 45% isopropanol. The reaction solution was purified using an SP-HP (GE Healthcare) column with a buffer containing sodium citrate (pH 3.0) and 45% EtOH and a KCI concentration gradient.

Next, to link the native insulin-attached PEG to the N-terminus of the immunoglobulin Fc fragment, the purified mono-PEGylated insulin and the immunoglobulin Fc fragment were reacted at a molar ratio of 1:1.2 at 25°C for 15 hours while the concentration of total proteins was 20 mg/mL. For the reaction solution, 20 mM sodium cyanoborohydride as a reducing agent was added to 100 mM HEPES buffer (pH 8.2) and sodium chloride.

After completion of the reaction, the reaction solution was applied to a Q-HP column (GE, USA) using Tris-HCl buffer (pH 7.5) and a NaCl concentration gradient, and applied to Source 15ISO (GE, USA) using ammonium sulfate and a concentration gradient of Tris-HCl (pH 7.5), thereby purifying a native insulin-3.4K PEG-immunoglobulin Fc conjugate.

### Test Example 1: Determination of in vitro activity of triple agonists and long-acting conjugates thereof

To determine the activity of the triple agonists and long-acting conjugates thereof prepared in Example 1, a method of determining cell activity *in vitro* by using cell lines into which GLP-1, glucagon (GCG), and GIP receptors were transformed, respectively, was used.

The cell lines were obtained by transforming Chinese hamster ovary (CHO) cells to express human GLP-1 receptor, human GCG receptor, and human GIP receptor, respectively, and are suitable for the determination of the activity of GLP-1, GCG, and GIP. Therefore, the activity for the receptors were determined using the transformed cell lines, respectively.

For the determination of GLP-1 activity of the triple agonists and the long-acting conjugates thereof prepared in Examples 1 and 2, human GLP-1 was serially diluted, and the triple agonists and the long-acting conjugates thereof prepared in Examples 1 and 2 were serially diluted. The cultures were removed from the cultured CHO cells expressing the human GLP-1 receptor, and each of the serially diluted substances was added in 5 µL to the cells, and then 5 µL of a buffer containing cAMP antibody was added thereto, followed by incubation at room temperature for 15 minutes. Then, a detection mix containing a cell lysis buffer was added in 10 µL to lyse the cells, followed by incubation at room temperature for 90 minutes. The cell lysate upon the completion of the incubation was applied to the LANCE cAMP kit (PerkinElmer, USA) to calculate EC₅₀ values through accumulated cAMP, and then the values were compared with each other. Relative titers compared with human GLP-1 are shown in Tables 2 and 3 below.

For the determination of GGG activity of the triple agonists and the long-acting conjugates thereof prepared in Examples 1 and 2, human GGG was serially diluted, and the triple agonists and the long-acting conjugates thereof prepared in Examples 1 and 2 were serially diluted. The cultures were removed from the cultured CHO cells expressing the human GGG receptor, and each of the serially diluted substances was added in 5 µL to the cells, and then 5 µL of a buffer containing cAMP antibody was added thereto, followed by incubation at room temperature for 15 minutes. Then, a detection mix containing a cell lysis buffer was added in 10 µL to lyse the cells, followed by incubation at room temperature for 90 minutes. The cell lysate upon the completion of the incubation was applied to the LANCE cAMP kit (PerkinElmer, USA) to calculate EC₅₀ values through accumulated cAMP, and then the values were compared with each other. Relative titers compared with human GGG are shown in Tables 2 and 3 below.

For the determination of GIP activity of the triple agonists and the long-acting conjugates thereof prepared in Examples 1 and 2, human GIP was serially diluted, and the triple agonists and the long-acting conjugates thereof prepared in Examples 1 and 2 were serially diluted. The cultures were removed from the cultured CHO cells expressing the human GIP receptor, and each of the serially diluted substances was added in 5 µL to the cells, and then 5 µL of a buffer containing cAMP antibody was added thereto, followed by incubation at room temperature for 15 minutes. Then, a detection mix containing a cell lysis buffer was added in 10 µL to lyse the cells, followed by incubation at room temperature for 90 minutes. The cell lysate upon the completion of the incubation was applied to the LANCE cAMP kit (PerkinElmer, USA) to calculate EC₅₀ values through accumulated cAMP, and then the values were compared with each other. Relative titers compared with human GIP are shown in Tables 2 and 3 below.

**TABLE 2**

| Relative titer ratios of triple agonists | | | |
|---|---|---|---|
| **SEQ ID NO** | *In vitro* activity (%) compared with native peptide | | |
| | *vs.* GLP-1 | *vs.* Glucagon | *vs.* GIP |
| 1 | 3.2 | <0.1 | <0.1 |
| 2 | 5.9 | <0.1 | <0.1 |
| 3 | 1.8 | <0.1 | <0.1 |
| 4 | 8.5 | <0.1 | <0.1 |
| 5 | 42.1 | <0.1 | <0.1 |
| 6 | 17.0 | <0.1 | <0.1 |
| 7 | 13.7 | <0.1 | <0.1 |
| 8 | 14.2 | 0.10 | <0.1 |
| 9 | 32.1 | 0.13 | <0.1 |
| 10 | 46.0 | <0.1 | <0.1 |
| 11 | 1.4 | <0.1 | <0.1 |
| 12 | 0.4 | <0.1 | <0.1 |
| 13 | <0.1 | <0.1 | <0.1 |
| 14 | 28.0 | <0.1 | <0.1 |
| 15 | 79.2 | <0.1 | <0.1 |
| 16 | 2.1 | <0.1 | <0.1 |
| 17 | 0.2 | <0.1 | <0.1 |
| 18 | <0.1 | <0.1 | <0.1 |
| 19 | <0.1 | <0.1 | <0.1 |
| 20 | <0.1 | <0.1 | <0.1 |
| 21 | 17.8 | 267 | 22.7 |
| 22 | 20.1 | 140 | 59.7 |
| 23 | 4.01 | 9.3 | <0.1 |
| 24 | 41.2 | 9.3 | <0.1 |
| 25 | 82.6 | 0.1 | <0.1 |
| 26 | 64.5 | 0.2 | <0.1 |
| 27 | 83.1 | 0.8 | 0.9 |
| 28 | 17.2 | 1.6 | <0.1 |
| 29 | 38.5 | 6.0 | <0.1 |
| 30 | 142 | 0.7 | 0.8 |
| 31 | 135 | 2.2 | 2.4 |
| 32 | 151 | 1.7 | 8.8 |
| 33 | 24.5 | <0.1 | 10.4 |
| 34 | 19.1 | 0.92 | 0.6 |
| 35 | 7.5 | <0.1 | 1.3 |
| 36 | 37.4 | 0.39 | 0.2 |
| 37 | 236 | 6.21 | 2.2 |
| 38 | 2.3 | - | - |
| 39 | 13.9 | 0.53 | <0.1 |
| 40 | 75.2 | <0.1 | <0.1 |
| 41 | 34.3 | <0.1 | <0.1 |
| 42 | 33.9 | 205.8 | 7.8 |
| 43 | 12.6 | 88.4 | 3.70 |
| 44 | 1.3 | <0.1 | <0.1 |
| 45 | 6.6 | <0.1 | <0.1 |
| 46 | 1.4 | <0.1 | <0.1 |
| 47 | 2.4 | <0.1 | <0.1 |
| 48 | 1.5 | <0.1 | <0.1 |
| 49 | 29.8 | <0.1 | 3.3 |
| 50 | 67.4 | 50.5 | 2.7 |
| 51 | 14.4 | 2.0 | 0.1 |
| 52 | 44.1 | 7.5 | 0.3 |
| 53 | 161 | 8.4 | 1.3 |
| 54 | 30.6 | 1.4 | 0.1 |
| 55 | 27.1 | 0.7 | 2.4 |
| 56 | 57.9 | 4.9 | 0.8 |
| 57 | 11.7 | <0.1 | 0.3 |
| 58 | 39.1 | 2.6 | 0.2 |
| 59 | 40.3 | <0.1 | 4.0 |
| 60 | 106.2 | <0.1 | 8.2 |
| 61 | 59.8 | <0.1 | 2.8 |
| 62 | 5.2 | <0.1 | <0.1 |
| 63 | 15.3 | <0.1 | <0.1 |
| 64 | 64.6 | 60.1 | 92.9 |
| 65 | 95.4 | 25.2 | 11.6 |
| 66 | 15.8 | 172 | 17.2 |
| 67 | 28.5 | 46.2 | 39.8 |
| 68 | 27.9 | 8.8 | 107 |
| 69 | 24.3 | 9.6 | 62.8 |
| 70 | 15.1 | 71.3 | 64.4 |
| 71 | 90.1 | 12.7 | 94.7 |
| 72 | 11.5 | 1.0 | 1.6 |
| 73 | 22.6 | 5.4 | 3.0 |
| 74 | 12.9 | 0.9 | 1.0 |
| 75 | 35.1 | 8.5 | 18.0 |
| 76 | 10.3 | 47.6 | 11.7 |
| 77 | 38.7 | 12.2 | 35.5 |
| 78 | 51.0 | 14.0 | 0.12 |
| 79 | 41.5 | 4.9 | 1.4 |
| 80 | 8.1 | 0.0 | 0.1 |
| 81 | 7.8 | 0.3 | <0.1 |
| 82 | 9.5 | 1.1 | <0.1 |
| 83 | 47.3 | 1.3 | 0.4 |
| 84 | 4.2 | <0.1 | <0.1 |
| 85 | 4.3 | <0.1 | 0.3 |
| 86 | 28.4 | 0.4 | 0.2 |
| 87 | 0.9 | <0.1 | <0.1 |
| 88 | 9.6 | 0.3 | <0.1 |
| 89 | 7.1 | 0.7 | <0.1 |
| 90 | 7.4 | <0.1 | <0.1 |
| 91 | 31.9 | 16.8 | 0.3 |
| 92 | 0.8 | <0.1 | 0.4 |
| 93 | 5.7 | 0.3 | 0.7 |
| 94 | 0.5 | <0.1 | <0.1 |
| 95 | 2.1 | 0.4 | <0.1 |
| 96 | 34.4 | 194.8 | 5.2 |
| 97 | 10.5 | 62.8 | 2.6 |
| 98 | 28.1 | 8.2 | 47.1 |
| 99 | 20.9 | 14.9 | 57.7 |
| 100 | 42.2 | 12.7 | 118.5 |
| 101 | 23.2 | 13.9 | 40.1 |
| 102 | 23.3 | 29.5 | 58.0 |

**TABLE 3**

| Relative titer ratios of triple agonists | | | |
|---|---|---|---|
| Long-acting conjugate | *In vitro* activity (%) compared with native peptide | | |
| | vs. GLP-1 | vs. Glucagon | vs. GIP |
| SEQ ID NO: 21 long-acting conjugate | 0.1 | 1.6 | 0.2 |
| SEQ ID NO: 22 long-acting conjugate | 0.1 | 0.9 | 0.5 |
| SEQ ID NO: 42 long-acting conjugate | 3.1 | 23.1 | 1.2 |
| SEQ ID NO: 43 long-acting conjugate | 2.1 | 13.5 | 0.6 |
| SEQ ID NO: 50 long-acting conjugate | 15.4 | 6.9 | 0.7 |
| SEQ ID NO: 77 long-acting conjugate | 6.7 | 1.7 | 6.6 |
| SEQ ID NO: 96 long-acting conjugate | 0.3 | 4.0 | 0.3 |

### Test Example 2: Blood glucose control effect and A body weight in type II diabetic model mice by combined administration of long-acting conjugate of insulin and long-acting conjugate of triple agonist

To determine *in vivo* effects due to the administration of a composition containing a long-acting conjugate of the triple agonist (SEQ ID NO: 42) prepared in Example 1 or a long-acting conjugate of native insulin prepared in Example 2, or the combined administration of a long-acting conjugate of the triple agonist (SEQ ID NO: 42) and a long-acting conjugate of native insulin, db/db mice (Charles River, Japan), type II diabetic model mice, were used. The db/db mice (BKS.Cg-+Lepr^{db}/+ Lepr^{db}/OlaHsd mouse) were used in the present test example since the mice showed diabetic symptoms due to the removal of leptin receptors.

The blood glucose levels of 8-week-old db/db mice were measured using a glucometer (OneTouch Ultra, LifeScan, Inc., USA) from one to two drops of blood taken from the caudal vein using a 26-G syringe. The induction of diabetes was determined by the measured blood glucose levels (between 350 mg/dL and 600 mg/dL). The diabetes-induced mice were classified into four groups: G1, G2, G3, and G4, each group having seven mice.

The groups were assigned as a control group (Vehicle), a group with administration of a long-acting conjugate of native insulin (15.8 nmol/kg/Q2D), a group with administration of a long-acting conjugate of the triple agonist (1.4 nmol/kg/Q2D), and a group with combined administration of a long-acting conjugate of native insulin (15.8 nmol/kg/Q2D) and a long-acting conjugate of the triple agonist (1.4 nmol/kg/Q2D). After repeated administration of the test substances for two weeks, glycosylated hemoglobin (HbA1c) levels were measured for each group. Glycosylated hemoglobin is in the form in which glucose is bound to hemoglobin normally present in erythrocytes, and when blood glucose levels remain high, glycosylated hemoglobin levels also increase. The changes in body weight (ΔBW) of the test animals prior to the drug administration and on the last day of the test were calculated.

As a result, the group with combined administration of a long-acting conjugate of native insulin and a long-acting conjugate of the triple agonist showed a reduction in glycosylated hemoglobin level (FIG. 1). Specifically, as shown in FIG. 1, the change in glycosylated hemoglobin level compared with the control group was -0.1 in the group with administration of a long-acting conjugate of insulin; -0.3 in the group with administration of a long-acting conjugate of the triple agonist; and -0.5 in the group with combined administration of a long-acting conjugate of insulin and a long-acting conjugate of the triple agonist.

The combined administration showed significantly improved results compared with the group with administration of a long-acting conjugate of native insulin alone or the group with a long-acting conjugate of the triple agonist alone.

The measurement results of Δ body weight indicated that the group with combined administration of a long-acting conjugate of native insulin and a long-acting conjugate of the triple agonist showed an improved effect on weight gain compared with the group with administration of a long-acting conjugate of insulin alone and a significant weight loss effect compared with the control group (FIG. 2). Specifically, as shown in FIG. 2, the change in body weight was +15.8% in the control group; +23.1% in the group with administration of a long-acting conjugate of insulin; -9.3% in the group with administration of a long-acting conjugate of the triple agonist; and -3.8% in the group with combined administration of a long-acting conjugate of insulin and a long-acting conjugate of the triple agonist.

These results indicate that the combined administration of a long-acting conjugate of insulin and a long-acting conjugate of the triple agonist of the present invention can exhibit an excellent blood glucose control effect compared with the administration of a long-acting conjugate of insulin or a long-acting conjugate of the triple agonist alone, and the combined administration of a long-acting conjugate of insulin and a long-acting conjugate of the triple agonist of the present invention can significantly reduce a side effect of weight gain due to the administration of insulin alone.

While the present invention has been described with reference to the particular illustrative embodiments, a person skilled in the art to which the present invention pertains can understand that the present invention may be embodied in other specific forms without departing from the technical spirit or essential characteristics thereof. Therefore, the examples described above should be construed as exemplifying and not limiting the present invention. The scope of the present invention is not defined by the detailed description as set forth above but by the accompanying claims of the invention, and it should also be understood that all changes or modifications derived from the definitions and scopes of the claims and their equivalents fall within the scope of the invention.

## Claims

1. A pharmaceutical composition for prevention or treatment of an insulin-associated disease, the composition comprising:
(i) insulin; and
(ii) an isolated peptide having activity to glucagon, glucagon-like peptide-1 (GLP-1), and glucose-dependent insulinotropic polypeptide (GIP) receptors.

2. The composition of claim 1, wherein the insulin is in the form of a long-acting conjugate in which a biocompatible substance capable of increasing the *in vivo* half-life of the insulin is conjugated to the insulin.

3. The composition of claim 1, wherein the isolated peptide having activity to glucagon, GLP-1, and GIP receptors is in the form of a long-acting conjugate in which a biocompatible substance capable of increasing the *in vivo* half-life of the isolated peptide is conjugated to the isolated peptide.

4. The composition of claim 1, wherein the insulin is in the form of a long-acting conjugate in which a biocompatible substance capable of increasing the *in vivo* half-life of the insulin is conjugated to the insulin; and
wherein the isolated peptide having activity to glucagon, GLP-1, and GIP receptors is in the form of a long-acting conjugate in which a biocompatible substance capable of increasing the *in vivo* half-life of the isolated peptide is conjugated to the isolated peptide.

5. The composition of claim 1, wherein the composition is administered to a subject in need of insulin administration.

6. The composition of claim 1, wherein the insulin-associated disease is selected from the group consisting of insulin resistance disorders, diabetes, hyperglycemia, and obesity.

7. The composition of any one of claims 1 to 6, wherein the composition has both a blood glucose lowering effect and an effect of suppressing weight gain caused by administration of insulin alone.

8. The composition of any one of claims 1 to 6, wherein the composition comprises both of: insulin or a long-acting conjugate thereof; and an isolated peptide having activity to glucagon, GLP-1, and GIP receptors or a long-acting conjugate thereof; and
wherein the insulin or the conjugate thereof and the isolated peptide having activity to glucagon, GLP-1, and GIP receptors or the long-acting conjugate thereof are contained at a molar ratio of 1:1 to 100:1.

9. The composition of any one of claims 1 to 6, wherein the composition comprises both of: insulin or a long-acting conjugate thereof; and an isolated peptide having activity to glucagon, GLP-1, and GIP receptors or a long-acting conjugate thereof; and
wherein the insulin or the conjugate thereof and the isolated peptide having activity to glucagon, GLP-1, and GIP receptors or the long-acting conjugate thereof are contained at a molar ratio of 1:1 to 1:100.

10. The composition of any one of claims 1 to 6, wherein the isolated peptide having activity to glucagon, GLP-1, and GIP receptors is an analog of native glucagon, which has an alteration selected from the group consisting of a substitution, an addition, a deletion, a modification, and a combination thereof of at least one amino acid in the sequence of the native glucagon.

11. The composition of claim 10, wherein an amino acid sequence to be added is derived from the amino acid sequence of native GLP-1, native GIP, or native exendin-4.

12. The composition of claim 1, wherein the isolated peptide having activity to glucagon, GLP-1, and GIP receptors contains an amino acid sequence represented by General Formula 1 below: 
Xaa1–Xaa2–Xaa3–Gly–Thr–Phe–Xaa7–Ser–Asp–Xaa10–Ser–Xaa12–
Xaa13–Xaa14–Xaa15–Xaa16–Xaa17–Xaa18–Xaa19–Xaa20–Xaa21–Phe–
Xaa23–Xaa24–Trp–Leu–Xaa27–Xaa28–Xaa29–Xaa30–R1 (General Formula 1,
SEQ ID NO: 103),
 wherein, in General Formula 1,
Xaa1 is histidine (His, H), 4-imidazoacetyl (CA), or tyrosine (Tyr, Y);
Xaa2 is glycine (Gly, G), α-methyl-glutamic acid, or aminoisobutyric acid (Aib);
Xaa3 is glutamic acid (Glu, E) or glutamine (Gin, Q);
Xaa7 is threonine (Thr, T) or isoleucine (Ile, I);
Xaa10 is leucine (Leu, L), tyrosine (Tyr, Y), lysine (Lys, K), cysteine (Cys, C), or valine (Val, V);
Xaa12 is lysine (Lys, K), serine (Ser, S), or isoleucine (Ile, I);
Xaa13 is glutamine (Gin, Q), tyrosine (Tyr, Y), alanine (Ala, A), or cysteine (Cys, C);
Xaa14 is leucine (Leu, L), methionine (Met, M), or tyrosine (Tyr, Y);
Xaa15 is cysteine (Cys, C), aspartic acid (Asp, D), glutamic acid (Glu, E), or leucine (Leu, L);
Xaa16 is glycine (Gly, G), glutamic acid (Glu, E), or serine (Ser, S);
Xaa17 is glutamine (Gin, Q), arginine (Arg, R), isoleucine (Ile, I), glutamic acid (Glu, E), cysteine (Cys, C), or lysine (Lys, K);
Xaa18 is alanine (Ala, A), glutamine (Gin, Q), arginine (Arg, R), or histidine (His, H);
Xaa19 is alanine (Ala, A), glutamine (Gin, Q), cysteine (Cys, C), or valine (Val, V);
Xaa20 is lysine (Lys, K), glutamine (Gin, Q), or arginine (Arg, R);
Xaa21 is glutamic acid (Glu, E), glutamine (Gin, Q), leucine (Leu, L), cysteine (Cys, C), or aspartic acid (Asp, D);
Xaa23 is isoleucine (Ile, I) or valine (Val, V);
XXaa24 is alanine (Ala, A), glutamine (Gin, Q), cysteine (Cys, C), asparagine (Asn, N), aspartic acid (Asp, D), or glutamic acid (Glu, E);
Xaa27 is valine (Val, V), leucine (Leu, L), lysine (Lys, K), or methionine (Met, M);
Xaa28 is cysteine (Cys, C), lysine (Lys, K), alanine (Ala, A), asparagine (Asn, N), or aspartic acid (Asp, D);
Xaa29 is cysteine (Cys, C), glycine (Gly, G), glutamine (Gin, Q), threonine (Thr, T), glutamic acid (Glu, E), or histidine (His, H);
Xaa30 is cysteine (Cys, C), glycine (Gly, G), lysine (Lys, K), or histidine (His, H), or is absent; and
R1 is cysteine (Cys, C), GKKNDWKHNIT (SEQ ID NO: 106), m-SSGAPPPS―n (SEQ ID NO: 107), or m-SSGQPPPS-n (SEQ ID NO: 108), or is absent, wherein m is Cys, Pro, or Gly―Pro, and n is Cys, Gly, Ser, or His―Gly, or is absent.

13. The composition of claim 12, wherein Xaa14 is leucine or methionine; and Xaa15 is cysteine, aspartic acid, or leucine.

14. The composition of claim 12, wherein, in General Formula 1,
Xaa2 is glycine, α-methyl-glutamic acid, or Aib;
Xaa7 is threonine,
Xaa10 is tyrosine, cysteine, or valine;
Xaa12 is lysine or isoleucine;
Xaa13 is tyrosine, alanine, glutamine, or cysteine;
Xaa14 is leucine, cysteine, or methionine;
Xaa15 is cysteine, leucine, glutamic acid, or aspartic acid;
Xaa17 is glutamine, arginine, isoleucine, cysteine, glutamic acid, or lysine;
Xaa18 is alanine, glutamine, arginine, or histidine;
Xaa19 is alanine, glutamine, valine, or cysteine;
Xaa20 is lysine, arginine, or glutamine;
Xaa21 is glutamic acid, glutamine, leucine, cysteine, or aspartic acid; Xaa23 is isoleucine or valine;
Xaa24 is cysteine, alanine, glutamine, asparagine, glutamic acid, or aspartic acid; and
Xaa27 is leucine or lysine.

15. The composition of claim 12, wherein the peptide contains an amino acid sequence represented by General Formula 2 below: 
Xaa1–Xaa2–Gln–Gly–Thr–Phe–Thr–Ser–Asp–Xaa10–Ser–Lys–Xaa13–
Xaa14–Xaa15–Xaa16–Xaa17–Xaa18–Xaa19–Xaa20–Xaa21–Phe–Xaa23–
Xaa24–Trp–Leu–Leu–Xaa28–Xaa29–Xaa30–Xaa31–Ser–Ser–Gly–Gln–Pro–Pro–
Pro–Ser–Xaa40 (General Formula 2, SEQ ID NO: 104),
 wherein, in General Formula 2,
Xaa1 is 4-imidazoacetyl, histidine, or tyrosine;
Xaa2 is glycine, α-methyl-glutamic acid, or Aib;
Xaa10 is tyrosine or cysteine;
Xaa13 is alanine, glutamine, tyrosine, or cysteine;
Xaa14 is leucine, methionine, or tyrosine;
Xaa15 is aspartic acid, glutamic acid, or leucine;
Xaa16 is glycine, glutamic acid, or serine;
Xaa17 is glutamine, arginine, isoleucine, glutamic acid, cysteine, or lysine;
Xaa18 is alanine, glutamine, arginine, or histidine;
Xaa19 is alanine, glutamine, cysteine, or valine;
Xaa20 is lysine, glutamine, or arginine;
Xaa21 is cysteine, glutamic acid, glutamine, leucine, or aspartic acid;
Xaa23 is isoleucine or valine;
Xaa24 is cysteine, alanine, glutamine, asparagine, or glutamic acid;
Xaa28 is lysine, cysteine, asparagine, or aspartic acid;
Xaa29 is glycine, glutamine, cysteine, or histidine;
Xaa30 is cysteine, glycine, lysine, or histidine;
Xaa31 is proline or cysteine; and
Xaa40 is cysteine or is absent.

16. The composition of claim 12, wherein, in General Formula 1,
Xaa2 is glycine, α-methyl-glutamic acid, or Aib;
Xaa7 is threonine;
Xaa10 is tyrosine, cysteine, or valine;
Xaa12 is lysine or isoleucine;
Xaa13 is tyrosine, alanine, or cysteine;
Xaa14 is leucine or methionine;
Xaa15 is cysteine or aspartic acid;
Xaa17 is glutamine, arginine, isoleucine, cysteine, or lysine;
Xaa18 is alanine, arginine, or histidine;
Xaa19 is alanine, glutamine, or cysteine;
Xaa20 is lysine or glutamine;
Xaa21 is glutamic acid, cysteine, or aspartic acid;
Xaa23 is valine;
Xaa24 is alanine, glutamine, cysteine, asparagine, or aspartic acid; and
Xaa27 is leucine or lysine.

17. The composition of claim 15, wherein, in General Formula 2,
Xaa13 is alanine, tyrosine, or cysteine;
Xaa15 is aspartic acid or glutamic acid;
Xaa17 is glutamine, arginine, cysteine, or lysine;
Xaa18 is alanine, arginine, or histidine;
Xaa21 is cysteine, glutamic acid, glutamine, or aspartic acid;
Xaa23 is isoleucine or valine;
Xaa24 is cysteine, glutamine, or asparagine;
Xaa28 is cysteine, asparagine, or aspartic acid;
Xaa29 is glutamine, cysteine, or histidine; and
Xaa30 is cysteine, lysine, or histidine.

18. The composition of claim 12, wherein, in General Formula 1,
Xaa2 is α-methyl-glutamic acid or Aib;
Xaa7 is threonine;
Xaa10 is tyrosine or cysteine;
Xaa12 is lysine or isoleucine;
Xaa13 is tyrosine, alanine, or cysteine;
Xaa14 is leucine or methionine;
Xaa15 is cysteine or aspartic acid;
Xaa16 is glutamic acid;
Xaa17 is arginine, isoleucine, cysteine, or lysine;
Xaa18 is alanine, arginine, or histidine;
Xaa19 is alanine, glutamine, or cysteine;
Xaa20 is lysine or glutamine;
Xaa21 is glutamic acid or aspartic acid;
Xaa23 is valine;
Xaa24 is glutamine, asparagine, or aspartic acid;
Xaa27 is leucine; and
Xaa28 is cysteine, alanine, asparagine, or aspartic acid.

19. The composition of claim 12, wherein, in General Formula 1,
Xaa1 is histidine or 4-imidazoacetyl;
Xaa2 is α-methyl-glutamic acid or Aib;
Xaa3 is glutamine;
Xaa7 is threonine;
Xaa10 is tyrosine;
Xaa12 is isoleucine;
Xaa13 is alanine or cysteine;
Xaa14 is methionine;
Xaa15 is aspartic acid;
Xaa16 is glutamic acid;
Xaa17 is isoleucine or lysine;
Xaa18 is alanine or histidine;
Xaa19 is glutamine or cysteine;
Xaa20 is lysine;
Xaa21 is aspartic acid;
Xaa23 is valine;
Xaa24 is asparagine;
Xaa27 is leucine;
Xaa28 is alanine or asparagine;
Xaa29 is glutamine or threonine; and
Xaa30 is cysteine or lysine, or is absent.

20. The composition of claim 11, wherein the peptide contains an amino acid sequence represented by General Formula 3 below: 
Xaa1–Xaa2–Gln–Gly–Thr–Phe–Thr–Ser–Asp–Tyr–Ser–Lys–Xaa13–Leu–
Asp–Glu–Xaa17–Xaa18–Xaa19–Lys–Xaa21–Phe–Val–Xaa24–Trp–Leu–Leu–
Xaa28–Xaa29–Xaa30–Xaa31–Ser–Ser–Gly–Gln–Pro–Pro–Pro–Ser–Xaa40
(General Formula 3, SEQ ID NO: 105),
 wherein, in General Formula 3,
Xaa1 is histidine or tyrosine;
Xaa2 is α-methyl-glutamic acid or Aib;
Xaa13 is alanine, tyrosine, or cysteine;
Xaa17 is arginine, cysteine, or lysine;
Xaa18 is alanine or arginine;
Xaa19 is alanine or cysteine;
Xaa21 is glutamic acid or aspartic acid;
Xaa24 is glutamine or asparagine;
Xaa28 is cysteine or aspartic acid;
Xaa29 is cysteine, histidine, or glutamine;
Xaa30 is cysteine or histidine;
Xaa31 is proline or cysteine; and
Xaa40 is cysteine, or is absent.

21. The composition of claim 12, wherein R1 is cysteine, GKKNDWKHNIT (SEQ ID NO: 106), CSSGQPPPS (SEQ ID NO: 109), GPSSGAPPPS (SEQ ID NO: 110), GPSSGAPPPSC (SEQ ID NO: 111), PSSGAPPPS (SEQ ID NO: 112), PSSGAPPPSG (SEQ ID NO: 113), PSSGAPPPSHG (SEQ ID NO: 114), PSSGAPPPSS (SEQ ID NO: 115), PSSGQPPPS (SEQ ID NO: 116), or PSSGQPPPSC (SEQ ID NO: 117), or is absent.

22. The composition of claim 12, wherein the isolated peptide having activity to glucagon, GLP-1, and GIP receptors contains an amino acid sequence selected from SEQ ID NOS: 1 to 102.

23. The composition of claim 12, wherein the isolated peptide having activity to glucagon, GLP-1, and GIP receptors contains the amino acid sequence set forth in SEQ ID NO: 42.

24. The composition of any one of claims 12 to 21, wherein in the general formulas, the amino acids at positions 16 and 20 from the N-terminus form a ring with each other.

25. The composition of any one of claims 12 to 21, wherein the C-terminus of the isolated peptide having activity to glucagon, GLP-1, and GIP receptors is amidated.

26. The composition of claim 1, wherein the insulin is native insulin, or an insulin analog which has an alteration selected from the group consisting of a substitution, an addition, a deletion, a modification, and a combination thereof of at least one amino acid in the native insulin.

27. The composition of claim 26, wherein the insulin analog has an alteration of at least one amino acid selected from the group consisting of the amino acids at positions 1, 2, 3, 5, 8, 10, 12, 16, 23, 24, 25, 26, 27, 28, 29, and 30 in the B-chain and the amino acids at positions 1, 2, 5, 8, 10, 12, 14, 16, 17, 18, 19, and 21 in the A-chain of the native insulin, the alteration being a substitution with another amino acid, a deletion, or a combination thereof.

28. The composition of claim 226, wherein the insulin analog comprises: an A-chain of SEQ ID NO: 119 represented by General Formula 4; and a B-chain of SEQ ID NO: 120 represented by General Formula 5: 
[General Formula 4]
Xaa1-Xaa2-Val-Glu-Xaa5-Cys-Cys-Thr-Ser-Ile-Cys-Xaa12-Leu-
Xaa14-Gln-Xaa16-Glu-Asn-Xaa19-Cys-Xaa21 (SEQ ID NO: 119),
 wherein, in General Formula 4,
Xaa1 is alanine, glycine, glutamine, histidine, glutamic acid, or asparagine;
Xaa2 is alanine or isoleucine;
Xaa5 is alanine, glutamic acid, glutamine, histidine, or asparagine;
Xaa12 is alanine, serine, glutamine, glutamic acid, histidine, or asparagine;
Xaa14 is alanine, tyrosine, glutamic acid, histidine, lysine, aspartic acid, or asparagine;
Xaa16 is alanine, leucine, tyrosine, histidine, glutamic acid, or asparagine;
Xaa19 is alanine, tyrosine, serine, glutamic acid, histidine, threonine, or asparagine; and
Xaa21 is asparagine, glycine, histidine, or alanine, 
[General Formula 5]
Phe–Val–Asn–Gln–His–Leu–Cys–Xaa8–Ser–His–Leu–Val–Glu–Ala–Leu–
Xaa16–Leu–Val–Cys–Gly–Glu–Arg–Xaa23–Xaa24–Xaa25–Tyr–Xaa27–Xaa28–
Lys–Thr (SEQ ID NO: 120),
wherein, in General Formula 5,
Xaa8 is alanine or glycine;
Xaa16 is tyrosine, glutamic acid, serine, threonine, or aspartic acid, or is absent;
Xaa23 is glycine or alanine;
Xaa24 is alanine or phenylalanine;
Xaa25 is alanine, phenylalanine, aspartic acid, or glutamic acid, or is absent;
Xaa27 is threonine, or is absent; and
Xaa28 is proline, glutamic acid, or aspartic acid, or is absent
(wherein a peptide comprising the A-chain of SEQ ID NO: 121 and the B-chain of SEQ ID NO: 122 is excluded).

29. The composition of claim 26, wherein the insulin analog has a substitution with alanine, of at least one amino acid selected from the group consisting of the amino acids at positions 8, 23, 24, and 25 in the B-chain and the amino acids at positions 1, 2, and 19 in the A-chain of the native insulin, or a substitution of the amino acid at position 14 in the A-chain of the native insulin with glutamic acid or asparagine.

30. The composition of claim 29, wherein the insulin analog contains an amino acid sequence selected from the group consisting of SEQ ID NOS: 124, 126, 128, 130, 132, 134, 136, 138, and 140.

31. The composition of claim 26, wherein the insulin analog has an alteration by: a substitution of the amino acid at position 16 in the B-chain of the native insulin with glutamic acid; a deletion of the amino acid at position 25 in the B-chain of the native insulin; a substitution of the amino acid at position 14 in the A-chain of the native insulin with glutamic acid or alanine; or a combination thereof.

32. The composition of claim 31, wherein the insulin analog contains an amino acid sequence of SEQ ID NO: 142 or 144.

33. The composition of claim 26, wherein the insulin analog has an alteration by: a substitution of the amino acid at position 16 in the B-chain of the native insulin with glutamic acid, serine, threonine, or aspartic acid; a substitution of the amino acid at position 25 in the B-chain of the native insulin with aspartic acid or glutamic acid; a substitution of the amino acid at position 14 in the A-chain of the native insulin with histidine, lysine, alanine, or aspartic acid; a substitution of the amino acid at position 19 in the A-chain of the native insulin with glutamic acid, serine, or threonine; or a combination thereof.

34. The composition of claim 33, wherein the insulin analog contains an amino acid sequence selected from the group consisting of SEQ ID NOS: 146, 148, 150, 152, 154, 156, 158, 160, 162, 164, 166, 168, and 170.

35. The composition of claim 28, wherein the insulin analog is in the form of two polypeptide chains composed of an A-chain of SEQ ID NO: 119 represented by General Formula 4 and a B-chain of SEQ ID NO: 120 represented by General Formula 5.

36. The composition of claim 35, wherein the A-chain and the B-chain are linked via a disulfide linkage.

37. The composition of any one of claims 2 to 4, wherein the conjugate is represented by Chemical Formula 1:
[Chemical Formula 1] X―Lₐ―F,
wherein,
X is the insulin or the isolated peptide having activity to glucagon, GLP-1, and GIP receptors;
L is a linker;
a is 0 or a natural number, with the proviso that when a is 2 or greater, each L is independent from each other;
F is a substance capable of increasing the half-life of X; and
"―" is a covalent or non-covalent linkage.

38. The composition of claim 37, wherein F is selected from the group consisting of polymers, fatty acids, cholesterol, albumin and fragments thereof, albumin-binding substances, polymers of repeating units of particular amino acid sequences, antibodies, antibody fragments, FcRn-binding substances, *in vivo* connective tissues, nucleotides, fibronectin, transferrin, saccharides, heparin, and elastin.

39. The composition of claim 38, wherein the polymers are selected from the group consisting of polyethylene glycol, polypropylene glycol, ethylene glycol-propylene glycol copolymers, polyoxyethylated polyols, polyvinyl alcohols, polysaccharides, dextran, polyvinyl ethyl ether, biodegradable polymers, lipid polymers, chitin, hyaluronic acid, oligonucleotides, and a combination thereof.

40. The composition of claim 37, wherein F is an immunoglobulin Fc region.

41. The composition of claim 40, wherein F is an IgG Fc region.

42. The composition of claim 41, wherein the immunoglobulin Fc region is aglycosylated.

43. The composition of claim 40, wherein the immunoglobulin Fc region is selected from the group consisting of:
(a) a CH1 domain, a CH2 domain, a CH3 domain, and a CH4 domain;
(b) a CH1 domain and a CH2 domain;
(c) a CH1 domain and a CH3 domain;
(d) a CH2 domain and a CH3 domain;
(e) a combination of at least one of a CH1 domain, a CH2 domain, a CH3 domain, and a CH4 domain and an immunoglobulin hinge region or a moiety of the hinge region; and
(f) a dimer of each domain of a heavy chain constant region and a light chain constant region.

44. The composition of claim 40, wherein the immunoglobulin Fc region has a deletion of a site capable of forming a disulfide linkage, a deletion of some amino acids at the N-terminus of native Fc, an addition of a methionine residue at the N-terminus of native Fc, a deletion of a complement-binding site, or deletion of an antibody-dependent cell-mediated cytotoxicity (ADCC) site.

45. The composition of claim 40, wherein the immunoglobulin Fc region is an immunoglobulin Fc fragment derived from IgG, IgA, IgD, IgE, or IgM.

46. The composition of claim 40, wherein the immunoglobulin Fc region is a hybrid of domains with different origins derived from immunoglobulins selected from the group consisting of IgG, IgA, IgD, IgE, and IgM.

47. The composition of claim 37, wherein L is selected from the group consisting of peptides, fatty acids, sugars, polymers, low-molecular-weight compounds, nucleotides, and combinations thereof.

48. The composition of claim 47, wherein the polymers are selected from the group consisting of polyethylene glycol, polypropylene glycol, ethylene glycol-propylene glycol copolymers, polyoxyethylated polyols, polyvinyl alcohols, polysaccharides, dextran, polyvinyl ethyl ether, biodegradable polymers, lipid polymers, chitin, hyaluronic acid, oligonucleotides, and combinations thereof.

49. The composition of claim 37, wherein L is polyethylene glycol.

50. A kit for prevention or treatment of an insulin-associated disease, the kit comprising:
(i) insulin; and
(ii) an isolated peptide having activity to glucagon, GLP-1, and GIP receptors.

51. A pharmaceutical composition for reducing weight gain caused by insulin administration.
(i) insulin; and
(ii) an isolated peptide having activity to glucagon, GLP-1, and GIP receptors.

52. The composition of claim 51, wherein the insulin is in the form of a long-acting conjugate in which a biocompatible substance capable of increasing the *in vivo* half-life of the insulin is conjugated to the insulin.

53. The composition of claim 51, wherein the isolated peptide having activity to glucagon, GLP-1, and GIP receptors is in the form of a long-acting conjugate in which a biocompatible substance capable of increasing the *in vivo* half-life of the isolated peptide is conjugated to the isolated peptide.

54. The composition of claim 51, wherein the insulin is in the form of a long-acting conjugate in which a biocompatible substance capable of increasing the *in vivo* half-life of the insulin is conjugated to the insulin; and
wherein the isolated peptide having activity to glucagon, GLP-1, and GIP receptors is in the form of a long-acting conjugate in which a biocompatible substance capable of increasing the *in vivo* half-life of the isolated peptide is conjugated to the isolated peptide.

55. A complex preparation for weight loss in an insulin-administered patient, the complex preparation comprising:
(i) insulin; and
(ii) an isolated peptide having activity to glucagon, GLP-1, and GIP receptors.

56. The complex preparation of claim 55, wherein the insulin is in the form of a long-acting conjugate in which a biocompatible substance capable of increasing the *in vivo* half-life of the insulin is conjugated to the insulin.

57. The complex preparation of claim 55, wherein the isolated peptide having activity to glucagon, GLP-1, and GIP receptors is in the form of a long-acting conjugate in which a biocompatible substance capable of increasing the *in vivo* half-life of the isolated peptide is conjugated to the isolated peptide.

58. The complex preparation of claim 55, wherein the insulin is in the form of a long-acting conjugate in which a biocompatible substance capable of increasing the *in vivo* half-life of the insulin is conjugated to the insulin; and
wherein the isolated peptide having activity to glucagon, GLP-1, and GIP receptors is in the form of a long-acting conjugate in which a biocompatible substance capable of increasing the *in vivo* half-life of the isolated peptide is conjugated to the isolated peptide.

59. The complex preparation of any one of claims 55 to 58, wherein the composition comprises both of: insulin or a long-acting conjugate thereof; and an isolated peptide having activity to glucagon, GLP-1, and GIP receptors or a long-acting conjugate thereof; and
wherein the insulin and the isolated peptide having activity to glucagon, GLP-1, and GIP receptor are contained at a molar ratio of 1:1 to 100:1.

60. The complex preparation of any one of claims 55 to 58, wherein the composition comprises both of: insulin or a long-acting conjugate thereof; and an isolated peptide having activity to glucagon, GLP-1, and GIP receptors or a long-acting conjugate thereof; and
wherein the insulin or the conjugate thereof and the isolated peptide having activity to a GLP-1 receptor, and a GIP receptor, or the conjugate thereof are contained at a molar ratio of 1:1 to 1:100.


<110>     HANMI PHARM. CO., LTD.

<120>     A pharmaceutical composition comprising insulin and trigonal
          glucagon/GLP-1/GIP receptor agonist

<130>     OPA19304

<150>     KR 10-2018-0167698
<151>     2018-12-21

<160>     170

<170>     KoPatentIn 3.0

<210>     1
<211>     30
<212>     PRT
<213>     Artificial Sequence

<220>
<223>     Trigonal agonist


<220>
<221>     MISC_FEATURE
<222>     (2)
<223>     Xaa is aminoisobutyric acid (Aib)


<400>     1
His Xaa Gln Gly Thr Phe Thr Ser Asp Val Ser Ser Tyr Leu Asp Gly
  1               5                  10                  15

Gln Ala Ala Lys Glu Phe Ile Ala Trp Leu Val Lys Gly Cys
             20                  25                  30


<210>     2
<211>     30
<212>     PRT
<213>     Artificial Sequence

<220>
<223>     Trigonal agonist


<220>
<221>     MISC_FEATURE
<222>     (2)
<223>     Xaa is aminoisobutyric acid (Aib)


<400>     2
His Xaa Gln Gly Thr Phe Thr Ser Asp Val Ser Ser Tyr Leu Asp Gly
  1               5                  10                  15

Gln Ala Gln Lys Glu Phe Ile Ala Trp Leu Val Lys Gly Cys
             20                  25                  30


<210>     3
<211>     41
<212>     PRT

<213>    Artificial Sequence

<220>
<223>    Trigonal agonist


<220>
<221>    MISC_FEATURE
<222>    (2)
<223>    Xaa is aminoisobutyric acid (Aib)


<400>    3
His Xaa Gln Gly Thr Phe Thr Ser Asp Val Ser Ser Tyr Leu Leu Gly
1               5                   10                  15

Gln Ala Ala Lys Gln Phe Ile Ala Trp Leu Val Lys Gly Gly Gly Pro
            20                  25                  30

Ser Ser Gly Ala Pro Pro Pro Ser Cys
        35                  40


<210>    4
<211>    30
<212>    PRT
<213>    Artificial Sequence

<220>
<223>    Trigonal agonist


<220>
<221>    MISC_FEATURE
<222>    (2)
<223>    Xaa is aminoisobutyric acid (Aib)


<400>    4
His Xaa Gln Gly Thr Phe Thr Ser Asp Val Ser Ser Tyr Leu Leu Gly
1               5                   10                  15

Gln Gln Gln Lys Glu Phe Ile Ala Trp Leu Val Lys Gly Cys
            20                  25                  30


<210>    5
<211>    41
<212>    PRT
<213>    Artificial Sequence

<220>
<223>    Trigonal agonist


<220>
<221>    MISC_FEATURE
<222>    (2)
<223>    Xaa is aminoisobutyric acid (Aib)


<400>    5
His Xaa Gln Gly Thr Phe Thr Ser Asp Val Ser Ser Tyr Leu Leu Gly

1               5                   10                  15

Gln Gln Gln Lys Glu Phe Ile Ala Trp Leu Val Lys Gly Gly Gly Pro
            20                  25                  30

Ser Ser Gly Ala Pro Pro Pro Ser Cys
        35                  40

<210>   6
<211>   30
<212>   PRT
<213>   Artificial Sequence

<220>
<223>   Trigonal agonist

<220>
<221>   MISC_FEATURE
<222>   (2)
<223>   Xaa is aminoisobutyric acid (Aib)

<400>   6
His Xaa Gln Gly Thr Phe Thr Ser Asp Val Ser Ser Tyr Leu Asp Gly
1               5                   10                  15

Gln Ala Ala Lys Glu Phe Val Ala Trp Leu Leu Lys Gly Cys
            20                  25                  30

<210>   7
<211>   30
<212>   PRT
<213>   Artificial Sequence

<220>
<223>   Trigonal agonist

<220>
<221>   MISC_FEATURE
<222>   (2)
<223>   Xaa is aminoisobutyric acid (Aib)

<400>   7
His Xaa Gln Gly Thr Phe Thr Ser Asp Val Ser Lys Tyr Leu Asp Gly
1               5                   10                  15

Gln Ala Ala Lys Glu Phe Val Ala Trp Leu Leu Lys Gly Cys
            20                  25                  30

<210>   8
<211>   30
<212>   PRT
<213>   Artificial Sequence

<220>
<223>   Trigonal agonist

<220>
<221>    MISC_FEATURE
<222>    (2)
<223>    Xaa is aminoisobutyric acid (Aib)


<400>    8
His Xaa Gln Gly Thr Phe Thr Ser Asp Val Ser Lys Tyr Leu Asp Gly
1               5                   10                  15

Gln Ala Ala Gln Glu Phe Val Ala Trp Leu Leu Lys Gly Cys
            20                  25                  30


<210>    9
<211>    30
<212>    PRT
<213>    Artificial Sequence

<220>
<223>    Trigonal agonist


<220>
<221>    MISC_FEATURE
<222>    (2)
<223>    Xaa is aminoisobutyric acid (Aib)


<400>    9
His Xaa Gln Gly Thr Phe Thr Ser Asp Val Ser Lys Tyr Leu Asp Gly
1               5                   10                  15

Gln Ala Ala Gln Glu Phe Val Ala Trp Leu Leu Ala Gly Cys
            20                  25                  30


<210>    10
<211>    41
<212>    PRT
<213>    Artificial Sequence

<220>
<223>    Trigonal agonist


<220>
<221>    MISC_FEATURE
<222>    (2)
<223>    Xaa is aminoisobutyric acid (Aib)


<400>    10
His Xaa Gln Gly Thr Phe Thr Ser Asp Val Ser Lys Tyr Leu Asp Gly
1               5                   10                  15

Gln Ala Ala Gln Glu Phe Val Ala Trp Leu Leu Ala Gly Gly Gly Pro
            20                  25                  30

Ser Ser Gly Ala Pro Pro Pro Ser Cys
        35                  40

<210>    11
<211>    41
<212>    PRT
<213>    Artificial Sequence

<220>
<223>    Trigonal agonist


<220>
<221>    MISC_FEATURE
<222>    (1)
<223>    Xaa is 4-imidazoacetyl (CA)


<400>    11
Xaa Gly Glu Gly Thr Phe Thr Ser Asp Leu Ser Lys Tyr Leu Asp Ser
1               5                   10                  15

Arg Arg Gln Gln Leu Phe Val Gln Trp Leu Lys Ala Gly Gly Pro Ser
            20                  25                  30

Ser Gly Ala Pro Pro Pro Ser His Gly
        35                  40


<210>    12
<211>    41
<212>    PRT
<213>    Artificial Sequence

<220>
<223>    Trigonal agonist


<220>
<221>    MISC_FEATURE
<222>    (1)
<223>    Xaa is 4-imidazoacetyl (CA)


<400>    12
Xaa Gly Glu Gly Thr Phe Ile Ser Asp Leu Ser Lys Tyr Met Asp Glu
1               5                   10                  15

Gln Ala Val Gln Leu Phe Val Glu Trp Leu Met Ala Gly Gly Pro Ser
            20                  25                  30

Ser Gly Ala Pro Pro Pro Ser His Gly
        35                  40


<210>    13
<211>    41
<212>    PRT
<213>    Artificial Sequence

<220>
<223>    Trigonal agonist


<220>

<221>    MISC_FEATURE
<222>    (1)
<223>    Xaa is 4-imidazoacetyl (CA)

<400>    13
Xaa Gly Glu Gly Thr Phe Ile Ser Asp Tyr Ser Ile Gln Leu Asp Glu
1               5                   10                  15

Ile Ala Val Gln Asp Phe Val Glu Trp Leu Leu Ala Gln Lys Pro Ser
            20                  25                  30

Ser Gly Ala Pro Pro Pro Ser His Gly
        35                  40

<210>    14
<211>    41
<212>    PRT
<213>    Artificial Sequence

<220>
<223>    Trigonal agonist

<220>
<221>    MISC_FEATURE
<222>    (1)
<223>    Xaa is 4-imidazoacetyl (CA)

<400>    14
Xaa Gly Gln Gly Thr Phe Thr Ser Asp Tyr Ser Ile Gln Leu Asp Glu
1               5                   10                  15

Ile Ala Val Arg Asp Phe Val Glu Trp Leu Lys Asn Gly Gly Pro Ser
            20                  25                  30

Ser Gly Ala Pro Pro Pro Ser His Gly
        35                  40

<210>    15
<211>    41
<212>    PRT
<213>    Artificial Sequence

<220>
<223>    Trigonal agonist

<220>
<221>    MISC_FEATURE
<222>    (1)
<223>    Xaa is 4-imidazoacetyl (CA)

<400>    15
Xaa Gly Gln Gly Thr Phe Thr Ser Asp Leu Ser Lys Gln Met Asp Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser His Gly
        35                  40

<210>   16
<211>   41
<212>   PRT
<213>   Artificial Sequence

<220>
<223>   Trigonal agonist

<220>
<221>   MISC_FEATURE
<222>   (1)
<223>   Xaa is 4-imidazoacetyl (CA)

<400>   16
Xaa Gly Gln Gly Thr Phe Thr Ser Asp Leu Ser Lys Gln Met Asp Ser
 1               5                  10                  15

Glu Ala Gln Gln Leu Phe Ile Glu Trp Leu Lys Asn Gly Gly Pro Ser
            20                  25                  30

Ser Gly Ala Pro Pro Pro Ser His Gly
        35                  40

<210>   17
<211>   41
<212>   PRT
<213>   Artificial Sequence

<220>
<223>   Trigonal agonist

<220>
<221>   MISC_FEATURE
<222>   (1)
<223>   Xaa is 4-imidazoacetyl (CA)

<400>   17
Xaa Gly Gln Gly Thr Phe Thr Ser Asp Leu Ser Lys Gln Met Asp Glu
 1               5                  10                  15

Glu Arg Ala Arg Glu Phe Ile Glu Trp Leu Leu Ala Gln Lys Pro Ser
            20                  25                  30

Ser Gly Ala Pro Pro Pro Ser His Gly
        35                  40

<210>   18
<211>   41
<212>   PRT
<213>   Artificial Sequence

<220>

<223>    Trigonal agonist

<220>
<221>    MISC_FEATURE
<222>    (1)
<223>    Xaa is 4-imidazoacetyl (CA)

<400>    18
Xaa Gly Gln Gly Thr Phe Thr Ser Asp Leu Ser Lys Gln Met Asp Ser
1               5                   10                  15

Glu Arg Ala Arg Glu Phe Ile Glu Trp Leu Lys Asn Thr Gly Pro Ser
            20                  25                  30

Ser Gly Ala Pro Pro Pro Ser His Gly
        35                  40

<210>    19
<211>    41
<212>    PRT
<213>    Artificial Sequence

<220>
<223>    Trigonal agonist

<220>
<221>    MISC_FEATURE
<222>    (1)
<223>    Xaa is 4-imidazoacetyl (CA)

<400>    19
Xaa Gly Gln Gly Thr Phe Thr Ser Asp Leu Ser Ile Gln Tyr Asp Ser
1               5                   10                  15

Glu His Gln Arg Asp Phe Ile Glu Trp Leu Lys Asp Thr Gly Pro Ser
            20                  25                  30

Ser Gly Ala Pro Pro Pro Ser His Gly
        35                  40

<210>    20
<211>    41
<212>    PRT
<213>    Artificial Sequence

<220>
<223>    Trigonal agonist

<220>
<221>    MISC_FEATURE
<222>    (1)
<223>    Xaa is 4-imidazoacetyl (CA)

<400>    20
Xaa Gly Gln Gly Thr Phe Thr Ser Asp Leu Ser Ile Gln Tyr Glu Glu

1                   5                   10                  15

Glu Ala Gln Gln Asp Phe Val Glu Trp Leu Lys Asp Thr Gly Pro Ser
            20                  25                  30

Ser Gly Ala Pro Pro Pro Ser His Gly
        35                  40

<210>   21
<211>   39
<212>   PRT
<213>   Artificial Sequence

<220>
<223>   Trigonal agonist

<220>
<221>   MISC_FEATURE
<222>   (2)
<223>   Xaa is aminoisobutyric acid (Aib)

<220>
<221>   MISC_FEATURE
<222>   (16)..(20)
<223>   amino acids at positions 16 and 20 form a ring

<400>   21
Tyr Xaa Gln Gly Thr Phe Thr Ser Asp Tyr Ser Lys Tyr Leu Asp Glu
1               5                   10                  15

Cys Arg Ala Lys Glu Phe Val Gln Trp Leu Leu Asp His His Pro Ser
            20                  25                  30

Ser Gly Gln Pro Pro Pro Ser
        35

<210>   22
<211>   39
<212>   PRT
<213>   Artificial Sequence

<220>
<223>   Trigonal agonist

<220>
<221>   MISC_FEATURE
<222>   (2)
<223>   Xaa is aminoisobutyric acid (Aib)

<220>
<221>   MISC_FEATURE
<222>   (16)..(20)
<223>   amino acids at positions 16 and 20 form a ring

<400>   22

Tyr Xaa Gln Gly Thr Phe Thr Ser Asp Tyr Ser Lys Cys Leu Asp Glu
1               5                   10                  15

Lys Arg Ala Lys Glu Phe Val Gln Trp Leu Leu Asp His His Pro Ser
            20                  25                  30

Ser Gly Gln Pro Pro Pro Ser
        35

<210>   23
<211>   41
<212>   PRT
<213>   Artificial Sequence

<220>
<223>   Trigonal agonist

<220>
<221>   MISC_FEATURE
<222>   (2)
<223>   Xaa is aminoisobutyric acid (Aib)

<220>
<221>   MISC_FEATURE
<222>   (16)..(20)
<223>   amino acids at positions 16 and 20 form a ring

<400>   23
Tyr Xaa Gln Gly Thr Phe Thr Ser Asp Tyr Ser Lys Tyr Leu Asp Glu
1               5                   10                  15

Cys Arg Ala Lys Glu Phe Val Gln Trp Leu Leu Ala Gln Lys Gly Lys
            20                  25                  30

Lys Asn Asp Trp Lys His Asn Ile Thr
        35                  40

<210>   24
<211>   39
<212>   PRT
<213>   Artificial Sequence

<220>
<223>   Trigonal agonist

<220>
<221>   MISC_FEATURE
<222>   (2)
<223>   Xaa is aminoisobutyric acid (Aib)

<220>
<221>   MISC_FEATURE
<222>   (16)..(20)
<223>   amino acids at positions 16 and 20 form a ring

<400>    24
Tyr Xaa Gln Gly Thr Phe Thr Ser Asp Tyr Ser Lys Tyr Leu Asp Glu
1               5                   10                  15

Cys Arg Ala Lys Glu Phe Val Gln Trp Leu Lys Asn Gly Gly Pro Ser
            20                  25                  30

Ser Gly Ala Pro Pro Pro Ser
        35

<210>    25
<211>    39
<212>    PRT
<213>    Artificial Sequence

<220>
<223>    Trigonal agonist

<220>
<221>    MISC_FEATURE
<222>    (2)
<223>    Xaa is aminoisobutyric acid (Aib)

<400>    25
His Xaa Gln Gly Thr Phe Thr Ser Asp Cys Ser Lys Tyr Leu Asp Glu
1               5                   10                  15

Arg Ala Ala Gln Asp Phe Val Gln Trp Leu Leu Asp Gly Gly Pro Ser
            20                  25                  30

Ser Gly Ala Pro Pro Pro Ser
        35

<210>    26
<211>    39
<212>    PRT
<213>    Artificial Sequence

<220>
<223>    Trigonal agonist

<220>
<221>    MISC_FEATURE
<222>    (2)
<223>    Xaa is aminoisobutyric acid (Aib)

<400>    26
His Xaa Gln Gly Thr Phe Thr Ser Asp Cys Ser Lys Tyr Leu Asp Ser
1               5                   10                  15

Arg Ala Ala Gln Asp Phe Val Gln Trp Leu Leu Asp Gly Gly Pro Ser
            20                  25                  30

Ser Gly Ala Pro Pro Pro Ser
        35

<210>    27
<211>    40
<212>    PRT
<213>    Artificial Sequence

<220>
<223>    Trigonal agonist


<220>
<221>    MISC_FEATURE
<222>    (2)
<223>    Xaa is aminoisobutyric acid (Aib)


<400>    27
His Xaa Gln Gly Thr Phe Thr Ser Asp Tyr Ser Lys Tyr Leu Asp Glu
1               5                   10                  15

Arg Ala Cys Gln Asp Phe Val Gln Trp Leu Leu Asp Gln Gly Gly Pro
            20                  25                  30

Ser Ser Gly Ala Pro Pro Pro Ser
        35                  40


<210>    28
<211>    41
<212>    PRT
<213>    Artificial Sequence

<220>
<223>    Trigonal agonist


<220>
<221>    MISC_FEATURE
<222>    (2)
<223>    Xaa is aminoisobutyric acid (Aib)


<400>    28
His Xaa Gln Gly Thr Phe Thr Ser Asp Tyr Ser Lys Tyr Leu Asp Glu
1               5                   10                  15

Lys Arg Ala Gln Glu Phe Val Cys Trp Leu Leu Ala Gln Lys Gly Lys
            20                  25                  30

Lys Asn Asp Trp Lys His Asn Ile Thr
        35                  40


<210>    29
<211>    30
<212>    PRT
<213>    Artificial Sequence

<220>
<223>    Trigonal agonist


<220>
<221>    MISC_FEATURE

<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    29
His Xaa Gln Gly Thr Phe Thr Ser Asp Tyr Ser Lys Tyr Leu Asp Glu
1               5                   10                  15

Lys Ala Ala Lys Glu Phe Val Gln Trp Leu Leu Asn Thr Cys
            20                  25                  30


<210>    30
<211>    30
<212>    PRT
<213>    Artificial Sequence

<220>
<223>    Trigonal agonist


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    30
His Xaa Gln Gly Thr Phe Thr Ser Asp Tyr Ser Lys Tyr Leu Asp Glu
1               5                   10                  15

Lys Ala Gln Lys Glu Phe Val Gln Trp Leu Leu Asp Thr Cys
            20                  25                  30


<210>    31
<211>    29
<212>    PRT
<213>    Artificial Sequence

<220>
<223>    Trigonal agonist


<220>
<221>    MISC_FEATURE
<222>    (2)
<223>    Xaa is aminoisobutyric acid (Aib)


<220>

<221>    MISC_FEATURE
<222>    (16)..(20)
<223>    amino acids at positions 16 and 20 form a ring

<400>    31
His Xaa Gln Gly Thr Phe Thr Ser Asp Tyr Ser Lys Tyr Leu Asp Glu
1               5                   10                  15

Lys Ala Cys Lys Glu Phe Val Gln Trp Leu Leu Ala Gln
            20                  25

<210>    32
<211>    39
<212>    PRT
<213>    Artificial Sequence

<220>
<223>    Trigonal agonist

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    32
His Xaa Gln Gly Thr Phe Thr Ser Asp Tyr Ser Lys Tyr Leu Asp Glu
1               5                   10                  15

Lys Ala Cys Lys Asp Phe Val Gln Trp Leu Leu Asp Gly Gly Pro Ser
            20                  25                  30

Ser Gly Ala Pro Pro Pro Ser
        35

<210>    33
<211>    31
<212>    PRT
<213>    Artificial Sequence

<220>
<223>    Trigonal agonist

<220>
<221>    MISC_FEATURE
<222>    (2)
<223>    Xaa is aminoisobutyric acid (Aib)

<220>
<221>    MISC_FEATURE
<222>    (16)..(20)

<223>    amino acids at positions 16 and 20 form a ring

<400>    33
His Xaa Gln Gly Thr Phe Thr Ser Asp Tyr Ser Ile Ala Met Asp Glu
1               5                   10                  15

Ile His Gln Lys Asp Phe Val Asn Trp Leu Leu Ala Gln Lys Cys
            20                  25                  30

<210>    34
<211>    31
<212>    PRT
<213>    Artificial Sequence

<220>
<223>    Trigonal agonist

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    34
His Xaa Gln Gly Thr Phe Thr Ser Asp Tyr Ser Lys Tyr Leu Asp Glu
1               5                   10                  15

Lys Arg Gln Lys Glu Phe Val Asn Trp Leu Leu Ala Gln Lys Cys
            20                  25                  30

<210>    35
<211>    31
<212>    PRT
<213>    Artificial Sequence

<220>
<223>    Trigonal agonist

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    35
His Xaa Gln Gly Thr Phe Thr Ser Asp Tyr Ser Ile Ala Met Asp Glu
1               5                   10                  15

Ile His Gln Lys Asp Phe Val Asn Trp Leu Leu Asn Thr Lys Cys
            20                  25                  30

<210>    36
<211>    40
<212>    PRT
<213>    Artificial Sequence

<220>
<223>    Trigonal agonist

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    36
His Xaa Gln Gly Thr Phe Thr Ser Asp Tyr Ser Lys Tyr Leu Cys Glu
1               5                   10                  15

Lys Arg Gln Lys Glu Phe Val Gln Trp Leu Leu Asn Gly Gly Pro Ser
            20                  25                  30

Ser Gly Ala Pro Pro Pro Ser Gly
        35                  40

<210>    37
<211>    40
<212>    PRT
<213>    Artificial Sequence

<220>
<223>    Trigonal agonist

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    37

His Xaa Gln Gly Thr Phe Thr Ser Asp Tyr Ser Lys Tyr Leu Asp Glu
1               5                   10                  15

Cys Arg Gln Lys Glu Phe Val Gln Trp Leu Leu Asn Gly Gly Pro Ser
            20                  25                  30

Ser Gly Ala Pro Pro Pro Ser Gly
        35                  40

<210>   38
<211>   40
<212>   PRT
<213>   Artificial Sequence

<220>
<223>   Trigonal agonist

<220>
<221>   MISC_FEATURE
<222>   (1)
<223>   Xaa is 4-imidazoacetyl (CA)

<220>
<221>   MISC_FEATURE
<222>   (2)
<223>   Xaa is aminoisobutyric acid (Aib)

<400>   38
Xaa Xaa Gln Gly Thr Phe Thr Ser Asp Lys Ser Ser Tyr Leu Asp Glu
1               5                   10                  15

Arg Ala Ala Gln Asp Phe Val Gln Trp Leu Leu Asp Gly Gly Pro Ser
            20                  25                  30

Ser Gly Ala Pro Pro Pro Ser Ser
        35                  40

<210>   39
<211>   40
<212>   PRT
<213>   Artificial Sequence

<220>
<223>   Trigonal agonist

<220>
<221>   MISC_FEATURE
<222>   (2)
<223>   Xaa is aminoisobutyric acid (Aib)

<400>   39
His Xaa Gln Gly Thr Phe Thr Ser Asp Tyr Ser Lys Tyr Leu Asp Gly
1               5                   10                  15

Gln His Ala Gln Cys Phe Val Ala Trp Leu Leu Ala Gly Gly Gly Pro
            20                  25                  30

Ser Ser Gly Ala Pro Pro Pro Ser
        35                  40

<210>   40
<211>   39
<212>   PRT
<213>   Artificial Sequence

<220>
<223>   Trigonal agonist

<220>
<221>   MISC_FEATURE
<222>   (2)
<223>   Xaa is aminoisobutyric acid (Aib)

<400>   40
His Xaa Gln Gly Thr Phe Thr Ser Asp Lys Ser Lys Tyr Leu Asp Glu
 1               5                  10                  15

Arg Ala Cys Gln Asp Phe Val Gln Trp Leu Leu Asp Gly Gly Pro Ser
            20                  25                  30

Ser Gly Ala Pro Pro Pro Ser
        35

<210>   41
<211>   39
<212>   PRT
<213>   Artificial Sequence

<220>
<223>   Trigonal agonist

<220>
<221>   MISC_FEATURE
<222>   (2)
<223>   Xaa is aminoisobutyric acid (Aib)

<400>   41
His Xaa Gln Gly Thr Phe Thr Ser Asp Lys Ser Lys Tyr Leu Asp Glu
 1               5                  10                  15

Cys Ala Ala Gln Asp Phe Val Gln Trp Leu Leu Asp Gly Gly Pro Ser
            20                  25                  30

Ser Gly Ala Pro Pro Pro Ser
        35

<210>   42
<211>   40
<212>   PRT
<213>   Artificial Sequence

<220>

<223>    Trigonal agonist

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    42
Tyr Xaa Gln Gly Thr Phe Thr Ser Asp Tyr Ser Lys Tyr Leu Asp Glu
1               5                   10                  15

Lys Arg Ala Lys Glu Phe Val Gln Trp Leu Leu Asp His His Pro Ser
            20                  25                  30

Ser Gly Gln Pro Pro Pro Ser Cys
        35                  40

<210>    43
<211>    39
<212>    PRT
<213>    Artificial Sequence

<220>
<223>    Trigonal agonist

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    43
Tyr Xaa Gln Gly Thr Phe Thr Ser Asp Tyr Ser Lys Tyr Leu Asp Glu
1               5                   10                  15

Lys Arg Ala Lys Glu Phe Val Gln Trp Leu Leu Asp His His Cys Ser
            20                  25                  30

Ser Gly Gln Pro Pro Pro Ser
        35

<210>    44
<211>    39
<212>    PRT
<213>    Artificial Sequence

<220>
<223>    Trigonal agonist


<400>    44
His Gly Gln Gly Thr Phe Thr Ser Asp Cys Ser Lys Gln Leu Asp Gly
1               5                   10                  15

Gln Ala Ala Gln Glu Phe Val Ala Trp Leu Leu Ala Gly Gly Pro Ser
            20                  25                  30

Ser Gly Ala Pro Pro Pro Ser
        35


<210>    45
<211>    39
<212>    PRT
<213>    Artificial Sequence

<220>
<223>    Trigonal agonist


<400>    45
His Gly Gln Gly Thr Phe Thr Ser Asp Cys Ser Lys Tyr Met Asp Gly
1               5                   10                  15

Gln Ala Ala Gln Asp Phe Val Ala Trp Leu Leu Ala Gly Gly Pro Ser
            20                  25                  30

Ser Gly Ala Pro Pro Pro Ser
        35


<210>    46
<211>    39
<212>    PRT
<213>    Artificial Sequence

<220>
<223>    Trigonal agonist


<400>    46
His Gly Gln Gly Thr Phe Thr Ser Asp Cys Ser Lys Tyr Leu Asp Glu
1               5                   10                  15

Gln His Ala Gln Glu Phe Val Ala Trp Leu Leu Ala Gly Gly Pro Ser
            20                  25                  30

Ser Gly Ala Pro Pro Pro Ser
        35


<210>    47
<211>    39
<212>    PRT
<213>    Artificial Sequence

<220>
<223>    Trigonal agonist

<400>    47
His Gly Gln Gly Thr Phe Thr Ser Asp Cys Ser Lys Tyr Leu Asp Gly
1               5                   10                  15

Gln Arg Ala Gln Glu Phe Val Ala Trp Leu Leu Ala Gly Gly Pro Ser
            20                  25                  30

Ser Gly Ala Pro Pro Pro Ser
        35


<210>    48
<211>    39
<212>    PRT
<213>    Artificial Sequence

<220>
<223>    Trigonal agonist


<400>    48
His Gly Gln Gly Thr Phe Thr Ser Asp Cys Ser Lys Tyr Leu Asp Gly
1               5                   10                  15

Gln Arg Ala Gln Asp Phe Val Asn Trp Leu Leu Ala Gly Gly Pro Ser
            20                  25                  30

Ser Gly Ala Pro Pro Pro Ser
        35


<210>    49
<211>    30
<212>    PRT
<213>    Artificial Sequence

<220>
<223>    Trigonal agonist


<220>
<221>    MISC_FEATURE
<222>    (1)
<223>    Xaa is 4-imidazoacetyl (CA)


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    49
Xaa Xaa Gln Gly Thr Phe Thr Ser Asp Tyr Ser Ile Cys Met Asp Glu
1               5                   10                  15

Ile His Gln Lys Asp Phe Val Asn Trp Leu Leu Asn Thr Lys
            20                  25                  30

<210>   50
<211>   40
<212>   PRT
<213>   Artificial Sequence

<220>
<223>   Trigonal agonist

<220>
<221>   MISC_FEATURE
<222>   (2)
<223>   Xaa is aminoisobutyric acid (Aib)

<220>
<221>   MISC_FEATURE
<222>   (16)..(20)
<223>   amino acids at positions 16 and 20 form a ring

<400>   50
His Xaa Gln Gly Thr Phe Thr Ser Asp Tyr Ser Lys Tyr Leu Asp Glu
1               5                   10                  15

Lys Arg Ala Lys Glu Phe Val Gln Trp Leu Leu Asp His His Pro Ser
            20                  25                  30

Ser Gly Gln Pro Pro Pro Ser Cys
        35                  40

<210>   51
<211>   30
<212>   PRT
<213>   Artificial Sequence

<220>
<223>   Trigonal agonist

<220>
<221>   MISC_FEATURE
<222>   (2)
<223>   Xaa is aminoisobutyric acid (Aib)

<220>
<221>   MISC_FEATURE
<222>   (16)..(20)
<223>   amino acids at positions 16 and 20 form a ring

<400>   51
His Xaa Gln Gly Thr Phe Thr Ser Asp Tyr Ser Lys Tyr Leu Asp Glu
1               5                   10                  15

Lys Arg Gln Lys Glu Phe Val Gln Trp Leu Leu Asn Thr Cys
            20                  25                  30

<210>     52
<211>     30
<212>     PRT
<213>     Artificial Sequence

<220>
<223>     Trigonal agonist


<220>
<221>     MISC_FEATURE
<222>     (2)
<223>     Xaa is aminoisobutyric acid (Aib)


<220>
<221>     MISC_FEATURE
<222>     (16)..(20)
<223>     amino acids at positions 16 and 20 form a ring


<400>     52
His Xaa Gln Gly Thr Phe Thr Ser Asp Tyr Ser Lys Tyr Leu Asp Glu
1               5                   10                  15

Lys Arg Gln Lys Glu Phe Val Gln Trp Leu Leu Asp Thr Cys
            20                  25                  30


<210>     53
<211>     30
<212>     PRT
<213>     Artificial Sequence

<220>
<223>     Trigonal agonist


<220>
<221>     MISC_FEATURE
<222>     (2)
<223>     Xaa is aminoisobutyric acid (Aib)


<220>
<221>     MISC_FEATURE
<222>     (16)..(20)
<223>     amino acids at positions 16 and 20 form a ring


<400>     53
His Xaa Glu Gly Thr Phe Thr Ser Asp Tyr Ser Ile Ala Met Asp Glu
1               5                   10                  15

Ile His Gln Lys Asp Phe Val Asn Trp Leu Leu Ala Gln Cys
            20                  25                  30


<210>     54
<211>     30
<212>     PRT

<213>    Artificial Sequence

<220>
<223>    Trigonal agonist


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    54
His Xaa Glu Gly Thr Phe Thr Ser Asp Tyr Ser Ile Ala Met Asp Glu
1               5                   10                  15

Ile His Gln Lys Asp Phe Val Asp Trp Leu Leu Ala Glu Cys
            20                  25                  30


<210>    55
<211>    30
<212>    PRT
<213>    Artificial Sequence

<220>
<223>    Trigonal agonist


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    55
His Xaa Gln Gly Thr Phe Thr Ser Asp Tyr Ser Ile Ala Met Asp Glu
1               5                   10                  15

Ile His Gln Lys Asp Phe Val Asn Trp Leu Leu Ala Gln Cys
            20                  25                  30


<210>    56
<211>    30
<212>    PRT
<213>    Artificial Sequence

<220>
<223>    Trigonal agonist

<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    56
His Xaa Gln Gly Thr Phe Thr Ser Asp Tyr Ser Lys Tyr Leu Asp Glu
1               5                   10                  15

Lys Arg Gln Lys Glu Phe Val Asn Trp Leu Leu Ala Gln Cys
            20                  25                  30


<210>    57
<211>    30
<212>    PRT
<213>    Artificial Sequence

<220>
<223>    Trigonal agonist


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    57
His Xaa Gln Gly Thr Phe Thr Ser Asp Tyr Ser Ile Ala Met Asp Glu
1               5                   10                  15

Ile His Gln Lys Asp Phe Val Asn Trp Leu Leu Asn Thr Cys
            20                  25                  30


<210>    58
<211>    31
<212>    PRT
<213>    Artificial Sequence

<220>
<223>    Trigonal agonist


<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    58
His Xaa Gln Gly Thr Phe Thr Ser Asp Tyr Ser Lys Tyr Leu Asp Glu
1               5                   10                  15

Lys Arg Gln Lys Glu Phe Val Gln Trp Leu Leu Asn Thr Lys Cys
            20                  25                  30

<210>    59
<211>    30
<212>    PRT
<213>    Artificial Sequence

<220>
<223>    Trigonal agonist

<220>
<221>    MISC_FEATURE
<222>    (1)
<223>    Xaa is 4-imidazoacetyl (CA)

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    59
Xaa Xaa Gln Gly Thr Phe Thr Ser Asp Tyr Ser Ile Cys Met Asp Glu
1               5                   10                  15

Lys His Gln Lys Asp Phe Val Asn Trp Leu Leu Asn Thr Lys
            20                  25                  30

<210>    60
<211>    30
<212>    PRT
<213>    Artificial Sequence

<220>
<223>    Trigonal agonist

<220>
<221>    MISC_FEATURE

<222>     (1)
<223>     Xaa is 4-imidazoacetyl (CA)

<220>
<221>     MISC_FEATURE
<222>     (2)
<223>     Xaa is aminoisobutyric acid (Aib)

<220>
<221>     MISC_FEATURE
<222>     (16)..(20)
<223>     amino acids at positions 16 and 20 form a ring

<400>     60
Xaa Xaa Gln Gly Thr Phe Thr Ser Asp Tyr Ser Ile Ala Met Asp Glu
1               5                   10                  15

Lys His Cys Lys Asp Phe Val Asn Trp Leu Leu Asn Thr Lys
            20                  25                  30

<210>     61
<211>     30
<212>     PRT
<213>     Artificial Sequence

<220>
<223>     Trigonal agonist

<220>
<221>     MISC_FEATURE
<222>     (1)
<223>     Xaa is 4-imidazoacetyl (CA)

<220>
<221>     MISC_FEATURE
<222>     (2)
<223>     Xaa is aminoisobutyric acid (Aib)

<220>
<221>     MISC_FEATURE
<222>     (16)..(20)
<223>     amino acids at positions 16 and 20 form a ring

<400>     61
Xaa Xaa Gln Gly Thr Phe Thr Ser Asp Tyr Ser Ile Ala Met Asp Glu
1               5                   10                  15

Ile Ala Cys Lys Asp Phe Val Asn Trp Leu Leu Asn Thr Lys
            20                  25                  30

<210>     62
<211>     39
<212>     PRT
<213>     Artificial Sequence

<220>
<223>    Trigonal agonist

<220>
<221>    MISC_FEATURE
<222>    (1)
<223>    Xaa is 4-imidazoacetyl (CA)

<220>
<221>    MISC_FEATURE
<222>    (2)
<223>    Xaa is aminoisobutyric acid (Aib)

<400>    62
Xaa Xaa Gln Gly Thr Phe Thr Ser Asp Lys Ser Lys Tyr Leu Asp Glu
1               5                   10                  15

Arg Ala Ala Gln Asp Phe Val Gln Trp Leu Leu Asp Gly Gly Pro Ser
            20                  25                  30

Ser Gly Ala Pro Pro Pro Ser
        35

<210>    63
<211>    39
<212>    PRT
<213>    Artificial Sequence

<220>
<223>    Trigonal agonist

<220>
<221>    MISC_FEATURE
<222>    (1)
<223>    Xaa is 4-imidazoacetyl (CA)

<220>
<221>    MISC_FEATURE
<222>    (2)
<223>    Xaa is aminoisobutyric acid (Aib)

<400>    63
Xaa Xaa Gln Gly Thr Phe Thr Ser Asp Cys Ser Lys Tyr Leu Asp Glu
1               5                   10                  15

Arg Ala Ala Gln Asp Phe Val Gln Trp Leu Leu Asp Gly Gly Pro Ser
            20                  25                  30

Ser Gly Ala Pro Pro Pro Ser
        35

<210>    64
<211>    39
<212>    PRT

<213>    Artificial Sequence

<220>
<223>    Trigonal agonist


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    64
Tyr Xaa Gln Gly Thr Phe Thr Ser Asp Tyr Ser Lys Tyr Leu Asp Glu
1               5                   10                  15

Cys Ala Ala Lys Glu Phe Val Gln Trp Leu Leu Asp His His Pro Ser
            20                  25                  30

Ser Gly Gln Pro Pro Pro Ser
        35


<210>    65
<211>    39
<212>    PRT
<213>    Artificial Sequence

<220>
<223>    Trigonal agonist


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    65
His Xaa Gln Gly Thr Phe Thr Ser Asp Tyr Ser Lys Cys Leu Asp Glu
1               5                   10                  15

Lys Arg Ala Lys Glu Phe Val Gln Trp Leu Leu Asp His His Pro Ser
            20                  25                  30

Ser Gly Gln Pro Pro Pro Ser
        35


<210>    66
<211>    39

<212>    PRT
<213>    Artificial Sequence

<220>
<223>    Trigonal agonist


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    66
Tyr Xaa Gln Gly Thr Phe Thr Ser Asp Tyr Ser Lys Tyr Leu Asp Glu
1               5                   10                  15

Cys Arg Ala Lys Asp Phe Val Gln Trp Leu Leu Asp His His Pro Ser
            20                  25                  30

Ser Gly Gln Pro Pro Pro Ser
        35


<210>    67
<211>    39
<212>    PRT
<213>    Artificial Sequence

<220>
<223>    Trigonal agonist


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    67
Tyr Xaa Gln Gly Thr Phe Thr Ser Asp Tyr Ser Lys Tyr Leu Asp Glu
1               5                   10                  15

Cys Ala Ala Lys Asp Phe Val Gln Trp Leu Leu Asp His His Pro Ser
            20                  25                  30

Ser Gly Gln Pro Pro Pro Ser
        35


<210>    68

<211>    39
<212>    PRT
<213>    Artificial Sequence

<220>
<223>    Trigonal agonist


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    68
Tyr Xaa Gln Gly Thr Phe Thr Ser Asp Tyr Ser Lys Cys Leu Asp Glu
1               5                   10                  15

Lys Ala Ala Lys Glu Phe Val Gln Trp Leu Leu Asp His His Pro Ser
            20                  25                  30

Ser Gly Gln Pro Pro Pro Ser
        35


<210>    69
<211>    39
<212>    PRT
<213>    Artificial Sequence

<220>
<223>    Trigonal agonist


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    69
Tyr Xaa Gln Gly Thr Phe Thr Ser Asp Tyr Ser Lys Cys Leu Asp Glu
1               5                   10                  15

Arg Ala Ala Lys Glu Phe Val Gln Trp Leu Leu Asp His His Pro Ser
            20                  25                  30

Ser Gly Gln Pro Pro Pro Ser
        35

<210>    70
<211>    39
<212>    PRT
<213>    Artificial Sequence

<220>
<223>    Trigonal agonist


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    70
Tyr Xaa Gln Gly Thr Phe Thr Ser Asp Tyr Ser Lys Cys Leu Asp Glu
1               5                   10                  15

Lys Arg Ala Lys Asp Phe Val Gln Trp Leu Leu Asp His His Pro Ser
            20                  25                  30

Ser Gly Gln Pro Pro Pro Ser
        35


<210>    71
<211>    39
<212>    PRT
<213>    Artificial Sequence

<220>
<223>    Trigonal agonist


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    71
Tyr Xaa Gln Gly Thr Phe Thr Ser Asp Tyr Ser Lys Tyr Leu Asp Glu
1               5                   10                  15

Arg Ala Cys Lys Asp Phe Val Gln Trp Leu Leu Asp His His Pro Ser
            20                  25                  30

Ser Gly Gln Pro Pro Pro Ser
        35

<210>    72
<211>    39
<212>    PRT
<213>    Artificial Sequence

<220>
<223>    Trigonal agonist


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    72
Tyr Xaa Gln Gly Thr Phe Thr Ser Asp Cys Ser Lys Tyr Leu Asp Glu
1               5                   10                  15

Arg Ala Ala Lys Asp Phe Val Gln Trp Leu Leu Asp His His Pro Ser
            20                  25                  30

Ser Gly Gln Pro Pro Pro Ser
        35


<210>    73
<211>    39
<212>    PRT
<213>    Artificial Sequence

<220>
<223>    Trigonal agonist


<220>
<221>    MISC_FEATURE
<222>    (1)
<223>    Xaa is 4-imidazoacetyl (CA)


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    73
Xaa Xaa Gln Gly Thr Phe Thr Ser Asp Tyr Ser Lys Tyr Leu Asp Glu
1               5                   10                  15

Cys Arg Ala Lys Glu Phe Val Gln Trp Leu Leu Asp His His Pro Ser
            20                  25                  30

Ser Gly Gln Pro Pro Pro Ser
        35

<210>   74
<211>   39
<212>   PRT
<213>   Artificial Sequence

<220>
<223>   Trigonal agonist

<220>
<221>   MISC_FEATURE
<222>   (1)
<223>   Xaa is 4-imidazoacetyl (CA)

<220>
<221>   MISC_FEATURE
<222>   (2)
<223>   Xaa is aminoisobutyric acid (Aib)

<220>
<221>   MISC_FEATURE
<222>   (16)..(20)
<223>   amino acids at positions 16 and 20 form a ring

<400>   74
Xaa Xaa Gln Gly Thr Phe Thr Ser Asp Tyr Ser Lys Cys Leu Asp Glu
 1               5                  10                  15

Lys Arg Ala Lys Glu Phe Val Gln Trp Leu Leu Asp His His Pro Ser
            20                  25                  30

Ser Gly Gln Pro Pro Pro Ser
        35

<210>   75
<211>   40
<212>   PRT
<213>   Artificial Sequence

<220>
<223>   Trigonal agonist

<220>
<221>   MISC_FEATURE
<222>   (2)
<223>   Xaa is aminoisobutyric acid (Aib)

<220>
<221>   MISC_FEATURE

<222>    (16)..(20)
<223>    amino acids at positions 16 and 20 form a ring


<400>    75
Tyr Xaa Gln Gly Thr Phe Thr Ser Asp Tyr Ser Lys Tyr Leu Asp Glu
1               5                   10                  15

Lys Ala Ala Lys Glu Phe Val Gln Trp Leu Leu Asp His His Pro Ser
            20                  25                  30

Ser Gly Gln Pro Pro Pro Ser Cys
        35                  40


<210>    76
<211>    40
<212>    PRT
<213>    Artificial Sequence

<220>
<223>    Trigonal agonist


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    76
Tyr Xaa Gln Gly Thr Phe Thr Ser Asp Tyr Ser Lys Tyr Leu Asp Glu
1               5                   10                  15

Lys Arg Ala Lys Asp Phe Val Gln Trp Leu Leu Asp His His Pro Ser
            20                  25                  30

Ser Gly Gln Pro Pro Pro Ser Cys
        35                  40


<210>    77
<211>    40
<212>    PRT
<213>    Artificial Sequence

<220>
<223>    Trigonal agonist


<220>
<221>    MISC_FEATURE
<222>    (2)
<223>    Xaa is aminoisobutyric acid (Aib)


<220>

<221>    MISC_FEATURE
<222>    (16)..(20)
<223>    amino acids at positions 16 and 20 form a ring


<400>    77
Tyr Xaa Gln Gly Thr Phe Thr Ser Asp Tyr Ser Lys Tyr Leu Asp Glu
1               5                   10                  15

Lys Ala Ala Lys Asp Phe Val Gln Trp Leu Leu Asp His His Pro Ser
            20                  25                  30

Ser Gly Gln Pro Pro Pro Ser Cys
        35                  40


<210>    78
<211>    31
<212>    PRT
<213>    Artificial Sequence

<220>
<223>    Trigonal agonist


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    78
His Xaa Gln Gly Thr Phe Thr Ser Asp Tyr Ser Lys Tyr Leu Asp Glu
1               5                   10                  15

Lys Arg Gln Lys Glu Phe Val Gln Trp Leu Leu Asp Thr Lys Cys
            20                  25                  30


<210>    79
<211>    31
<212>    PRT
<213>    Artificial Sequence

<220>
<223>    Trigonal agonist


<220>
<221>    MISC_FEATURE
<222>    (2)
<223>    Xaa is aminoisobutyric acid (Aib)


<220>
<221>    MISC_FEATURE

<222>    (16)..(20)
<223>    amino acids at positions 16 and 20 form a ring

<400>    79
His Xaa Glu Gly Thr Phe Thr Ser Asp Tyr Ser Ile Ala Met Asp Glu
1               5                   10                  15

Ile His Gln Lys Asp Phe Val Asn Trp Leu Leu Ala Gln Lys Cys
            20                  25                  30

<210>    80
<211>    31
<212>    PRT
<213>    Artificial Sequence

<220>
<223>    Trigonal agonist

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    80
His Xaa Glu Gly Thr Phe Thr Ser Asp Tyr Ser Ile Ala Met Asp Glu
1               5                   10                  15

Ile His Gln Lys Asp Phe Val Asp Trp Leu Leu Ala Glu Lys Cys
            20                  25                  30

<210>    81
<211>    30
<212>    PRT
<213>    Artificial Sequence

<220>
<223>    Trigonal agonist

<220>
<221>    MISC_FEATURE
<222>    (1)
<223>    Xaa is 4-imidazoacetyl (CA)

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    81
Xaa Xaa Gln Gly Thr Phe Thr Ser Asp Tyr Ser Lys Tyr Leu Asp Glu
1               5                   10                  15

Lys Arg Gln Lys Glu Phe Val Gln Trp Leu Leu Asn Thr Cys
            20                  25                  30

<210>    82
<211>    30
<212>    PRT
<213>    Artificial Sequence

<220>
<223>    Trigonal agonist

<220>
<221>    MISC_FEATURE
<222>    (1)
<223>    Xaa is 4-imidazoacetyl (CA)

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    82
Xaa Xaa Gln Gly Thr Phe Thr Ser Asp Tyr Ser Lys Tyr Leu Asp Glu
1               5                   10                  15

Lys Arg Gln Lys Glu Phe Val Gln Trp Leu Leu Asp Thr Cys
            20                  25                  30

<210>    83
<211>    30
<212>    PRT
<213>    Artificial Sequence

<220>
<223>    Trigonal agonist

<220>
<221>    MISC_FEATURE
<222>    (1)
<223>    Xaa is 4-imidazoacetyl (CA)

<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    83
Xaa Xaa Glu Gly Thr Phe Thr Ser Asp Tyr Ser Ile Ala Met Asp Glu
1               5                   10                  15

Ile His Gln Lys Asp Phe Val Asn Trp Leu Leu Ala Gln Cys
            20                  25                  30


<210>    84
<211>    30
<212>    PRT
<213>    Artificial Sequence

<220>
<223>    Trigonal agonist


<220>
<221>    MISC_FEATURE
<222>    (1)
<223>    Xaa is 4-imidazoacetyl (CA)


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    84
Xaa Xaa Glu Gly Thr Phe Thr Ser Asp Tyr Ser Ile Ala Met Asp Glu
1               5                   10                  15

Ile His Gln Lys Asp Phe Val Asp Trp Leu Leu Ala Glu Cys
            20                  25                  30


<210>    85
<211>    30
<212>    PRT
<213>    Artificial Sequence

<220>

<223>    Trigonal agonist

<220>
<221>    MISC_FEATURE
<222>    (1)
<223>    Xaa is 4-imidazoacetyl (CA)

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    85
Xaa Xaa Gln Gly Thr Phe Thr Ser Asp Tyr Ser Ile Ala Met Asp Glu
1               5                   10                  15

Ile His Gln Lys Asp Phe Val Asn Trp Leu Leu Ala Gln Cys
            20                  25                  30

<210>    86
<211>    30
<212>    PRT
<213>    Artificial Sequence

<220>
<223>    Trigonal agonist

<220>
<221>    MISC_FEATURE
<222>    (1)
<223>    Xaa is 4-imidazoacetyl (CA)

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    86
Xaa Xaa Gln Gly Thr Phe Thr Ser Asp Tyr Ser Lys Tyr Leu Asp Glu
1               5                   10                  15

Lys Arg Gln Lys Glu Phe Val Asn Trp Leu Leu Ala Gln Cys
            20                  25                  30

<210>	87
<211>	30
<212>	PRT
<213>	Artificial Sequence

<220>
<223>	Trigonal agonist


<220>
<221>	MISC_FEATURE
<222>	(1)
<223>	Xaa is 4-imidazoacetyl (CA)


<220>
<221>	MISC_FEATURE
<222>	(2)
<223>	Xaa is aminoisobutyric acid (Aib)


<220>
<221>	MISC_FEATURE
<222>	(16)..(20)
<223>	amino acids at positions 16 and 20 form a ring


<400>	87
Xaa Xaa Gln Gly Thr Phe Thr Ser Asp Tyr Ser Ile Ala Met Asp Glu
1               5                   10                  15

Ile His Gln Lys Asp Phe Val Asn Trp Leu Leu Asn Thr Cys
            20                  25                  30


<210>	88
<211>	31
<212>	PRT
<213>	Artificial Sequence

<220>
<223>	Trigonal agonist


<220>
<221>	MISC_FEATURE
<222>	(1)
<223>	Xaa is 4-imidazoacetyl (CA)


<220>
<221>	MISC_FEATURE
<222>	(2)
<223>	Xaa is aminoisobutyric acid (Aib)


<220>
<221>	MISC_FEATURE
<222>	(16)..(20)
<223>	amino acids at positions 16 and 20 form a ring

<400>    88
Xaa Xaa Gln Gly Thr Phe Thr Ser Asp Tyr Ser Lys Tyr Leu Asp Glu
1               5                   10                  15

Lys Arg Gln Lys Glu Phe Val Gln Trp Leu Leu Asn Thr Lys Cys
            20                  25                  30

<210>    89
<211>    31
<212>    PRT
<213>    Artificial Sequence

<220>
<223>    Trigonal agonist

<220>
<221>    MISC_FEATURE
<222>    (1)
<223>    Xaa is 4-imidazoacetyl (CA)

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    89
Xaa Xaa Gln Gly Thr Phe Thr Ser Asp Tyr Ser Lys Tyr Leu Asp Glu
1               5                   10                  15

Lys Arg Gln Lys Glu Phe Val Gln Trp Leu Leu Asp Thr Lys Cys
            20                  25                  30

<210>    90
<211>    31
<212>    PRT
<213>    Artificial Sequence

<220>
<223>    Trigonal agonist

<220>
<221>    MISC_FEATURE
<222>    (1)
<223>    Xaa is 4-imidazoacetyl (CA)

<220>
<221>    MISC_FEATURE
<222>    (2)

<223>    Xaa is aminoisobutyric acid (Aib)

<220>
<221>    MISC_FEATURE
<222>    (16)..(20)
<223>    amino acids at positions 16 and 20 form a ring

<400>    90
Xaa Xaa Glu Gly Thr Phe Thr Ser Asp Tyr Ser Ile Ala Met Asp Glu
1               5                   10                  15

Ile His Gln Lys Asp Phe Val Asn Trp Leu Leu Ala Gln Lys Cys
            20                  25                  30

<210>    91
<211>    31
<212>    PRT
<213>    Artificial Sequence

<220>
<223>    Trigonal agonist

<220>
<221>    MISC_FEATURE
<222>    (1)
<223>    Xaa is 4-imidazoacetyl (CA)

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    91
Xaa Xaa Glu Gly Thr Phe Thr Ser Asp Tyr Ser Ile Ala Met Asp Glu
1               5                   10                  15

Ile His Gln Lys Asp Phe Val Asp Trp Leu Leu Ala Glu Lys Cys
            20                  25                  30

<210>    92
<211>    31
<212>    PRT
<213>    Artificial Sequence

<220>
<223>    Trigonal agonist

<220>
<221>    MISC_FEATURE
<222>    (1)
<223>    Xaa is 4-imidazoacetyl (CA)


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    92
Xaa Xaa Gln Gly Thr Phe Thr Ser Asp Tyr Ser Ile Ala Met Asp Glu
1               5                   10                  15

Ile His Gln Lys Asp Phe Val Asn Trp Leu Leu Ala Gln Lys Cys
            20                  25                  30



<210>    93
<211>    31
<212>    PRT
<213>    Artificial Sequence

<220>
<223>    Trigonal agonist


<220>
<221>    MISC_FEATURE
<222>    (1)
<223>    Xaa is 4-imidazoacetyl (CA)


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    93
Xaa Xaa Gln Gly Thr Phe Thr Ser Asp Tyr Ser Lys Tyr Leu Asp Glu
1               5                   10                  15

Lys Arg Gln Lys Glu Phe Val Asn Trp Leu Leu Ala Gln Lys Cys
            20                  25                  30

<210>    94
<211>    31
<212>    PRT
<213>    Artificial Sequence

<220>
<223>    Trigonal agonist


<220>
<221>    MISC_FEATURE
<222>    (1)
<223>    Xaa is 4-imidazoacetyl (CA)


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    94
Xaa Xaa Gln Gly Thr Phe Thr Ser Asp Tyr Ser Ile Ala Met Asp Glu
1               5                   10                  15

Ile His Gln Lys Asp Phe Val Asn Trp Leu Leu Asn Thr Lys Cys
            20                  25                  30



<210>    95
<211>    39
<212>    PRT
<213>    Artificial Sequence

<220>
<223>    Trigonal agonist


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    95
Tyr Xaa Gln Gly Thr Phe Thr Ser Asp Tyr Ser Lys Tyr Leu Asp Glu
1               5                   10                  15

Lys Arg Ala Lys Glu Phe Val Gln Trp Leu Leu Cys His His Pro Ser
            20                  25                  30

Ser Gly Gln Pro Pro Pro Ser
        35

<210>   96
<211>   39
<212>   PRT
<213>   Artificial Sequence

<220>
<223>   Trigonal agonist

<220>
<221>   MISC_FEATURE
<222>   (2)
<223>   Xaa is aminoisobutyric acid (Aib)

<220>
<221>   MISC_FEATURE
<222>   (16)..(20)
<223>   amino acids at positions 16 and 20 form a ring

<400>   96
Tyr Xaa Gln Gly Thr Phe Thr Ser Asp Tyr Ser Lys Tyr Leu Asp Glu
1               5                   10                  15

Lys Arg Ala Lys Glu Phe Val Gln Trp Leu Leu Asp His Cys Pro Ser
            20                  25                  30

Ser Gly Gln Pro Pro Pro Ser
        35

<210>   97
<211>   39
<212>   PRT
<213>   Artificial Sequence

<220>
<223>   Trigonal agonist

<220>
<221>   MISC_FEATURE
<222>   (2)
<223>   Xaa is aminoisobutyric acid (Aib)

<220>
<221>   MISC_FEATURE
<222>   (16)..(20)
<223>   amino acids at positions 16 and 20 form a ring

<400>   97
Tyr Xaa Gln Gly Thr Phe Thr Ser Asp Tyr Ser Lys Tyr Leu Asp Glu
1               5                   10                  15

Lys Arg Ala Lys Glu Phe Val Gln Trp Leu Leu Asp Cys His Pro Ser

20                  25                  30

Ser Gly Gln Pro Pro Pro Ser
        35


<210>   98
<211>   40
<212>   PRT
<213>   Artificial Sequence

<220>
<223>   Trigonal agonist


<220>
<221>   MISC_FEATURE
<222>   (2)
<223>   Xaa is aminoisobutyric acid (Aib)


<220>
<221>   MISC_FEATURE
<222>   (16)..(20)
<223>   amino acids at positions 16 and 20 form a ring


<400>   98
Tyr Xaa Gln Gly Thr Phe Thr Ser Asp Tyr Ser Lys Ala Leu Asp Glu
 1               5                  10                  15

Lys Ala Ala Lys Glu Phe Val Asn Trp Leu Leu Asp His His Pro Ser
            20                  25                  30

Ser Gly Gln Pro Pro Pro Ser Cys
        35                  40


<210>   99
<211>   40
<212>   PRT
<213>   Artificial Sequence

<220>
<223>   Trigonal agonist


<220>
<221>   MISC_FEATURE
<222>   (2)
<223>   Xaa is aminoisobutyric acid (Aib)


<220>
<221>   MISC_FEATURE
<222>   (16)..(20)
<223>   amino acids at positions 16 and 20 form a ring


<400>   99
Tyr Xaa Gln Gly Thr Phe Thr Ser Asp Tyr Ser Lys Ala Leu Asp Glu
 1               5                  10                  15

Lys Ala Ala Lys Asp Phe Val Asn Trp Leu Leu Asp His His Pro Ser
            20                  25                  30

Ser Gly Gln Pro Pro Pro Ser Cys
        35                  40

<210>   100
<211>   40
<212>   PRT
<213>   Artificial Sequence

<220>
<223>   Trigonal agonist

<220>
<221>   MISC_FEATURE
<222>   (2)
<223>   Xaa is aminoisobutyric acid (Aib)

<220>
<221>   MISC_FEATURE
<222>   (16)..(20)
<223>   amino acids at positions 16 and 20 form a ring

<400>   100
Tyr Xaa Gln Gly Thr Phe Thr Ser Asp Tyr Ser Lys Ala Leu Asp Glu
 1               5                  10                  15

Lys Ala Ala Lys Glu Phe Val Gln Trp Leu Leu Asp Gln His Pro Ser
            20                  25                  30

Ser Gly Gln Pro Pro Pro Ser Cys
        35                  40

<210>   101
<211>   40
<212>   PRT
<213>   Artificial Sequence

<220>
<223>   Trigonal agonist

<220>
<221>   MISC_FEATURE
<222>   (2)
<223>   Xaa is aminoisobutyric acid (Aib)

<220>
<221>   MISC_FEATURE
<222>   (16)..(20)
<223>   amino acids at positions 16 and 20 form a ring

<400>   101
Tyr Xaa Gln Gly Thr Phe Thr Ser Asp Tyr Ser Lys Ala Leu Asp Glu
 1               5                  10                  15

Lys Ala Ala Lys Glu Phe Val Asn Trp Leu Leu Asp Gln His Pro Ser
            20                  25                  30

Ser Gly Gln Pro Pro Pro Ser Cys
        35                  40


<210>   102
<211>   40
<212>   PRT
<213>   Artificial Sequence

<220>
<223>   Trigonal agonist


<220>
<221>   MISC_FEATURE
<222>   (2)
<223>   Xaa is aminoisobutyric acid (Aib)


<220>
<221>   MISC_FEATURE
<222>   (16)..(20)
<223>   amino acids at positions 16 and 20 form a ring


<400>   102
Tyr Xaa Gln Gly Thr Phe Thr Ser Asp Tyr Ser Lys Ala Leu Asp Glu
 1               5                  10                  15

Lys Ala Ala Lys Asp Phe Val Asn Trp Leu Leu Asp Gln His Pro Ser
            20                  25                  30

Ser Gly Gln Pro Pro Pro Ser Cys
        35                  40


<210>   103
<211>   30
<212>   PRT
<213>   Artificial Sequence

<220>
<223>   General Formula 1


<220>
<221>   MISC_FEATURE
<222>   (1)
<223>   Xaa is His (H), 4-imidazoacetyl (CA), or Tyr (Y)


<220>
<221>   MISC_FEATURE
<222>   (2)
<223>   Xaa is Gly (G), alpha-methyl-glutamic acid, or Aib
        (aminoisobutyric acid)


<220>

<221>	MISC_FEATURE
<222>	(3)
<223>	Xaa is Glu (E) or Gln (Q)


<220>
<221>	MISC_FEATURE
<222>	(7)
<223>	Xaa is Thr (T) or Ile (I)


<220>
<221>	MISC_FEATURE
<222>	(10)
<223>	Xaa is Leu (L), Tyr (Y), Lys (K), Cys (C), or Val (V)


<220>
<221>	MISC_FEATURE
<222>	(12)
<223>	Xaa is Lys (K), Ser (S), or Ile (I)


<220>
<221>	MISC_FEATURE
<222>	(13)
<223>	Xaa is Gln (Q), Tyr (Y), Ala (A), or Cys (C)


<220>
<221>	MISC_FEATURE
<222>	(14)
<223>	Xaa is Leu (L), Met (M), or Tyr (Y)


<220>
<221>	MISC_FEATURE
<222>	(15)
<223>	Xaa is Cys (C), Asp (D), Glu (E), or Leu (L)


<220>
<221>	MISC_FEATURE
<222>	(16)
<223>	Xaa is Gly (G), Glu (E), or Ser (S)


<220>
<221>	MISC_FEATURE
<222>	(17)
<223>	Xaa is Gln (Q), Arg (R), Ile (I), Glu (E), Cys (C), or Lys (K)


<220>
<221>	MISC_FEATURE
<222>	(18)
<223>	Xaa is Ala (A), Gln (Q), Arg (R), or His (H)


<220>
<221>	MISC_FEATURE
<222>	(19)
<223>	Xaa is Ala (A), Gln (Q), Cys (C), or Val (V)

<220>
<221>    MISC_FEATURE
<222>    (20)
<223>    Xaa is Lys (K), Gln (Q), or Arg (R)


<220>
<221>    MISC_FEATURE
<222>    (21)
<223>    Xaa is Glu (E), Gln (Q), Leu (L), Cys (C), or Asp (D)


<220>
<221>    MISC_FEATURE
<222>    (23)
<223>    Xaa is Ile (I) or Val (V)


<220>
<221>    MISC_FEATURE
<222>    (24)
<223>    Xaa is Ala (A), Gln (Q), Cys (C), Asn (N), Asp (D), or Glu (E)


<220>
<221>    MISC_FEATURE
<222>    (27)
<223>    Xaa is Val (V), Leu (L), Lys (K), or Met (M)


<220>
<221>    MISC_FEATURE
<222>    (28)
<223>    Xaa is Cys (C), Lys (K), Ala (A), Asn (N), or Asp (D)


<220>
<221>    MISC_FEATURE
<222>    (29)
<223>    Xaa is Cys (C), Gly (G), Gln (Q), Thr (T), Glu (E), or His (H)


<220>
<221>    MISC_FEATURE
<222>    (30)
<223>    Xaa is Cys, Gly, Lys, or His, or is absent; and Xaa can be
         further linked to R1, wherein R1 is Cys, GKKNDWKHNIT,
         m-SSGAPPPS-n, or m-SSGQPPPS-n, or is absent, and wherein m is
         -Cys-, -Pro-, or -Gly-Pro-, and n is -Cys-, -Gly-, -Ser-, or
         -His-Gly-, or is absent


<400>    103
Xaa Xaa Xaa Gly Thr Phe Xaa Ser Asp Xaa Ser Xaa Xaa Xaa Xaa Xaa
1               5                   10                  15

Xaa Xaa Xaa Xaa Xaa Phe Xaa Xaa Trp Leu Xaa Xaa Xaa Xaa
            20                  25                  30


<210>    104

<211>	40
<212>	PRT
<213>	Artificial Sequence

<220>
<223>	General Formula 2


<220>
<221>	MISC_FEATURE
<222>	(1)
<223>	Xaa is His (H), 4-imidazoacetyl (CA), or Tyr (Y)


<220>
<221>	MISC_FEATURE
<222>	(2)
<223>	Xaa is Gly (G), alpha-methyl-glutamic acid, or Aib
	(aminoisobutyric acid)


<220>
<221>	MISC_FEATURE
<222>	(10)
<223>	Xaa is Tyr (Y) or Cys (C)


<220>
<221>	MISC_FEATURE
<222>	(13)
<223>	Xaa is Gln (Q), Tyr (Y), Ala (A), or Cys (C)


<220>
<221>	MISC_FEATURE
<222>	(14)
<223>	Xaa is Leu (L), Met (M), or Tyr (Y)


<220>
<221>	MISC_FEATURE
<222>	(15)
<223>	Xaa is Asp (D), Glu (E), or Leu (L)


<220>
<221>	MISC_FEATURE
<222>	(16)
<223>	Xaa is Gly (G), Glu (E), or Ser (S)


<220>
<221>	MISC_FEATURE
<222>	(17)
<223>	Xaa is Gln (Q), Arg (R), Ile (I), Glu (E), Cys (C), or Lys (K)


<220>
<221>	MISC_FEATURE
<222>	(18)
<223>	Xaa is Ala (A), Gln (Q), Arg (R), or His (H)

<220>
<221>    MISC_FEATURE
<222>    (19)
<223>    Xaa is Ala (A), Gln (Q), Cys (C), or Val (V)

<220>
<221>    MISC_FEATURE
<222>    (20)
<223>    Xaa is Lys (K), Gln (Q), or Arg (R)

<220>
<221>    MISC_FEATURE
<222>    (21)
<223>    Xaa is Glu (E), Gln (Q), Leu (L), Cys (C), or Asp (D)

<220>
<221>    MISC_FEATURE
<222>    (23)
<223>    Xaa is Ile (I) or Val (V)

<220>
<221>    MISC_FEATURE
<222>    (24)
<223>    Xaa is Ala (A), Gln (Q), Cys (C), Asn (N), or Glu (E)

<220>
<221>    MISC_FEATURE
<222>    (28)
<223>    Xaa is Cys (C), Lys (K), Asn (N), or Asp (D)

<220>
<221>    MISC_FEATURE
<222>    (29)
<223>    Xaa is Cys (C), Gly (G), Gln (Q), or His (H)

<220>
<221>    MISC_FEATURE
<222>    (30)
<223>    Xaa is Cys (C), Gly (G), Lys (K), or His (H)

<220>
<221>    MISC_FEATURE
<222>    (31)
<223>    Xaa is Pro (P) or Cys (C)

<220>
<221>    MISC_FEATURE
<222>    (40)
<223>    Xaa is Cys (C), or is absent

<400>    104
Xaa Xaa Gln Gly Thr Phe Thr Ser Asp Xaa Ser Lys Xaa Xaa Xaa Xaa
1               5                   10                  15

Xaa Xaa Xaa Xaa Xaa Phe Xaa Xaa Trp Leu Leu Xaa Xaa Xaa Xaa Ser
            20                  25                  30

Ser Gly Gln Pro Pro Pro Ser Xaa
        35                  40

<210>    105
<211>    40
<212>    PRT
<213>    Artificial Sequence

<220>
<223>    General Formula 3

<220>
<221>    MISC_FEATURE
<222>    (1)
<223>    Xaa is His (H) or Tyr (Y)

<220>
<221>    MISC_FEATURE
<222>    (2)
<223>    Xaa is alpha-methyl-glutamic acid, or Aib (aminoisobutyric acid)

<220>
<221>    MISC_FEATURE
<222>    (13)
<223>    Xaa is Tyr (Y), Ala (A), or Cys (C)

<220>
<221>    MISC_FEATURE
<222>    (17)
<223>    Xaa is Arg (R), Cys (C), or Lys (K)

<220>
<221>    MISC_FEATURE
<222>    (18)
<223>    Xaa is Ala (A) or Arg (R)

<220>
<221>    MISC_FEATURE
<222>    (19)
<223>    Xaa is Ala (A) or Cys (C)

<220>
<221>    MISC_FEATURE
<222>    (21)
<223>    Xaa is Glu (E) or Asp (D)

<220>
<221>    MISC_FEATURE
<222>    (24)
<223>    Xaa is Gln (Q) or Asn (N)

<220>
<221>    MISC_FEATURE
<222>    (28)
<223>    Xaa is Cys (C) or Asp (D)


<220>
<221>    MISC_FEATURE
<222>    (29)
<223>    Xaa is Cys (C), Gln (Q), or His (H)


<220>
<221>    MISC_FEATURE
<222>    (30)
<223>    Xaa is Cys (C) or His (H)


<220>
<221>    MISC_FEATURE
<222>    (31)
<223>    Xaa is Pro (P) or Cys (C)


<220>
<221>    MISC_FEATURE
<222>    (40)
<223>    Xaa is Cys (C), or is absent


<400>    105
Xaa Xaa Gln Gly Thr Phe Thr Ser Asp Tyr Ser Lys Xaa Leu Asp Glu
1               5                   10                  15

Xaa Xaa Xaa Lys Xaa Phe Val Xaa Trp Leu Leu Xaa Xaa Xaa Xaa Ser
            20                  25                  30

Ser Gly Gln Pro Pro Pro Ser Xaa
        35                  40


<210>    106
<211>    11
<212>    PRT
<213>    Artificial Sequence

<220>
<223>    R1


<400>    106
Gly Lys Lys Asn Asp Trp Lys His Asn Ile Thr
1               5                   10


<210>    107
<211>    8
<212>    PRT
<213>    Artificial Sequence

<220>

<223>    R1

<400>    107
Ser Ser Gly Ala Pro Pro Pro Ser
1               5

<210>    108
<211>    8
<212>    PRT
<213>    Artificial Sequence

<220>
<223>    R1

<400>    108
Ser Ser Gly Gln Pro Pro Pro Ser
1               5

<210>    109
<211>    9
<212>    PRT
<213>    Artificial Sequence

<220>
<223>    R1

<400>    109
Cys Ser Ser Gly Gln Pro Pro Pro Ser
1               5

<210>    110
<211>    10
<212>    PRT
<213>    Artificial Sequence

<220>
<223>    R1

<400>    110
Gly Pro Ser Ser Gly Ala Pro Pro Pro Ser
1               5                   10

<210>    111
<211>    11
<212>    PRT
<213>    Artificial Sequence

<220>
<223>    R1

<400>    111
Gly Pro Ser Ser Gly Ala Pro Pro Pro Ser Cys
1               5                   10

<210>    112
<211>    9
<212>    PRT
<213>    Artificial Sequence

<220>
<223>    R1

<400>    112
Pro Ser Ser Gly Ala Pro Pro Pro Ser
1               5

<210>    113
<211>    10
<212>    PRT
<213>    Artificial Sequence

<220>
<223>    R1

<400>    113
Pro Ser Ser Gly Ala Pro Pro Pro Ser Gly
1               5                   10

<210>    114
<211>    11
<212>    PRT
<213>    Artificial Sequence

<220>
<223>    R1

<400>    114
Pro Ser Ser Gly Ala Pro Pro Pro Ser His Gly
1               5                   10

<210>    115
<211>    10
<212>    PRT
<213>    Artificial Sequence

<220>
<223>    R1

<400>    115
Pro Ser Ser Gly Ala Pro Pro Pro Ser Ser
1               5                   10

<210>    116
<211>    9
<212>    PRT
<213>    Artificial Sequence

<220>

<223>    R1

<400>    116
Pro Ser Ser Gly Gln Pro Pro Pro Ser
1               5

<210>    117
<211>    10
<212>    PRT
<213>    Artificial Sequence

<220>
<223>    R1

<400>    117
Pro Ser Ser Gly Gln Pro Pro Pro Ser Cys
1               5                   10

<210>    118
<211>    29
<212>    PRT
<213>    Homo sapiens

<400>    118
His Ser Gln Gly Thr Phe Thr Ser Asp Tyr Ser Lys Tyr Leu Asp Ser
1               5                   10                  15

Arg Arg Ala Gln Asp Phe Val Gln Trp Leu Met Asn Thr
            20                  25

<210>    119
<211>    21
<212>    PRT
<213>    Artificial Sequence

<220>
<223>    Insulin analog, A-chain

<220>
<221>    MISC_FEATURE
<222>    (1)
<223>    Xaa is alanine, glycine, glutamine, histidine, glutamic acid, or
         asparagine

<220>
<221>    MISC_FEATURE
<222>    (2)
<223>    Xaa is alanine, or isoleucine

<220>
<221>    MISC_FEATURE
<222>    (5)
<223>    Xaa is alanine, glutamic acid, glutamine, histidine, or
         asparagine

<220>
<221>    MISC_FEATURE
<222>    (12)
<223>    Xaa is alanine, serine, glutamine, glutamic acid, histidine, or
         asparagine


<220>
<221>    MISC_FEATURE
<222>    (14)
<223>    Xaa is alanine, tyrosine, glutamic acid, histidine, lysine,
         aspartic acid, or asparagine


<220>
<221>    MISC_FEATURE
<222>    (16)
<223>    Xaa is alanine, leucine, tyrosine, histidine, glutamic acid, or
         asparagine


<220>
<221>    MISC_FEATURE
<222>    (19)
<223>    Xaa is alanine, tyrosine, serine, glutamic acid, histidine,
         threonine, or asparagine


<220>
<221>    MISC_FEATURE
<222>    (21)
<223>    Xaa is asparagine, glycine, histidine, or alanine


<400>    119
Xaa Xaa Val Glu Xaa Cys Cys Thr Ser Ile Cys Xaa Leu Xaa Gln Xaa
1               5                   10                  15

Glu Asn Xaa Cys Xaa
            20


<210>    120
<211>    30
<212>    PRT
<213>    Artificial Sequence

<220>
<223>    Insulin analog, B-chain


<220>
<221>    MISC_FEATURE
<222>    (8)
<223>    Xaa is alanine, or glycine


<220>
<221>    MISC_FEATURE
<222>    (16)
<223>    Xaa is tyrosine, glutamic acid, serine, threonine, or aspartic
         acid or is absent

<220>
<221>    MISC_FEATURE
<222>    (23)
<223>    Xaa is glycine, or alanine


<220>
<221>    MISC_FEATURE
<222>    (24)
<223>    Xaa is alanine or phenylalanine


<220>
<221>    MISC_FEATURE
<222>    (25)
<223>    Xaa is alanine, phenylalanine, aspartic acid, or glutamic acid or
         is absent


<220>
<221>    MISC_FEATURE
<222>    (27)
<223>    Xaa is threonine, or is absent


<220>
<221>    MISC_FEATURE
<222>    (28)
<223>    Xaa is proline, glutamic acid, or aspartic acid, or is absent


<400>    120
Phe Val Asn Gln His Leu Cys Xaa Ser His Leu Val Glu Ala Leu Xaa
1               5                   10                  15

Leu Val Cys Gly Glu Arg Xaa Xaa Xaa Tyr Xaa Xaa Lys Thr
            20                  25                  30


<210>    121
<211>    21
<212>    PRT
<213>    Homo sapiens

<400>    121
Gly Ile Val Glu Gln Cys Cys Thr Ser Ile Cys Ser Leu Tyr Gln Leu
1               5                   10                  15

Glu Asn Tyr Cys Asn
            20


<210>    122
<211>    30
<212>    PRT
<213>    Homo sapiens

<400>    122
Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr
            20                  25                  30

<210>   123
<211>   258
<212>   DNA
<213>   Artificial Sequence

<220>
<223>   Analog 1

<400>   123
ttcgttaacc aacacttgtg tggctcacac ctggtggaag ctctctacct agtgtgcggg      60

gaacgaggct tcttctacac acccaagacc cgccgggagg cagaggacct gcaggtgggg     120

caggtggagc tgggcggggg ccctggtgca ggcagcctgc agcccttggc cctggagggg     180

tccctgcaga agcgtgcgat tgtggaacaa tgctgtacca gcatctgctc cctctaccag     240

ctggagaact actgcaac                                                   258

<210>   124
<211>   86
<212>   PRT
<213>   Artificial Sequence

<220>
<223>   Analog 1

<400>   124
Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Ala Ile Val Glu Gln Cys Cys Thr Ser Ile Cys Ser Leu Tyr Gln
65                  70                  75                  80

Leu Glu Asn Tyr Cys Asn
                85

<210>   125
<211>   258
<212>   DNA
<213>   Artificial Sequence

<220>
<223>   Analog 2

<400>    125
ttcgttaacc aacacttgtg tggctcacac ctggtggaag ctctctacct agtgtgcggg    60

gaacgaggct tcttctacac acccaagacc cgccgggagg cagaggacct gcaggtgggg    120

caggtggagc tgggcggggg ccctggtgca ggcagcctgc agcccttggc cctggagggg    180

tccctgcaga agcgtggcgc ggtggaacaa tgctgtacca gcatctgctc cctctaccag    240

ctggagaact actgcaac                                                  258


<210>    126
<211>    86
<212>    PRT
<213>    Artificial Sequence

<220>
<223>    Analog 2


<400>    126
Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ala Val Glu Gln Cys Cys Thr Ser Ile Cys Ser Leu Tyr Gln
65                  70                  75                  80

Leu Glu Asn Tyr Cys Asn
                85


<210>    127
<211>    258
<212>    DNA
<213>    Artificial Sequence

<220>
<223>    Analog 3


<400>    127
ttcgttaacc aacacttgtg tggctcacac ctggtggaag ctctctacct agtgtgcggg    60

gaacgaggct tcttctacac acccaagacc cgccgggagg cagaggacct gcaggtgggg    120

caggtggagc tgggcggggg ccctggtgca ggcagcctgc agcccttggc cctggagggg    180

tccctgcaga agcgtggcat tgtggaacaa tgctgtacca gcatctgctc cctctaccag    240

ctggagaacg cgtgcaac                                                  258


<210>    128

<211>    86
<212>    PRT
<213>    Artificial Sequence

<220>
<223>    Analog 3


<400>    128
Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Tyr Gln
65                  70                  75                  80

Leu Glu Asn Ala Cys Asn
                85


<210>    129
<211>    258
<212>    DNA
<213>    Artificial Sequence

<220>
<223>    Analog 4


<400>    129
ttcgttaacc aacacttgtg tgcgtcacac ctggtggaag ctctctacct agtgtgcggg      60

gaacgaggct tcttctacac acccaagacc cgccgggagg cagaggacct gcaggtgggg     120

caggtggagc tgggcggggg ccctggtgca ggcagcctgc agcccttggc cctggagggg     180

tccctgcaga agcgtggcat tgtggaacaa tgctgtacca gcatctgctc cctctaccag     240

ctggagaact actgcaac                                                   258


<210>    130
<211>    86
<212>    PRT
<213>    Artificial Sequence

<220>
<223>    Analog 4


<400>    130
Phe Val Asn Gln His Leu Cys Ala Ser His Leu Val Glu Ala Leu Tyr
1               5                   10                  15

Leu Val Cys Gly Glu Arg Gly Phe Phe Tyr Thr Pro Lys Thr Arg Arg

20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Tyr Gln
65                  70                  75                  80

Leu Glu Asn Tyr Cys Asn
                85


<210>    131
<211>    258
<212>    DNA
<213>    Artificial Sequence

<220>
<223>    Analog 5


<400>    131
ttcgttaacc aacacttgtg tggctcacac ctggtggaag ctctctacct agtgtgcggg      60

gaacgagcgt tcttctacac acccaagacc cgccgggagg cagaggacct gcaggtgggg     120

caggtggagc tgggcggggg ccctggtgca ggcagcctgc agcccttggc cctggagggg     180

tccctgcaga agcgtggcat tgtggaacaa tgctgtacca gcatctgctc cctctaccag     240

ctggagaact actgcaac                                                   258


<210>    132
<211>    86
<212>    PRT
<213>    Artificial Sequence

<220>
<223>    Analog 5


<400>    132
Phe Val Asn Gln His Leu Cys Gly Ser His Leu Val Glu Ala Leu Tyr
1               5                   10                  15

Leu Val Cys Gly Glu Arg Ala Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Tyr Gln
65                  70                  75                  80

Leu Glu Asn Tyr Cys Asn
                85

<210>    133
<211>    258
<212>    DNA
<213>    Artificial Sequence

<220>
<223>    Analog 6


<400>    133
ttcgttaacc aacacttgtg tggctcacac ctggtggaag ctctctacct agtgtgcggg         60

gaacgaggcg cgttctacac acccaagacc cgccgggagg cagaggacct gcaggtgggg        120

caggtggagc tgggcggggg ccctggtgca ggcagcctgc agcccttggc cctggagggg        180

tccctgcaga agcgtggcat tgtggaacaa tgctgtacca gcatctgctc cctctaccag        240

ctggagaact actgcaac                                                      258


<210>    134
<211>    86
<212>    PRT
<213>    Artificial Sequence

<220>
<223>    Analog 6


<400>    134
Phe Val Asn Gln His Leu Cys Gly Ser His Leu Val Glu Ala Leu Tyr
1               5                   10                  15

Leu Val Cys Gly Glu Arg Gly Ala Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Tyr Gln
65                  70                  75                  80

Leu Glu Asn Tyr Cys Asn
                85


<210>    135
<211>    258
<212>    DNA
<213>    Artificial Sequence

<220>
<223>    Analog 7


<400>    135
ttcgttaacc aacacttgtg tggctcacac ctggtggaag ctctctacct agtgtgcggg         60

gaacgaggct tcgcgtacac acccaagacc cgccgggagg cagaggacct gcaggtgggg 120

caggtggagc tgggcggggg ccctggtgca ggcagcctgc agcccttggc cctggagggg 180

tccctgcaga agcgtggcat tgtggaacaa tgctgtacca gcatctgctc cctctaccag 240

ctggagaact actgcaac 258

<210> 136
<211> 86
<212> PRT
<213> Artificial Sequence

<220>
<223> Analog 7

<400> 136
Phe Val Asn Gln His Leu Cys Gly Ser His Leu Val Glu Ala Leu Tyr
1               5                   10                  15

Leu Val Cys Gly Glu Arg Gly Phe Ala Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Tyr Gln
65                  70                  75                  80

Leu Glu Asn Tyr Cys Asn
                85

<210> 137
<211> 261
<212> DNA
<213> Artificial Sequence

<220>
<223> Analog 8

<400> 137
ttcgttaacc aacacttgtg tggctcacac ctggtggaag ctctctacct agtgtgcggg 60

gaacgaggct tcttctacac acccaagacc cgccgggagg cagaggacct gcaggtgggg 120

caggtggagc tgggcggggg ccctggtgca ggcagcctgc agcccttggc cctggagggg 180

tccctgcaga agcgtggcat tgtggaacaa tgctgtacca gcatctgctc cctcgaacag 240

ctggagaact actgcaactg a 261

<210> 138
<211> 86
<212> PRT

<213>    Artificial Sequence

<220>
<223>    Analog 8


<400>    138
Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Glu Gln
65                  70                  75                  80

Leu Glu Asn Tyr Cys Asn
                85


<210>    139
<211>    261
<212>    DNA
<213>    Artificial Sequence

<220>
<223>    Analog 9


<400>    139
ttcgttaacc aacacttgtg tggctcacac ctggtggaag ctctctacct agtgtgcggg      60

gaacgaggct tcttctacac acccaagacc cgccgggagg cagaggacct gcaggtgggg     120

caggtggagc tgggcggggg ccctggtgca ggcagcctgc agcccttggc cctggagggg     180

tccctgcaga agcgtggcat tgtggaacaa tgctgtacca gcatctgctc cctcaaccag     240

ctggagaact actgcaactg a                                               261


<210>    140
<211>    86
<212>    PRT
<213>    Artificial Sequence

<220>
<223>    Analog 9


<400>    140
Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Asn Gln
65                  70                  75                  80

Leu Glu Asn Tyr Cys Asn
                85

<210>    141
<211>    258
<212>    DNA
<213>    Artificial Sequence

<220>
<223>    Analog 10

<400>    141
ttcgttaacc aacacttgtg tggctcacac ctggtggaag ctctctacct agtgtgcggg      60

gaacgaggct tctacacacc caagacccgc cgggaggcag aggacctgca ggtggggcag     120

gtggagctgg gcgggggccc tggtgcaggc agcctgcagc ccttggccct ggaggggtcc     180

ctgcagaagc gtggcattgt ggaacaatgc tgtaccagca tctgctccct cgaacagctg     240

gagaactact gcaactga                                                   258

<210>    142
<211>    85
<212>    PRT
<213>    Artificial Sequence

<220>
<223>    Analog 10

<400>    142
Phe Val Asn Gln His Leu Cys Gly Ser His Leu Val Glu Ala Leu Tyr
1               5                   10                  15

Leu Val Cys Gly Glu Arg Gly Phe Tyr Thr Pro Lys Thr Arg Arg Glu
            20                  25                  30

Ala Glu Asp Leu Gln Val Gly Gln Val Glu Leu Gly Gly Gly Pro Gly
        35                  40                  45

Ala Gly Ser Leu Gln Pro Leu Ala Leu Glu Gly Ser Leu Gln Lys Arg
    50                  55                  60

Gly Ile Val Glu Gln Cys Cys Thr Ser Ile Cys Ser Leu Glu Gln Leu
65                  70                  75                  80

Glu Asn Tyr Cys Asn
                85

<210>    143
<211>    258
<212>    DNA
<213>    Artificial Sequence

<220>
<223>    Analog 11


<400>    143
ttcgttaacc aacacttgtg tggctcacac ctggtggaag ctctcgagct agtgtgcggg    60

gaacgaggct tctacacacc caagacccgc cgggaggcag aggacctgca ggtggggcag    120

gtggagctgg gcgggggccc tggtgcaggc agcctgcagc ccttggccct ggaggggtcc    180

ctgcagaagc gtggcattgt ggaacaatgc tgtaccagca tctgctccct cgcccagctg    240

gagaactact gcaactga    258


<210>    144
<211>    85
<212>    PRT
<213>    Artificial Sequence

<220>
<223>    Analog 11


<400>    144
Phe Val Asn Gln His Leu Cys Gly Ser His Leu Val Glu Ala Leu Glu
1               5                   10                  15

Leu Val Cys Gly Glu Arg Gly Phe Tyr Thr Pro Lys Thr Arg Arg Glu
            20                  25                  30

Ala Glu Asp Leu Gln Val Gly Gln Val Glu Leu Gly Gly Gly Pro Gly
        35                  40                  45

Ala Gly Ser Leu Gln Pro Leu Ala Leu Glu Gly Ser Leu Gln Lys Arg
    50                  55                  60

Gly Ile Val Glu Gln Cys Cys Thr Ser Ile Cys Ser Leu Ala Gln Leu
65                  70                  75                  80

Glu Asn Tyr Cys Asn
                85


<210>    145
<211>    258
<212>    DNA
<213>    Artificial Sequence

<220>
<223>    Analog 12


<400>    145
ttcgttaacc aacacttgtg tggctcacac ctggtggaag ctctctacct agtgtgcggg    60

gaacgaggct tcttctacac acccaagacc cgccgggagg cagaggacct gcaggtgggg    120

caggtggagc tgggcggggg ccctggtgca ggcagcctgc agcccttggc cctggagggg 180

tccctgcaga agcgtggcat tgtggaacaa tgctgtacca gcatctgctc cctccatcag 240

ctggagaact actgcaac 258

<210> 146
<211> 86
<212> PRT
<213> Artificial Sequence

<220>
<223> Analog 12

<400> 146
Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu His Gln
65                  70                  75                  80

Leu Glu Asn Tyr Cys Asn
                85

<210> 147
<211> 258
<212> DNA
<213> Artificial Sequence

<220>
<223> Analog 13

<400> 147
ttcgttaacc aacacttgtg tggctcacac ctggtggaag ctctctacct agtgtgcggg 60

gaacgaggct tcttctacac acccaagacc cgccgggagg cagaggacct gcaggtgggg 120

caggtggagc tgggcggggg ccctggtgca ggcagcctgc agcccttggc cctggagggg 180

tccctgcaga agcgtggcat tgtggaacaa tgctgtacca gcatctgctc cctcaagcag 240

ctggagaact actgcaac 258

<210> 148
<211> 86
<212> PRT
<213> Artificial Sequence

<220>
<223>    Analog 13


<400>    148
Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Lys Gln
65                  70                  75                  80

Leu Glu Asn Tyr Cys Asn
                85


<210>    149
<211>    258
<212>    DNA
<213>    Artificial Sequence

<220>
<223>    Analog 14


<400>    149
ttcgttaacc aacacttgtg tggctcacac ctggtggaag ctctctacct agtgtgcggg      60

gaacgaggct tcttctacac acccaagacc cgccgggagg cagaggacct gcaggtgggg     120

caggtggagc tgggcggggg ccctggtgca ggcagcctgc agcccttggc cctggagggg     180

tccctgcaga agcgtggcat tgtggaacaa tgctgtacca gcatctgctc cctctaccag     240

ctggagaacg agtgcaac                                                   258


<210>    150
<211>    86
<212>    PRT
<213>    Artificial Sequence

<220>
<223>    Analog 14


<400>    150
Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Tyr Gln
65                  70                  75                  80

Leu Glu Asn Glu Cys Asn
                85


<210>    151
<211>    258
<212>    DNA
<213>    Artificial Sequence

<220>
<223>    Analog 15


<400>    151
ttcgttaacc aacacttgtg tggctcacac ctggtggaag ctctctacct agtgtgcggg        60

gaacgaggct tcttctacac acccaagacc cgccgggagg cagaggacct gcaggtgggg       120

caggtggagc tgggcggggg ccctggtgca ggcagcctgc agcccttggc cctggagggg       180

tccctgcaga agcgtggcat tgtggaacaa tgctgtacca gcatctgctc cctctaccag       240

ctggagaact cctgcaac                                                     258


<210>    152
<211>    86
<212>    PRT
<213>    Artificial Sequence

<220>
<223>    Analog 15


<400>    152
Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Tyr Gln
65                  70                  75                  80

Leu Glu Asn Ser Cys Asn
                85


<210>    153
<211>    258

<212>     DNA
<213>     Artificial Sequence

<220>
<223>     Analog 16


<400>     153
ttcgttaacc aacacttgtg tggctcacac ctggtggaag ctctctacct agtgtgcggg          60

gaacgaggct tcttctacac acccaagacc cgccgggagg cagaggacct gcaggtgggg         120

caggtggagc tgggcggggg ccctggtgca ggcagcctgc agcccttggc cctggagggg         180

tccctgcaga agcgtggcat tgtggaacaa tgctgtacca gcatctgctc cctctaccag         240

ctggagaaca cctgcaac                                                       258


<210>     154
<211>     86
<212>     PRT
<213>     Artificial Sequence

<220>
<223>     Analog 16


<400>     154
Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Tyr Gln
65                  70                  75                  80

Leu Glu Asn Thr Cys Asn
                85


<210>     155
<211>     258
<212>     DNA
<213>     Artificial Sequence

<220>
<223>     Analog 17


<400>     155
ttcgttaacc aacacttgtg tggctcacac ctggtggaag ctctcgagct agtgtgcggg          60

gaacgaggct tcttctacac acccaagacc cgccgggagg cagaggacct gcaggtgggg         120

caggtggagc tgggcggggg ccctggtgca ggcagcctgc agcccttggc cctggagggg         180

tccctgcaga agcgtggcat tgtggaacaa tgctgtacca gcatctgctc cctctaccag 240

ctggagaact actgcaac 258

<210> 156
<211> 86
<212> PRT
<213> Artificial Sequence

<220>
<223> Analog 17

<400> 156
Phe Val Asn Gln His Leu Cys Gly Ser His Leu Val Glu Ala Leu Glu
1               5                   10                  15

Leu Val Cys Gly Glu Arg Gly Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Tyr Gln
65                  70                  75                  80

Leu Glu Asn Tyr Cys Asn
                85

<210> 157
<211> 258
<212> DNA
<213> Artificial Sequence

<220>
<223> Analog 18

<400> 157
ttcgttaacc aacacttgtg tggctcacac ctggtggaag ctctctccct agtgtgcggg 60

gaacgaggct tcttctacac acccaagacc cgccgggagg cagaggacct gcaggtgggg 120

caggtggagc tgggcggggg ccctggtgca ggcagcctgc agcccttggc cctggagggg 180

tccctgcaga agcgtggcat tgtggaacaa tgctgtacca gcatctgctc cctctaccag 240

ctggagaact actgcaac 258

<210> 158
<211> 86
<212> PRT
<213> Artificial Sequence

<220>
<223> Analog 18

<400>    158
Phe Val Asn Gln His Leu Cys Gly Ser His Leu Val Glu Ala Leu Ser
1               5                   10                  15

Leu Val Cys Gly Glu Arg Gly Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Tyr Gln
65                  70                  75                  80

Leu Glu Asn Tyr Cys Asn
                85


<210>    159
<211>    258
<212>    DNA
<213>    Artificial Sequence

<220>
<223>    Analog 19


<400>    159
ttcgttaacc aacacttgtg tggctcacac ctggtggaag ctctcaccct agtgtgcggg        60

gaacgaggct tcttctacac acccaagacc cgccgggagg cagaggacct gcaggtgggg       120

caggtggagc tgggcggggg ccctggtgca ggcagcctgc agcccttggc cctggagggg       180

tccctgcaga agcgtggcat tgtggaacaa tgctgtacca gcatctgctc cctctaccag       240

ctggagaact actgcaac                                                     258


<210>    160
<211>    86
<212>    PRT
<213>    Artificial Sequence

<220>
<223>    Analog 19


<400>    160
Phe Val Asn Gln His Leu Cys Gly Ser His Leu Val Glu Ala Leu Thr
1               5                   10                  15

Leu Val Cys Gly Glu Arg Gly Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys

50                  55                  60

Arg Gly Ile Val Glu Gln Cys Cys Thr Ser Ile Cys Ser Leu Tyr Gln
65                  70                  75                  80

Leu Glu Asn Tyr Cys Asn
                85

<210>    161
<211>    258
<212>    DNA
<213>    Artificial Sequence

<220>
<223>    Analog 20

<400>    161
ttcgttaacc aacacttgtg tggctcacac ctggtggaag ctctctacct agtgtgcggg      60

gaacgaggct tcttctacac acccaagacc cgccgggagg cagaggacct gcaggtgggg     120

caggtggagc tgggcggggg ccctggtgca ggcagcctgc agcccttggc cctggagggg     180

tccctgcaga agcgtggcat tgtggaacaa tgctgtacca gcatctgctc cctcgcccag     240

ctggagaact actgcaac                                                   258

<210>    162
<211>    86
<212>    PRT
<213>    Artificial Sequence

<220>
<223>    Analog 20

<400>    162
Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Ala Gln
65                  70                  75                  80

Leu Glu Asn Tyr Cys Asn
                85

<210>    163
<211>    258
<212>    DNA
<213>    Artificial Sequence

<220>
<223>    Analog 21


<400>    163
ttcgttaacc aacacttgtg tggctcacac ctggtggaag ctctctacct agtgtgcggg      60

gaacgaggct tcttctacac acccaagacc cgccgggagg cagaggacct gcaggtgggg     120

caggtggagc tgggcggggg ccctggtgca ggcagcctgc agcccttggc cctggagggg     180

tccctgcaga agcgtggcat tgtggaacaa tgctgtacca gcatctgctc cctcgaccag     240

ctggagaact actgcaac                                                   258


<210>    164
<211>    86
<212>    PRT
<213>    Artificial Sequence

<220>
<223>    Analog 21


<400>    164
Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Asp Gln
65                  70                  75                  80

Leu Glu Asn Tyr Cys Asn
                85


<210>    165
<211>    258
<212>    DNA
<213>    Artificial Sequence

<220>
<223>    Analog 22


<400>    165
ttcgttaacc aacacttgtg tggctcacac ctggtggaag ctctcgacct agtgtgcggg      60

gaacgaggct tcttctacac acccaagacc cgccgggagg cagaggacct gcaggtgggg     120

caggtggagc tgggcggggg ccctggtgca ggcagcctgc agcccttggc cctggagggg     180

tccctgcaga agcgtggcat tgtggaacaa tgctgtacca gcatctgctc cctctaccag     240

ctggagaact actgcaac                                                    258

<210>    166
<211>    86
<212>    PRT
<213>    Artificial Sequence

<220>
<223>    Analog 22

<400>    166
Phe Val Asn Gln His Leu Cys Gly Ser His Leu Val Glu Ala Leu Asp
1               5                   10                  15

Leu Val Cys Gly Glu Arg Gly Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Tyr Gln
65                  70                  75                  80

Leu Glu Asn Tyr Cys Asn
                85

<210>    167
<211>    258
<212>    DNA
<213>    Artificial Sequence

<220>
<223>    Analog 23

<400>    167
ttcgttaacc aacacttgtg tggctcacac ctggtggaag ctctctacct agtgtgcggg      60

gaacgaggct tcgactacac acccaagacc cgccgggagg cagaggacct gcaggtgggg     120

caggtggagc tgggcggggg ccctggtgca ggcagcctgc agcccttggc cctggagggg     180

tccctgcaga agcgtggcat tgtggaacaa tgctgtacca gcatctgctc cctctaccag     240

ctggagaact actgcaac                                                    258

<210>    168
<211>    86
<212>    PRT
<213>    Artificial Sequence

<220>
<223>    Analog 23

<400>    168
Phe Val Asn Gln His Leu Cys Gly Ser His Leu Val Glu Ala Leu Tyr
1               5                   10                  15

Leu Val Cys Gly Glu Arg Gly Phe Asp Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Tyr Gln
65                  70                  75                  80

Leu Glu Asn Tyr Cys Asn
                85


<210>    169
<211>    258
<212>    DNA
<213>    Artificial Sequence

<220>
<223>    Analog 24


<400>    169
ttcgttaacc aacacttgtg tggctcacac ctggtggaag ctctctacct agtgtgcggg      60

gaacgaggct tcgagtacac acccaagacc cgccgggagg cagaggacct gcaggtgggg     120

caggtggagc tgggcggggg ccctggtgca ggcagcctgc agcccttggc cctggagggg     180

tccctgcaga agcgtggcat tgtggaacaa tgctgtacca gcatctgctc cctctaccag     240

ctggagaact actgcaac                                                   258


<210>    170
<211>    86
<212>    PRT
<213>    Artificial Sequence

<220>
<223>    Analog 24


<400>    170
Phe Val Asn Gln His Leu Cys Gly Ser His Leu Val Glu Ala Leu Tyr
1               5                   10                  15

Leu Val Cys Gly Glu Arg Gly Phe Glu Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Tyr Gln
65                  70                  75                  80

Leu Glu Asn Tyr Cys Asn
                85